(19) Europäisches Patentamt
European Patent Office
Office européen des brevets

(11) Veröffentlichungsnummer: **0 316 695 B1**

(12) **EUROPÄISCHE PATENTSCHRIFT**

(45) Veröffentlichungstag der Patentschrift: **31.03.93**

(51) Int. Cl.[5]: **A61K 39/21**, C07K 15/00, C12N 15/00, C12P 21/02, G01N 33/569, A61K 39/395

(21) Anmeldenummer: **88118488.1**

(22) Anmeldetag: **05.11.88**

# (54) Rekombinante HIV-2 Polypeptide.

(30) Priorität: **16.11.87 CH 4454/87**

(43) Veröffentlichungstag der Anmeldung:
**24.05.89 Patentblatt 89/21**

(45) Bekanntmachung des Hinweises auf die Patenterteilung:
**31.03.93 Patentblatt 93/13**

(84) Benannte Vertragsstaaten:
**AT BE CH DE ES FR GB IT LI NL SE**

(56) Entgegenhaltungen:
**EP-A- 283 327**
**EP-A- 284 383**
**EP-A- 292 454**
**WO-A-87/04459**
**WO-A-88/05440**

**NATURE, Band 326, 16 April 1987; M.GUYADER et al., Seiten 662-669***

(73) Patentinhaber: **F. HOFFMANN-LA ROCHE AG**
**Postfach 3255**
**CH-4002 Basel(CH)**

(72) Erfinder: **Bannwarth, Wilhelm, Dr.**
**Liebenbergstr. 8**
**W-7888 Rheinfelden-Beuggen(DE)**
Erfinder: **Caspers, Patrick, Dr.**
**Rohrhagstr. 6**
**CH-4104 Oberwil(CH)**
Erfinder: **Le Grice, Stuart, Dr.**
**Spalenring 63**
**CH-4055 Basel(CH)**
Erfinder: **Mous, Jan, Dr.**
**Füllinsdörferstr. 65**
**CH-4304 Giebenach(CH)**

(74) Vertreter: **Mezger, Wolfgang, Dr. et al**
**Grenzacherstrasse 124 Postfach 3255**
**CH-4002 Basel (CH)**

**EP 0 316 695 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Polypeptide, welche mindestens eine antigene und/oder immunogene determinante Gruppe des Hüllproteins (env) des HIV-2 Virus einschliessen, DNA-Sequenzen, die diese Polypeptide kodieren, rekombinante Vektoren, die diese DNA-Sequenzen enthalten, mit diesen rekombinanten Vektoren transformierte Mikroorganismen sowie Verfahren zu deren Herstellung mittels rekombinanter DNA Technologie. Die Erfindung betrifft ferner Verfahren zum Nachweis von HIV-Antikörpern (HIV-2 Antikörper oder HIV-1 und HIV-2 Antikörper) oder HIV-Viren (HIV-2 Viren oder HIV-1 und HIV-2 Viren) in menschlichen Seren oder anderen biologischen Körperflüssigkeiten.

1986 wurde ein neues Virus mit der Bezeichnung HIV-2 aus westafrikanischen AIDS-Patienten isoliert [Clavel et al., Science 233, 343-346 (1986); Clavel et al., Nature 324, 691-695 (1986)]. Dieses Virus wurde auf Grund seiner Morphologie, seines Lymphotropismus und seiner cytophatischen in vitro Wirkung auf $T_4$-positive Zellen mit den AIDS verursachenden HIV-1 Viren in Verbindung gebracht. Trotz dieser ähnlichen Eigenschaften zeigt ein genetischer Vergleich von HIV-1 und HIV-2 Viren jedoch nur eine begrenzte Sequenzhomologie [Guyader et al., Nature 326, 662-669 (1987)1.

Mehr als 20 verschiedene HIV-2 Viren wurden aus westafrikanischen AIDS-Patienten aber auch aus europäischen AIDS-Patienten isoliert [Guyader et al., supra; Brun-Vezinet et al., Lancet 1, 128-132 (1987)]. Die Seren dieser AIDS-Patienten waren im HIV-1 ELISA alle negativ [Brun-Vezinet et al., supra]. Deshalb ist das Bedürfnis nach genauen und schnellen Verfahren für die Diagnose von HIV-2 Viren in menschlichem Blut und in anderen Körperflüssigkeiten sehr gross.

EP-A-284 383 (Publikationsdatum 28.9.1988) beschreibt kurze Peptide aus dem Hüllprotein (env) und Strukturprotein (gag) des LAV-2, die zur Detektion von Antikörpern gegen dieses Virus geeignet sind. In der PCT-Anmeldung WO87/04459 (Publikationsdatum 30.7.1987) wird die Isolierung und Charakterisierung von HIV-2, insbesondere des grossen Hüllproteins (env) sowie bestimmter Fragmente davon, beschrieben. EP-A-283 327 (Publikationsdatum 21.9.1988) beschreibt kurze Peptide aus den env Proteinen von HIV-1, HIV-2 und SIV-1 mit homologen Sequenzen.

Durch die Anwendung rekombinanter DNA-Techniken wurden deshalb neue Polypeptide mit einer Aminosäuresequenz, die mit mindestens einer antigenen und/oder immunogenen determinanten Gruppe des Hüllproteins (env) des HIV-2 Virus übereinstimmt, hergestellt. Diese Polypeptide gestatten den Nachweis von HIV-2 Antikörpern oder HIV-2 Viren oder Fragmenten davon in menschlichen Seren oder anderen biologischen Körperflüssigkeiten.

Die vorliegende Erfindung betrifft deshalb Polypeptide mit einer Aminosäuresequenz, die mit mindestens einer antigenen und/oder immunogenen determinanten Gruppe des HIV-2 env-Proteins übereinstimmt.

Genauer gesagt, betrifft die vorliegende Erfindung ein Polypeptid mit der Aminosäuresequenz

SerAlaArgLeuAsnSerTrpGlyCysAlaPheArgGlnValCysHisThrThr
ValProTrpValAsnAspSerLeuAlaProAspTrpAspAsnMetThrTrpGln
GluTrpGluLysGlnValArgTyrLeuGluAlaAsnIleSerLysSerLeuGlu
GlnAlaGlnGly (ENV(60)) (I)

oder Fragmente oder funktionelle Aequivalente davon, kovalent verknüpft mit einem Affinitätspeptid und einem Polypeptid, dessen Aminosäuresequenz mit mindestens einer antigenen und/oder immunogenen determinanten Gruppe des HIV-1 Hüllproteins (env) und/oder des HIV-1 Strukturproteins (gag) übereinstimmt. Da solche Fusionsproteine sowohl antigene und/oder immunogene determinanten Gruppe von HIV-1 als auch HIV-2 Viren aufweisen, stellen diese Fusionspolypeptide ein wirksames diagnostisches Werkzeug zum gleichzeitigen Nachweis von HIV-1 und HIV-2 Antikörpen oder HIV-1 und HIV-2 Viren oder Fragmenten davon in menschlichen Seren oder anderen biologischen Körperflüssigkeiten dar.

Die bevorzugten Polypeptide gemäss der vorliegenden Erfindung sind durch die allgemeinen Formeln

A - B - C
A - C - B
und
C - B - A

definiert, worin

A ein Affinitätspeptid ist,

B ein Polypeptid mit der Aminosäuresequenz der Formel I ist, und

C ein Polypeptid, dessen Aminosäuresequenz mit mindestens einer antigenen und/oder immunogenen determinanten Gruppe HIV-1 Hüllproteins (env) und/oder des HIV-1 Strukturproteins (gag) übereinstimmt, ist.

Ein besonders bevorzugt erfindungsgemäss Polypeptid hat der Formel

MetArgGlySerGluAlaGlnGlnHisLeuLeuGlnLeuThrValTrpGlyIleLysGln
LeuGlnAlaArgIleLeuAlaValGluArgTyrLeuLysAspGlnGlnLeuLeuGlyIle
TrpGlyCysSerGlyLysLeuIleCysThrThrAlaValProTrpAsnAlaSerTrpSer
AsnLysLeuLeuGluGlnIleTrpAsnAsnMetThrTrpMetGluTrpAspArgGluIle
AsnAsnTyrThrGlySerGlyIleArgLeuArgProGlyGlyLysLysLysTyrLysLeu
LysHisIleValTrpAlaSerArgGluLeuGluArgPheAlaValAsnProGlyLeuLeu
GluThrSerGluGlyCysArgGlnIleLeuGlyGlnLeuGlnProSerLeuGlnThrGly
SerLysGluLeuArgSerLeuTyrAsnThrValAlaThrLeuTyrCysValHisGlnArg
IleGluIleLysAspThrLysGluAlaLeuAspLysValGluGluGlnAsnAsnSer
LysLysLysAlaGlnGlnGluAlaAlaAspAlaGlyAsnArgAsnGlnValSerGlnAsn
TyrProIleValGlnAsnLeuGlnGlyGlnMetValHisGlnAlaIleSerProArgThr
LeuAsnAlaTrpValLysValValGluGluLysAlaPheSerProGluValIleProMet
PheSerAlaLeuSerGluGlyAlaThrProGlnAspLeuAsnThrMetLeuAsnThrVal
GlyGlyHisGlnAlaAlaMetGlnMetLeuLysGluThrIleAsnGluGluAlaAlaGlu
TrpAspArgLeuHisProValHisAlaGlyProIleAlaProGlyGlnMetArgGluPro
ArgGlySerAspIleAlaGlyThrThrSerThrLeuGlnGluGlnIleGlyTrpMetThr
AsnAsnProProIleProValGlyGluIleTyrLysArgTrpIleIleLeuGlyLeuAsn
LysIleValArgMetTyrSerProThrSerIleLeuAspIleLysGlnGlyProLysGlu
ProPheArgAspTyrValAspArgPheTyrLysThrLeuArgAlaGluGlnAlaThrGln
GluValLysAsnTrpMetThrGluThrLeuLeuValGlnAsnAlaAsnProAspCysLys
ThrIleLeuLysAlaLeuGlyProAlaAlaThrLeuGluGluMetMetThrAlaCysGln
GlyValGlyGlyProGlyHisLysAlaArgValLeuAlaGluAlaMetSerGlnValThr
GlySerAlaAlaIleMetMetGlnArgGlyAsnPheArgAsnGlnArgLysThrValLys
CysPheAsnCysGlyLysGluGlyHisIleAlaArgAsnCysArgAlaProArgLysLys
GlyCysTrpLysCysGlyLysGluGlyHisGlnMetLysAspCysThrGluArgGlnAla
AsnPheLeuGlyLysIleGlyArgSerAlaArgLeuAsnSerTrpGlyCysAlaPheArg
GlnValCysHisThrThrValProTrpValAsnAspSerLeuAlaProAspTrpAspAsn
MetThrTrpGlnGluTrpGluLysGlnValArgTyrLeuGluAlaAsnIleSerLysSer
LeuGluGlnAlaGlnGlySerHisHisHisHisHisHis

(ENV(80)-GAG(419)-ENV(60)) (IV)

Der Ausdruck "funktionelle Aequivalente" der im Zusammenhang mit den erfindungsgemässen Polypeptiden verwendet wird, bezieht sich auf Polypeptide, deren Aminosäuresequenzen durch Nukleotidsubstitutionen, Nukleotid-Deletionen, Nukleotid-Insertionen oder Nukleotid-Additionen aus den oben gezeigten Aminosäuresequenzen hervorgegangen sind und mit mindestens einer antigenen und/oder immunogenen determinanten Gruppe des HIV-2 env-Proteins übereinstimmen.

Gewisse Substitutionen in der Aminosäuresequenz eines Polypeptids haben keinen Einfluss auf die biologische Aktivität eines Polypeptids. Beispiele solcher Aminosäuresubstitutionen sind Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu und vice versa (vgl. Doolittle, in "The Proteins", ed. Neurath, H, und Hill, R.L., Academic Press, New York [1979]).

Unter einem Affinitätspeptid werden Peptide verstanden, die eine Aminosäuresequenz enthalten, die bevorzugt an ein Affinitätschromatographieträgermaterial bindet. Beispiele für solche Affinitätspeptide sind Peptide, die mindestens zwei Histidinreste enthalten (siehe hierzu europäische Patentanmeldung, Publ. Nr. 282 042). Solche Affinitätspeptide binden selektiv an Nitrilotriessigsäure-Nickelchelatharze [Hochuli and Döbeli, Biol. Chem. Hoppe-Seyler 368, 748 (1987); europäische Patentanmeldung, Publ, Nr. 253 303]. Polypeptide, die ein solches Affinitätspeptid enthalten, können damit selektiv von den übrigen Polypeptiden abgetrennt werden. Das Affinitätspeptid kann entweder mit dem C-Terminus oder dem N-Terminus der Polypeptide mit der Aminosäuresequenz der Formel I oder Fragmenten oder funktionellen Aequivalenten davon verknüpft sein.

Die erfindungsgemässen Polypeptide können aufgrund ihrer Herstellungsverfahren Methionin (für das ATG kodiert) als erste N-terminale Aminosäure enthalten. Andererseits kann der mikrobielle Wirt das Translationsprodukt teilweise oder vollständig prozessieren, wodurch das N-terminale Methionin abgespalten wird.

Die erfindungsgemässen Polypeptide können auch in Form von Multimeren, z.B. in Form von Dimeren, Trimeren, Tetrameren etc., vorliegen. Natürlich können die erfindungsgemässen Polypeptide auch mit Polypeptiden, deren Aminosäuresequenz mit mindestens einer antigenen und/oder immunogenen Determinante des HIV-2 Strukturproteins (gag) übereinstimmt, kovalent verknüpft sein.

Die Erfindung liefert ferner DNA-Sequenzen, die die erfindungsgemässen Polypeptide kodieren, rekombinante Vektoren die diese DNA-Sequenzen enthalten, einzellige Organismen zur Herstellung der erfindungsgemässen Polypeptide sowie Verfahren zur Herstellung solcher DNA-Sequenzen, rekombinanter Vektoren und einzelligen Organismen. Es werden auch Methoden zur Expression, Isolierung und Reinigung der erfindungsgemässen Polypeptide beschrieben. Die so hergestellten erfindungsgemässen Polypeptide können mit den Methoden dieser Erfindung für eine Anzahl wichtiger immunologischer Prozesse verwendet werden.

Die erfindungsgemässen Polypeptide können als diagnostisches Reagens zum Nachweis von Antikörpern gegen HIV-2 Viren oder zum gleichzeitigen Nachweis von Antikörpern gegen HIV-1 und HIV-2 Viren (im folgenden kollektiv als Antikörper gegen HIV-Viren bezeichnet) in menschlichen Seren verwendet werden. Da sie in homogener Form hergestellt werden können, können Probleme mit unspezifischen Reaktionen, die die Verwendung von diagnostischen Reagenzien auf der Basis relativ roher viraler HIV Proteinlysate in der Vergangenheit eingeschränkt haben, eliminiert werden.

Als Immunogen verwendet, können die erfindungsgemässen Polypeptide zur Produktion von Antikörpern, die gegen die in diesen Polypeptiden enthaltenen antigenen determinanten Gruppen gerichtet sind, in Tieren verwendet werden. Solche Antikörper wiederum können in Verbindung mit den erfindungsgemässen Polypeptiden, die entsprechend markiert wurden, in einem Radioimmunassay (RIA) oder in einem Enzymimmunassay (ELISA) verwendet werden, um die Gegenwart von HIV-2 Viren oder HIV-1 und HIV-2 Viren (im folgenden kollektiv als HIV-Viren bezeichnet) oder Partikeln davon in menschlichen Seren oder in anderen biologischen Flüssigkeiten, wie z.B. in Tränenflüssigkeit, Samen, Vaginalsekret und Speichel, festzustellen. Die Partikel (oder Fragmente) von HIV-Viren, die mit diesen Methoden nachgewiesen werden können, umfassen natürliche Teile der viralen HIV-Hüllproteine (env).

Die erfindungsgemässen Polypeptide können mittels konventioneller Methoden der Peptidsynthese, in flüssiger oder, vorzugsweise an fester Phase, wie der Methode von Merrifield (J. Am. Chem. Soc. 85, 2149-2154 [1963]), oder mittels anderer gleichwertiger Methoden des Standes der Technik, hergestellt werden.

Andererseits können die erfindungsgemässen Polypeptide auch unter Verwendung der Methoden der DNA-Rekombinationstechnik [Maniatis et al. in "Molecular Cloning - A Laboratory Manual", Cold Spring Harbor Laboratory (1982)] hergestellt werden. Beispielsweise können die DNA-Sequenzen, die für die erfindungsgemässen Polypeptide codieren, mittels konventioneller chemischer Methoden synthetisiert werden, z.B. mittels der Phosphotriestermethode [Narang et al., In Meth. Enzymol. 68, 90-108 (1979)] oder mittels der Phosphodiestermethode [Brown et al., Meth. Enzymol. 68, 109-151 (1979)]. Bei beiden Methoden werden in der Regel längere Oligonukleotide synthetisiert, die in vorgegebener Art und Weise aneinander gehängt werden. Die Nukleotidsequenzen der DNA-Fragmente können mit denjenigen Nukleotidsequenzen, die die natürlichen HIV-2 oder HIV-1 und HIV-2 Polypeptide codieren, identisch sein. Da der genetische Code degeneriert ist, besteht andererseits die Möglichkeit, dass teilweise oder vollständig unterschiedliche Nukleotidsequenzen die gleichen Polypeptide codieren. Gegebenenfalls können für die Nukleotidsequenzen solche Codons gewählt werden, die auch vom Wirtsorganismus, der zur Expression des Polypeptids verwendet wird, bevorzugt verwendet werden [Grosjean et al., Gene 18, 199-209 (1982)]. Dabei muss aber darauf geachtet werden, dass die so erhaltenen DNA-Sequenzen keine Teilsequenzen enthalten, die die Konstruktion von Expressionsvektoren, z.B. durch Einführung einer unerwünschten Restriktionsenzymschnittstelle, erschweren.

Im weiteren können die DNA-Sequenzen, die für die erfindungsgemässen Polypeptide kodieren, auch hergestellt werden, in dem man ein DNA-Fragment, das für die Aminosäuresequenz der Formel I kodiert, aus isolierter proviraler HIV-2 DNA oder aus genomischer DNA von Zellen, in welche das provirale HIV-2 Genom integriert wurde, isoliert und anschliessend in einen geeigneten Vektor, der die Teilsequenzen A und C der allgemeinen Formeln A-B-C, A-C-B und C-B-A kodiert, einbaut.

Nach der Herstellung der DNA-Sequenzen, die für die erfindungsgemässen Polypeptide kodieren, können diese nach bekannten Methoden in jeden geeigneten Expressionsvektor, der die notwendigen Expressionssignale liefert, eingebaut werden. Geeignete Vektoren können aus Segmenten von chromosomalen, nicht-chromosomalen und synthetischen DNA-Sequenzen zusammengesetzt sein, wie z.B. verschiedene bekannte Plasmide und Phagen DNA's. Hierzu kann auf das vorstehend genannte Lehrbuch von Maniatis et al. verwiesen werden. Besonders geeignete Vektoren sind Plasmide der pDS-Familie [Bujard et al., Methods in Enzymology, Hrsg. Wu und Grossmann, Academic Press, Inc., Vol. 155, 416-433 (1987)].

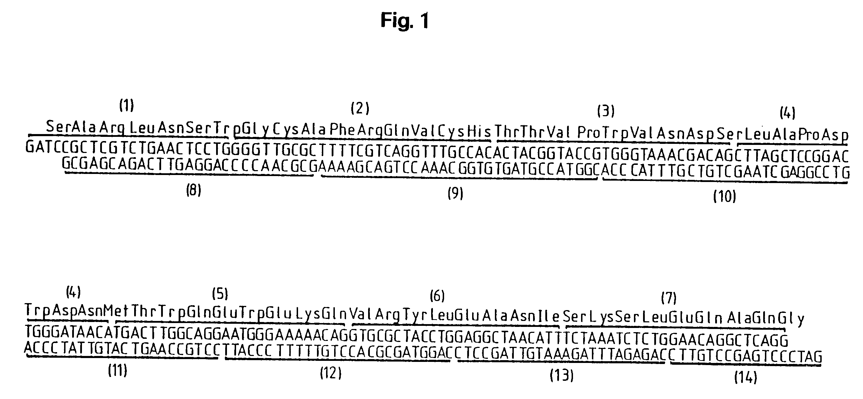

In den bevorzugten Ausführungsformen der vorliegenden Erfindung wurde ein synthetisches BamH1 Fragment, welches die Aminosäuresequenz der Formel I kodiert (env(60)-Gen), mit BamH1 bzw. BglII gespaltener DNA des Plasmids pDS78/RBSII,6xHis und mit BglII gespaltener DNA des Plasmids pRBSII-env(80)-gag(419)-6xHis verbunden, um die Expressionsvektoren penv(60)-DHFR, pDHFR-env(60) und pRBSII-env(80)-gag(419)-env(60)-6xHis zu isolieren, welche die Synthese der besonders bevorzugten erfindungsgemässen Polypeptide der Formeln II-IV kodieren. Die Synthese des synthetischen env(60)-Gens ist in Beispiel 1 beschrieben. Seine Nukleotidsequenz und die davon abgeleitete Aminosäuresequenz (ENV-(60)) ist in Figur 1 gezeigt. Die Konstruktionen der Plasmide pDS78/RBSII, 6xHis, penv(60)-DHFR, pDHFR-env(60), pRBSII-env(80)-gag(419)-6xHis und pRBSII-env(80)-gag(419)-env(60)-6xHis werden im Detail in den Beispielen 2-4, 6 und 7 beschrieben.

Die Expressionsvektoren, welche die DNA-Sequenzen, die für die erfindungsgemässen Polypeptide kodieren, operativ mit einer Expressionskontrollsequenz verknüpft enthalten, können in an sich bekannter Weise in jeden geeigneten Wirtsorganismus eingeführt werden. Die Auswahl eines geeigneten Wirtsorganismus wird von verschiedenen dem Fachmann bekannten Faktoren bestimmt. So spielen beispielsweise Kompatibilität mit dem ausgewählten Vektor, Toxizität des Expressionsproduktes, Expressionscharakteristika, notwendige biologische Sicherheitsvorkehrungen und Kosten eine Rolle und es muss ein Mittelweg zwischen allen diesen Faktoren gefunden werden.

Als geeignete Wirtsorganismen kommen gram-negative und gram-positive Bakterien in Frage, beispielsweise E. coli und B. subtilis Stämme. Besonders bevorzugte Wirtsorganismen der vorliegenden Erfindung sind der E. coli Stamm M15 (von Villarejo et al. in J. Bacteriol. 120, 466-474 [1974] als OZ 291 beschrieben) und E. coli W3110 (ATCC Nr. 273325). Ausser den oben genannten E. coli Stämmen können aber auch andere allgemein zugängliche E. coli Stämme, beispielsweise E. coli 294 (ATCC Nr. 31446) und E. coli RR1 (ATCC Nr. 31343) verwendet werden.

Die Art und Weise wie die Expression der erfindungsgemässen Polypeptide erfolgt ist abhängig vom gewählten Expressionsvektor/Wirtszellsystem. Ueberlicherweise werden die Wirtsorganismen, die einen gewünschten Expressionsvektor enthalten, unter Bedingungen, die für das Wachstum der Wirtsorganismen optimal sind, vermehrt. Gegen Ende des exponentiellen Wachstums, wenn die Zunahme der Zellzahl pro Zeiteinheit abnimmt, wird die Expression des gewünschten Polypeptids induziert, d.h. die das gewünschte Polypeptid kodierende DNA wird transkribiert und die transkribierte mRNA wird translatiert. Die Induktion kann durch Zugabe eines Induktors oder eines Derepressors zum Wachstummedium oder durch Veränderung eines physikalischen Parameters, z.B. einer Temperaturänderung, erfolgen. In den in den bevorzugten Ausführungsformen der vorliegenden Erfindung verwendeten Expressionsvektoren, wird die Expression durch den lac-Repressor kontrolliert. Durch Zugabe von Isopropyl-$\beta$-D-thiogalactopyranosid (IPTG) wird die Expressionskontrollsequenz dereprimiert und dadurch die Synthese des gewünschten Polypeptids induziert.

Zur Isolierung von geringen Mengen an erfindungsgemässen Polypeptiden für analytische Zwecke, z.B. für Polyacrylamidgelektrophoresen, können die Wirtsorganismen mittels eines Detergenz, z.B. Natriumdodecylsulfat (SDS) aufgeschlossen werden. Zur Isolierung von grossen Mengen an erfindungsgemässen Polypeptiden können die Wirtsorganismen durch mechanische [Charm et al., Meth. Enzymol. 22, 476-556 (1971)], enzymatische (Lysozymbehandlung) oder chemische (Detergenzbehandlung, Harnstoff- oder Guanidiniumchlorid-Behandlung, etc.) Mittel, oder durch eine Kombination dieser Mittel, aufgeschlossen werden.

Nachdem die erfindungsgemässen Polypeptide aus den Wirtsorganismen herausgelöst sind, können sie mittels bekannter Methoden, z.B. mittels Zentrifugation bei unterschiedlichen Geschwindigkeiten, mittels Präzipitation mit Ammoniumsulfat, mittels Dialyse (bei Normaldruck oder bei reduziertem Druck), mittels präparativer Isoelektrofokussierung, mittels präparativer Gelelektrophorese oder mittels verschiedener chromatographischer Methoden wie Gelfiltration, Hochleistungs-Flüssigkeitschromatographie (HPLC), Ionenaustauscherchromatographie, Umkehrphasenchromatographie und Affinitätschromatographie (z.B. an Sepharose Blau C1-6B oder an trägergebundenen, gegen die erfindungsgemässen Polypeptide gerichteten monoklonalen Antikörpern) gereinigt werden. Vorzugsweise jedoch werden die erfindungsgemässen Polypeptide an Nitrilotriessigsäure (NTA)-Harzen der allgemeinen Formel Trägermatrix-Spacer-NH-$(CH_2)_x$ -CH(COOH-N-$(CH_2COO^-)_2Ni^{2+}$, worin x 2, 3 oder 4 bedeutet, gereinigt. Als Trägermatrix kommen Materialien in Frage, wie sie in der Affinitäts- und Gelchromatographie verwendet werden, beispielsweise vernetzte Dextrane, Agarose (insbesondere in der unter dem Warenzeichen Sepharose® bekannten Form) oder Polyacrylamide. Als Spacer kommen die aus der Affinitäts-Chromatographie bereits bekannten Spacer-Gruppen in Frage, wobei die Gruppen -O-$CH_2$-CH(OH) -$CH_2$- und -O-CO- bevorzugt sind.

Ein besonders bevorzugtes NTA-Harz für die Reinigung der erfindungsgemässen Polypeptide ist das der Formel

[Sepharose®CL 6B]-O-$CH_2$-CH(OH)-$CH_2$-NH-$(CH_2)_4$CH(COOH)-N$(CH_2COO^-)_2Ni^{2+}$.

Die nach den vorstehend beschriebenen Methoden erhältlichen erfindungsgemässen Polypeptide können, wie bereits vorstehend erwähnt, als diagnostische Werkzeuge zum Nachweis von Antikörpern gegen HIV-Viren in menschlichen Seren verwendet werden. Die erfindungsgemässen Polypeptide können zu diesem Zweck in zahlreichen, bekannten Nachweisverfahren verwendet werden.

Beispielsweise können die erfindungsgemässen Polypeptide nach bekannten Methoden markiert werden und diese markierten Polypeptide dann zur Ausbildung von Polypeptid/Antikörper-Komplexen mit einer menschlichen Serumsprobe, die im Verdacht steht Antikörper gegen HIV-Viren zu enthalten, vermischt werden. Die gebildeten Komplexe können dann in an sich bekannter Weise nachgewiesen werden. Die erfindungsgemässen Polypeptide können aber auch an einen festen Träger gekoppelt und dann mit einer menschlichen Serumsprobe in Kontakt gebracht werden, um ein weiteres Beispiel zu geben. Antikörper gegen HIV-Viren in der Probe binden an dieses gekoppelte Polypeptid und die so gebildeten Komplexe können, nachdem nicht-gebundene Polypeptide und Antikörper durch Waschung entfernt wurden, mittels eines Reagenz, wie beispielsweise Staphylococcus aureus Protein A (beispielsweise mit $^{125}$Jod markiert) oder einem zweiten anti-Ig-Antikörper (beispielsweise mit einem Radioisotop oder mit Meerrettichperoxidase markiert) nachgewiesen werden. Viele Modifikationen und Variationen dieser vorstehend genannten Nachweismethoden liegen für den Fachmann im Bereich des möglichen, von denen einige im folgenden vorgeschlagen werden.

Durch Verwendung von Antikörpern gegen die erfindungsgemässen Polypeptide (im folgenden anti-HIV-Antikörper) können verschiedene diagnostische Tests zur Erkennung von HIV-Viren oder Fragmenten davon in menschlichen Seren oder in anderen biologischen Flüssigkeiten entwickelt werden. Solche Antikörper können durch Injektion einer immunogenen Zusammensetzung, enthaltend ein erfindungsgemässes Polypeptid und ein physiologisch verträgliches Trägermaterial, in einen Säuger oder Vogel hergestellt werden. Die für die Injektion notwendige Menge Protein ist dem Fachmann bekannt oder kann nach bekannten Methoden bestimmt werden. Der im Zusammenhang mit der vorliegenden Erfindung verwendete Ausdruck "Tragermaterial" bezieht sich entweder auf bekannte Zusammensetzungen, die für menschliche Verabreichung geeignet sind, oder auf bekannte, bei der Tierimpfung verwendete Adjuvantien. Geeignete Adjuvantien für die Anwendung an Mensch und Tier sind dem Fachmann bekannt [WHO Techn. Rep. Series 595, 1-40 (1976); Jollis et al., "Chemical and Biological Basis of Adjuvants", in Molecular Biochemistry and Biophysics Vol. 13, 1-148 (1973) Springer Verlag Berlin]. Die erfindungsgemässen Polypeptide können aber auch nach Einbau in Liposomen oder andere Mikro-Trägermaterialien oder nach Kopplung an Polysaccharide, andere Polypeptide oder andere Polymere verabreicht werden.

In einem besonders typischen Beispiel folgen einige Wochen nach der ersten Impfung eine oder mehrere Zusatzimpfungen, was einen hohen Gehalt an anti-HIV-Antikörpern zur Folge hat. Diese können dann in an sich bekannter Weise isoliert werden.

Natürlich können auch monoklonale Antikörper für die vorstehend genannten Tests verwendet werden. Die Methoden zur Herstellung solcher Antikörper sind dem Fachmann bekannt [Köhler et al., Nature 256, 495-497 (1975)].

Die nach den vorstehend beschriebenen Methoden erhältlichen anti-HIV-Antikörper können, wie bereits vorstehend erläutert, für verschiedene diagnostische Tests zum Nachweis von HIV-Viren oder Fragmenten davon verwendet werden. Solche Tests können in Form von Radioimmunoassays, entweder in Lösung oder an festem Träger, durchgeführt werden. Es können aber auch Enzymimmunoassays durchgeführt werden. Diese Test konnen entweder direkt oder indirekt mittels eines zweiten Antikörpers, der gegen die anti-HIV-Antikörper gerichtet ist, durchgeführt werden. Zahlreiche Enzymaktivitäten können an die Antikörper gekoppelt werden, z.B. Peroxidase, Glucoseoxidase, $\beta$-Galactosidase und Alkalische Phosphatase, die nach Zugabe einer Substratlösung eine Färbung erzeugen.

Das Prinzip das vielen dieser Tests zugrunde liegt beruht darauf, dass man menschliches Serum oder andere biologische Flüssigkeiten, die im Verdacht stehen HIV-Viren oder Fragmente davon zu enthalten, mit einer bekannten Menge an anti-HIV-Antikörpern reagieren lässt, so dass Antigen/Antikörper-Komplexeentstehen können und diese Komplexe in an sich bekannter Weise nachweist.

Der Fachmann wird auch erkennen, dass es viele andere Nachweisverfahren gibt, bei denen anti-HIV-Antiseren zum Einsatz kommen, wie z. B. verschiedene Agglutinationstests. Hierbei wird die Wechselwirkung zwischen Antikörpern und HIV-Viren oder Fragmenten davon mittels Anordnungen, in denen Partikel mit anti-HIV-Antikörpern überzogen sind, nachgewiesen. Solche Partikel sind beispielsweise Latex-Kugeln, Liposomen, Erythrozyten, Polyacrylamidkugeln oder irgendein geeignetes Polymer.

Die vorstehend beschriebenen Methoden zum Nachweis von HIV-Viren oder von Antikörpern gegen HIV-Viren können in geeigneten Test-Kits bestehend aus einem Gefäss, welches ein erfindungsgemässes Polypeptid oder anti-HIV-Antikörper der vorliegenden Erfindung enthält, durchgeführt werden.

Die folgenden Figuren und die nachfolgenden Beispiele tragen zum besseren Verständnis der vorliegenden Erfindung bei, ohne sie zu beschränken.

In den Figuren treten die folgenden Abkürzungen und Symbole auf:

B, Bg, E, H, P, Sa, X und Xb bezeichnen Schnittstellen für die Restriktionsenzyme BamHI, BglII, EcoRI, HindIII, PstI, SalI, XhoI bzw. XbaI.

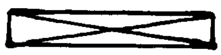

repräsentiert die Promotoren der Gene bla, lacI und neo;

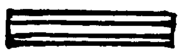

repräsentiert die ribosomalen Bindungsstellen der Gene bla, cat, neo und lacI;

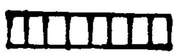

repräsentiert die Terminatoren $t_0$ und T1;

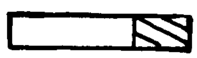

repräsentiert das regulierbare Promotor/Operator Element N25OPSN25OP29;

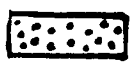

repräsentiert die ribosomale Bindungstelle RBSII;→ repräsentiert die kodierende Region unter Kontrolle dieser ribosomalen Bindungsstelle;

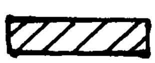

repräsentiert die Regionen, die für die sechs Histidine kodiert;

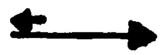

repräsentiert die für die Replikation benötigte Region (repl.);

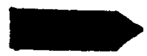

repräsentiert kodierende Regionen für Dihydrofolatreduktase (dhfr), Chloramphenicolacetyltransferase, lac-Repressor (lacI), β-Lactamase (bla) und Neomycinphosphotransferase (neo);

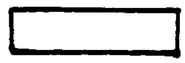

repräsentiert das synthetische env(60)-Gen;

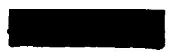

repräsentiert HIV-1 gag-Genfragmente.

Fig. 1 Darstellung der Nukleotidsequenz des synthetischen HIV-2 env(60)-Gens und die davon abgeleitete Aminosäuresequenz des HIV-2 ENV(60) Polypeptide. Die individuellen Oligonukleotidfragmente, aus denen das synthetische HIV-2T env(60)-Gen zusammengesetzt ist, sind durch die Zahlen 1-14 gekennzeichnet.

Fig. 2 Schematische Darstellung des Plasmids pDS78/RBSII.

Fig. 3 Nukleotidsequenz des Plasmid pDS78/RBSII. In der Sequenz sind die Erkennungsstellen für die in Fig. 2 aufgeführten Restriktionsenzyme überstrichen, während die für β-Lactamase bzw. Dihydrodfolatreduktase kodierenden Regionen unterstrichen sind.

Fig. 4 Schematische Darstellung des Plasmids pDMI,1

Fig. 5 Nukleotidsequenz des Plasmids pDMI,1. In der Sequenz sind die Erkennungsstellen für die in Fig. 4 aufgeführten Restriktionsenzyme überstrichen, während die für Neomycinphosphotransferase bzw. lac-Repressor kodierenden Regionen unterstrichen sind.

Fig. 6 Schematische Darstellung der Herstellung des XhoI/BamHI-Fragments mit dem regulierbaren Promotor/Operator-Element N250PSN250P29, der ribosomalen Bindungsstelle RBSII sowie der für sechs Histidine kodierenden Region.

Fig. 7 Schematische Darstellung der Konstruktion des Plasmids pDS78/RBSII, 6xHis unter Verwendung des Plasmids pDS78/RBSII und des XhoI/BamHI-Fragments mit dem regulierbaren Promotor/Operator-Element N250PSN250P29, der ribosomalen Bindungsstelle RBSII sowie der für sechs Histidine kodierenden Region.

Fig. 8 Schematische Darstellung der Konstruktion des Plasmides penv(60)-DHFR.

Fig. 9 Schematische Darstellung der Konstruktion des Plasmids pDHFR-env(80).

Fig. 10 Reaktivität der ENV(60)-DHFR und DFHR-ENV(60) Polypeptide mit HIV positiven Seren. Der obere Teil zeigt die elektrophoretische Analyse von E. coli M15 Lysaten enthaltend die Plasmide pDS78/RBSII, 6xHis (Spur a), penv(60)-DHFR (Spur b) bzw. pDHFR-env(60) (Spur c). Spur d enthält gereinigtes HIV-1 ENV(80)-DHFR. Der untere Teil zeigt die entsprechenden Immunoblots, die mit HIV-2 (links) bzw. HIV-1 (rechts) positivem Serum entwickelt wurden. Die M Spuren enthalten vorgefärbte Molekulargewichtsstandards, deren Grösse in Kilodalton angegeben ist.

Fig. 11 Schematische Darstellung des Plasmids pDS56/RBSII.

Fig. 12 Nukleosidsequenz des Plasmids pDS56/RBSII. Die in Abbildung 11 aufgeführten Schnittstellen für Restriktionsenzyme sind überstrichen während die unter Kontrolle der RBSII stehende Region unterstrichen ist.

Fig. 13 Schematische Darstellung der Konstruktion und Isolierung des BamHI/XbaI-Fragments mit der für 6 Histidine kodierenden Region dem Terminator $t_o$, dem cat-Gen und dem Terminator T1, welches bei der Konstruktion des Plasmids pRBSII-6xHis eingesetzt wurde.

Fig. 14 Schematische Darstellung der Konstruktion des Plasmids pRBSII-6xHis durch Verknüpfen des in Abbildung 13 gezeigten BamHI/XbaI-Fragments mit dem die Replikationsregion enthaltenden XbaI/BamHI-Fragment des Plasmids pDS56/RBSII.

Fig. 15 Schematische Darstellung der Herstellung des HIV-1 BamHI/BglII-gag(419)-Genfragments.

Fig. 16 Darstellung der Nukleotidsequenz des im Plasmid pU-GAG enthaltenen HIV-1 gag-Genfragments.

Fig. 17 Darstellung der Nukleotidsequenz des im Plasmid p2-3U/HindIII 10 enthaltenen HIV-1 gag-Genfragments.

Fig. 18 Schematische Darstellung der Konstruktion des Plasmids pRBSII-gag(419)-6xHis.

Fig. 19 Schematische Darstellung der Konstruktion des Plasmids pRBSII-env(80)-gag(419)-6xHis.

Fig. 20 Schematische Darstellung der Konstruktion des Plasmids pRBSII-env(80)-gag(419)-env(60)-6xHis.

## Beispiel 1

## Herstellung des synthetischen env(60)-Gens

### A) Prinzipien

Das synthetische HIV-2 env(60)-Gen wurde aus 14 Oligonukleotidfragmenten, deren Länge zwischen 17 und 31 Nukleotiden variierte, zusammengesetzt (siehe Figur 1).

### B) Synthese der Oligonukleotidfragmente

Die Oligonukleotidfragmente 1-14 wurden simultan, an festem Trägermaterial nach dem von Bannwarth und Iaiza in DNA 5, 413-419 (1986) beschriebenen Verfahren synthetisiert.

### C) Zusammensetzen der Oligonukleotidfragmente

Je 100 pmol der Oligonukleotidfragmente 2, 3, 8, 9, 10 bzw. 4, 5, 6, 7, 11, 12, 13 wurden in 100 µl Kinasepuffer [Maniatis et al., "Molecular Cloning", Cold Spring Harbor Laboratory (1982)] enthaltend 100 Einheiten Polynukleotidkinase und 100 µCi $\gamma$-$^{32}$ P-ATP (5000 Ci/mmol, bei 37°C während 15 Minuten phosphoryliert. Danach wurde ein 10-facher Ueberschuss an kaltem ATP zu den Reaktionsmischungen hinzugefügt. Nach einer weiteren Inkubationszeit von 90 Minuten wurde die Polynukleotidkinase hitzeinaktiviert (2 Min., 95°C) und die phosphorylierten Oligonukleotidfragmente wurden nach Zugabe von µl 5M Lithiumacetat (LiOAc) und 100 µl Isopropanol während 30 Minuten bei -78°C präzipitiert. Die präzipitierten Oligonukleotidfragmente wurden dann mit Aethanol gewaschen und getrocknet. Die phosphorylierten Oligonukleotidfragmente 2, 3, 8, 9, 10 und das nicht phosphorylierte Fragment 1 bzw. die phosphorylierten Oligonukleotidfragmente 4, 5, 6, 7, 11, 12, 13 und das nicht phosphorylierte Fragment 14 wurden nach bekannten, in der Literatur beschriebenen Methoden [Maniatis et al., supra] hybridisiert und dann in 100 µl Ligasepuffer [Maniatis et al., supra] enthaltend 31 Einheiten T4-Ligase ligiert (37°C, 1,5 Stunden). Anschliessend wurden die beiden erhaltenen Subfragmente, wie vorstehend beschrieben, präzipiert, dann mit Aethanol gewaschen und getrocknet. Die beiden Subfragmente wurden nachfolgend mittels 6% Polyacrylamidgelelektrophorese aufgetrennt, nach Standardmethoden eluiert [Maniatis et al., supra], mittels einer Sephadex G-50 Säule entsalzt und mittels T4-Ligase, wie vorstehend beschrieben, zum gewünschten HIV-2 env(60)-Gen miteinander verknüpft. Nach der Reinigung des HIV-2 env(60)-Gens mittels den vorstehend beschriebenen Verfahren (Polyacrylamidgelelektrophorese, Elution und Entsalzen) wurde es, wie vorstehend beschrieben, phosphoryliert.

## Beispiel 2

## Konstruktion des Plasmids pDS78/RBSII,6xHis

### A. Beschreibung der Plasmide pDS78/RBSII und pDMI,1

Zur Konstruktion des Plasmids pDS78/RBSII,6xHis wurde das Plasmid pDS78/RBSII verwendet. Mit diesem Plasmid sowie dem Plasmid pDMI,1 transformierte E. coli Zellen wurden bei der Deutschen Sammlung von Mikroorganismen in Göttingen am 3. September 1987 nach dem Budapester Vertrag hinterlegt [E. coli M15 (pDS78/RBSII, pDMI,1), DSM-Nr: 4232].

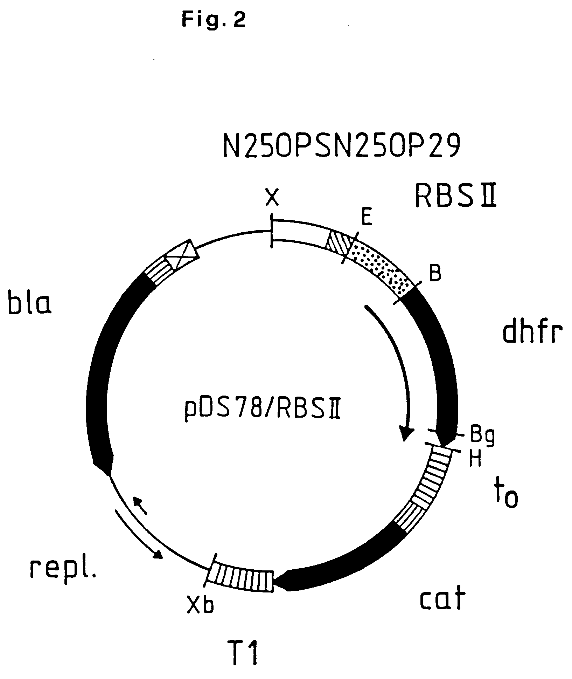

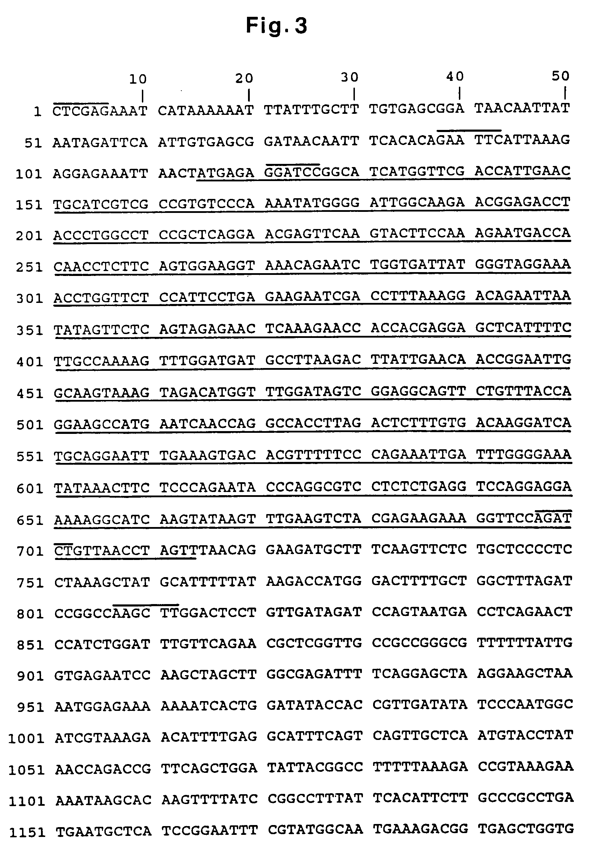

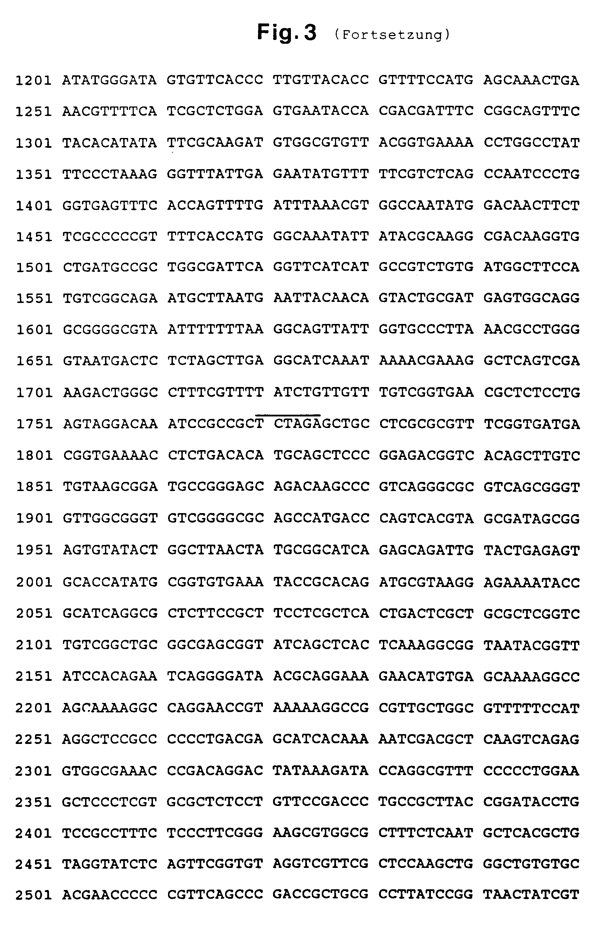

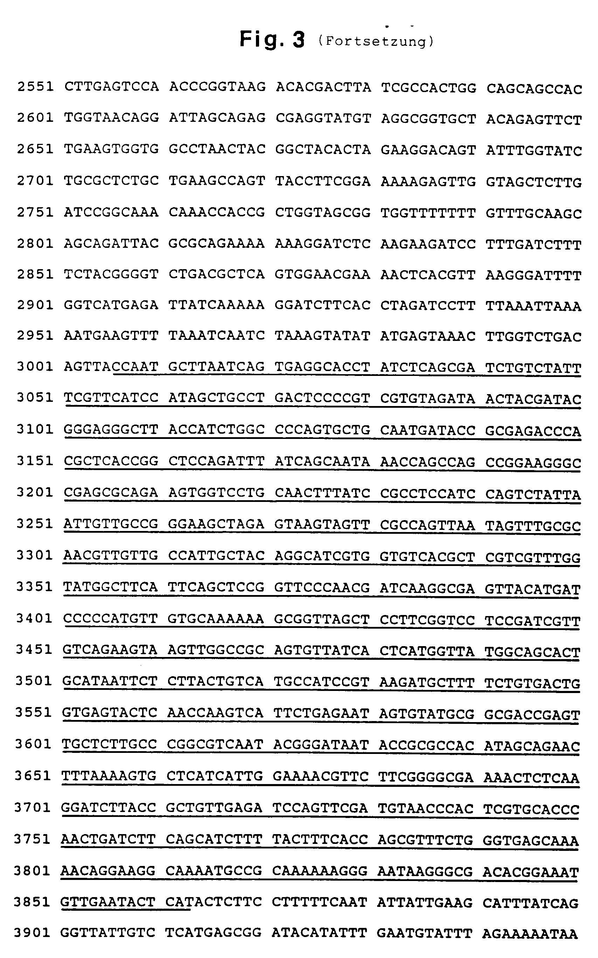

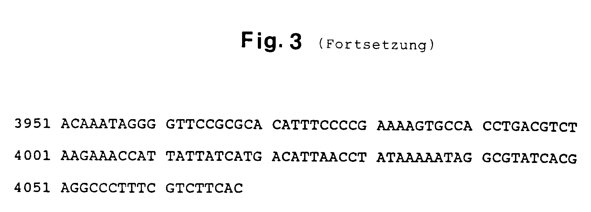

Der Anteil von pDS78/RBSII (Fig. 2 und 3), der zwischen den Restriktionsschnittstellen für XbaI und XhoI liegt und die Replikationsregion sowie das Gen für β-Lactamase, das den Zellen Ampicillinresistenz verleiht, enthält, stammt ursprünglich aus dem Plasmid pBR322 [Bolivar et al., Gene 2, 95-113 (1977); Sutcliffe, Cold Spring Harbor Symp. Quant. Biol. 43, 77-90 (1979)]. Jedoch ist das Gen für die β-Lactamase dahingegen modifiziert, dass die Schnittstellen für die Restriktionsenzyme HincII und PstI eliminiert sind. Diese Veränderungen in der DNA-Sequenz wirken sich aber nicht auf die Aminosäuresequenz der β-Lactamase aus. Der verbleibende Teil des Plasmids trägt das regulierbare Promotor/Operator-Element N25OPSN25OP29 und die ribosomale Bindungsstelle RBSII. Diese ribosomale Bindungsstelle wurde von der ribosomalen Bindungsstelle des Promotors $P_{G25}$ des E.coli-Phagen T5 [R. Gentz, Dissertation, Universität Heidelberg, BRD (1984)] abgeleitet und als EcoRI/BamHI-Fragment über DNA-Synthese erhalten. Es folgt das Gen der Dihydrofolatreduktase der Maus-Zellinie AT-3000 [Chang et al., Nature 275, 617-624 (1978); Masters et al., Gene 21, 59-63 (1983)], das dahingehend verändert wurde, dass direkt vor dem Terminationscodon für die Translation eine Schnittstelle für das Restriktionsenzym BglII eingeführt wurde. Weiterhin enthält das Plasmid pDS78/RBSII den Terminator $t_o$ des E. coli-Phagen Lambda [Schwarz et al., Nature 272, 410-414 (1978)], das Promotor-freie Gen der Chloramphenicolacetyltransferase [Marcoli et al., FEBS Letters, 110, 11-14 (1980)] und den Terminator T1 des E. coli rrnB Operons [Brosius et al., J. Mol. Biol., 148, 107-127 (1981)].

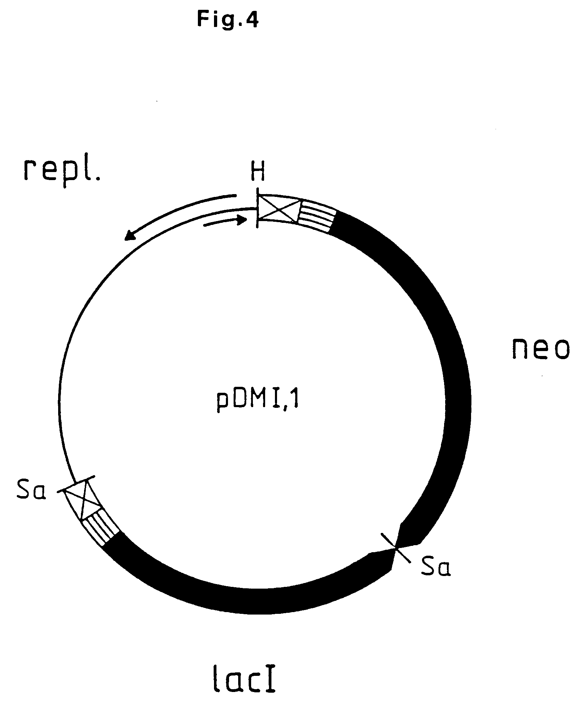

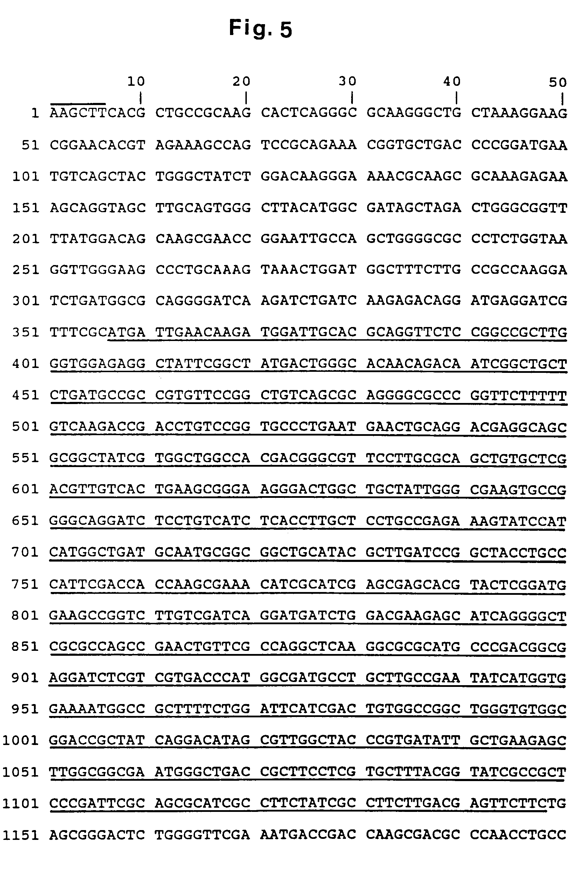

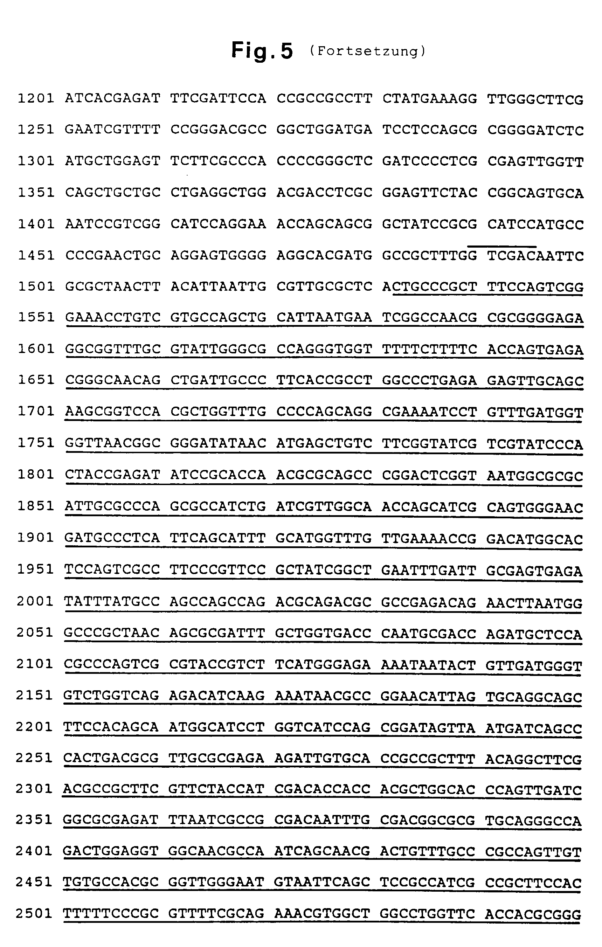

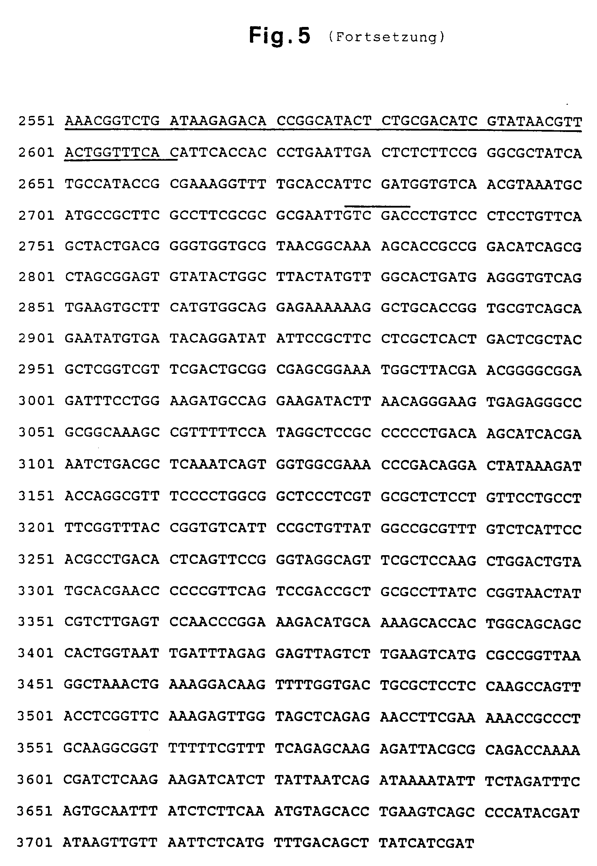

pDS78/RBSII enthält das regulierbare Promotor/Operator-Element N25OPSN25OP29 sowie die ribosomale Bindungsstelle RBSII. Auf Grund der hohen Effizienz dieser Expressionssignale können das Plasmid pDS78/RBSII und seine Derivate wie das Plasmid pDS78/RBSII,6xHis nur dann stabil in E.coli-Zellen gehalten werden, wenn das Promotor/Operator Element durch das Binden eines lac-Repressors an den Operator reprimiert wird. Der lac-Repressor ist im lacI-Gen kodiert. N25OPSN25OP29 kann nur dann effizient reprimiert werden, wenn eine ausreichende Zahl von Repressormolekülen in den Zellen vorhanden ist. Daher wurde das $lacI^q$-Allel benutzt, das eine Promotormutante enthält, die zu einer erhöhten Expression des Repressorgens führt. Dieses $lacI^q$-Allel ist im Plasmid pDMI,1 (Fig. 4 und 5) enthalten. Dieses Plasmid trägt zusätzlich zum lac-I-Gen das neo-Gen, das den Bakterien Kanamycinresistenz verleiht, welche als Selektionsmarker benutzt wird. pDMI,1 ist mit den oben erwähnten Plasmiden kompatibel. E.coli-Zellen, die mit solchen Expressionsvektoren transformiert werden, müssen pDMI,1 enthalten, um sicherzustellen, dass der Expressionsvektor stabil in den Zellen gehalten wird. Eine Induktion dieses Systems wird durch Zugabe von IPTG ins Medium bei der gewünschten Zelldichte erreicht.

Das Plasmid pDMI,1 (Fig. 4 und 5) trägt das neo-Gen der Neomycinphosphotransferase aus dem Transposon Tn5 [Beck et al., Gene 19, 327-336 (1982)], das E.coli-Zellen Kanamycinresistenz verleiht und das lac I-Gen [Farabough, Nature 274, 765-769 (1978)] mit der Promotormutation $I^q$ [Calos, Nature 274, 762-765 (1978)], das den lac-Repressor kodiert. Ausserdem enthält das Plasmid pDMI,1 eine Region des Plasmids pACYC184 [Chang et al., J. Bacteriol. 134 1141-1156 (1978)], die alle Informationen enthält, die für die Replikation und stabile Weitergabe an die Tochterzellen notwendig sind.

B. Konstruktion des Plasmids pDS78/RBSII,6xHis

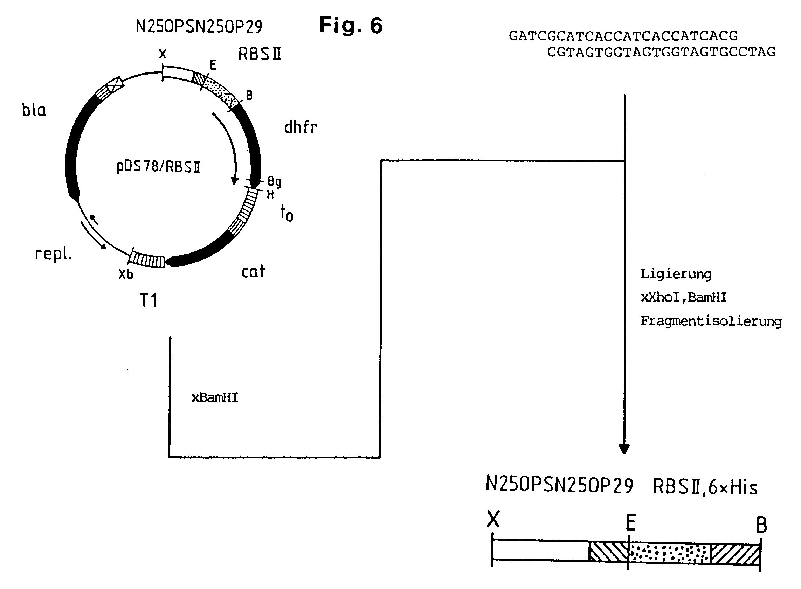

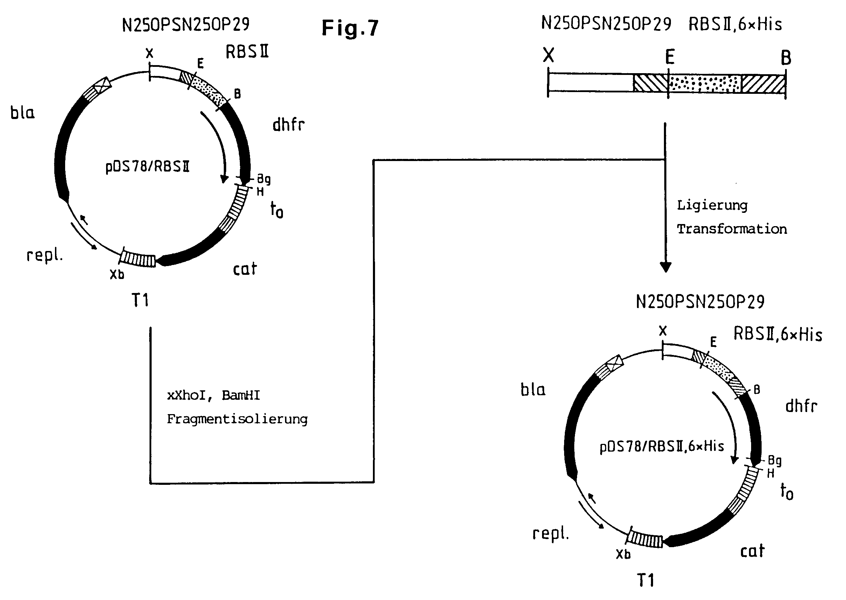

Zur Konstruktion des Plasmids pDS78/RBSII,6xHis (Fig. 6 und 7) wurde das EcoRI/BamHI-Fragment von pDS78/RBSII mit der ribosomalen Bindungsstelle RBSII um eine Region verlängert, die für sechs Histidine kodiert.

Dazu wurden zunächst zwei komplementäre Oligonukleotide, deren Nukleotidsequenz in Fig. 6 als doppelsträngige DNA-Sequenz dargestellt ist, wie vorstehend beschrieben (Bsp. 1, Abschnitt B), synthetisiert. Die lyophilisierten Oligonukleotide wurden in Wasser aufgenommen und 1 Stunde bei 4°C gelöst. Die DNA-Konzentration betrug 100 nmol/ml. Zur Phosphorylierung wurden jeweils 150 pmol der beiden Oligonukleotide in 20 µl 50 mM Tris/HCl [pH 8,5) und 10 mM $MgCl_2$ mit 2 pmol $\gamma$-[$^{32}P$]-ATP (5000 Ci/mmol) und 1 Einheit (E) Polynukleotidkinase während 20 Minuten bei 37°C inkubiert. Anschliessend wurden 5 nmol ATP zugegeben und nach weiteren 20 Minuten bei 37°C wurden die Reaktionen durch Erhitzen auf 65°C beendet.

Die DNA des Plasmids pDS78/RBSII wurde zur Ligierung mit den beiden phosphorylierten Oligonukleotiden vorbereitet, indem zunächst 2 pmol der Plasmid DNA mit dem Restriktionsenzym BamHI geschnitten wurden. Die Probe wurde mit Phenol extrahiert, ausgeäthert und die DNA mittels Lithiumacetat/Isopropanol, wie vorstehend beschrieben, ausgefällt. Das Sediment wurde getrocknet und in 20 µl TE-Puffer aufgenommen. Zur Ligierung mit den phosphorylierten Oligonukleotiden wurden nun 1,5 pmol der mit BamHI geschnittenen Plasmid-DNA mit je 60 pmol der phosphorylierten Oligonukleotide in Ligasepuffer mit 2 Einheiten T4-DNA Ligase während 2 Stunden bei 15°C inkubiert. Nach einer Inkubation bei 65°C während 5 Minuten wurde die ligierte DNA mit den Restriktionsenzymen XhoI und BamHI geschnitten. Danach wurde das XhoI/BamHI-Fragment mit dem regulierbaren Promotor N25OPSN25OP29, der ribosomalen Bindungsstelle RBSII sowie der für 6 Histidine kodierenden Region (Fig. 6) mittels Agarose Gelelektrophorese isoliert wurde.

Zur Konstruktion des Plasmids pDS78/RBSII,6xHis wurde das genannte XhoI/BamHI-Fragment in das Plasmid pDS78/RBSII integriert, wobei das ursprüngliche XhoI-BamHI-Fragment dieses Plasmids ersetzt wurde (Fig. 7). Dazu wurden zunächst 1 pmol DNA des Plasmids pDS78/RBSII mit den Restriktionsenzymen XhoI und BamHI geschnitten, bevor das grössere DNA Fragment mittels Agarose Gelelektrophorese isoliert wurde. 0,05 pmol dieses Fragments wurden nun mit 0,1 pmol des isolierten XhoI/BamHI-Fragments in Ligierungspuffer mit 2 Einheiten T4-DNA Ligase während 2 Stunden bei 15°C inkubiert. E.coli M15 Zellen enthaltend Plasmid pDMI,1 wurden nach der Methode von Morrison [Methods Enzymol. 68, 326-331 (1979)] für die Transformation vorbereitet. Die Ligierungsmischung wurde nach 7-minütigem Erhitzen auf 65°C zu 200 µl kompetenter Zellen hinzugefügt. Die Mischung wurde 30 Minuten in Eis gehalten, dann 2 Minuten bei 42°C inkubiert, und nach der Zugabe von 0,5 ml LB-Medium während 90 Minuten bei 37°C inkubiert. Die Zellen wurden dann auf LB-Agarplatten, die 100 µg/ml Ampicillin und 25 µl/ml Kanamycin enthielten, ausplattiert und über Nacht bei 37°C inkubiert. Einzelne Kolonien wurden mit einem sterilen Zahnstocher gepickt, in ein Röhrchen überführt, das 10 ml LB-Medium mit 100 µl/ml Ampicillin und 25 µg/ml Kanamycin enthielt, und 12 Stunden im Schüttelinkubator gehalten. Danach wurden die Zellen sedimentiert und die Plasmid-DNA nach der Methode von Methode von Birnboim und Doly [Nucleic Acids Res. 7, 1515-1523 (1979)] isoliert.

Je 0,2 µg der isolierten Plasmide wurden mit den Restriktionsenzymen XhoI und BamHI geschnitten, um nachzuprüfen, ob ein Fragment, welches das regulierbare Operator/Repressor-Element N25OPSN25OP29, die ribosomale Bindungsstelle RBSII sowie die für die sechs Histidine kodierende Region enthält, in diesen Plasmiden enthalten ist. Plasmide mit einem solchen Fragment erhielten die Bezeichnung pDS78/RBSII,6xHis (Fig. 7).

Um nachzuweisen, dass die richtige Sequenz in pDS78/RBSII,6xHis enthalten ist, wurde die doppelsträngige zirkuläre Plasmid-DNA sequenziert, wobei eine mit γ-[$^{32}$P]-ATP markierte Startersequenz (primer) benutzt wurde. Diese Startersequenz enthält die Nukleotide von Position 89-108 des Plasmids pDS78/RBSII. 0,3 pmol der isolierten Plasmid-DNA wurden mit Alkohol gefällt, das Sediment einmal mit 80% Aethanol gewaschen, getrocknet und schliesslich in 8 µl 1/4 Te-Puffer gelöst. Die Probe wurde für 5 Minuten bei 95°C inkubiert, auf 0°C abgekühlt und zentrifugiert (Eppendorf-Tischzentrifuge, 2 Minuten, 12 000 Upm). 1,5 pmol Startersequenz in einem Volumen von 2 µl wurden zugegeben bevor die Probe zunächst für 2 Minuten bei 95°C und dann für 5 Minuten bei 42°C inkubiert wurde. Dann wurde die DNA nach der Methode von Sanger et al. [Proc. Natl. Acad. Sci. USA 74, 5463-6567 (1977) sequenziert. Da ein radioaktiver markierter "Primer" verwendet wurde, wurden alle Reaktionen mit unmarkierten Deoxynukleotidtriphosphaten durchgeführt. Die DNA-Sequenzanalyse ergab, dass pDS78/RBSII,6xHis die in Fig. 6 angegebene Sequenz enthält.

Beispiel 3

Konstruktion des Plasmids penv(60)-DHFR

A) Prinzipien

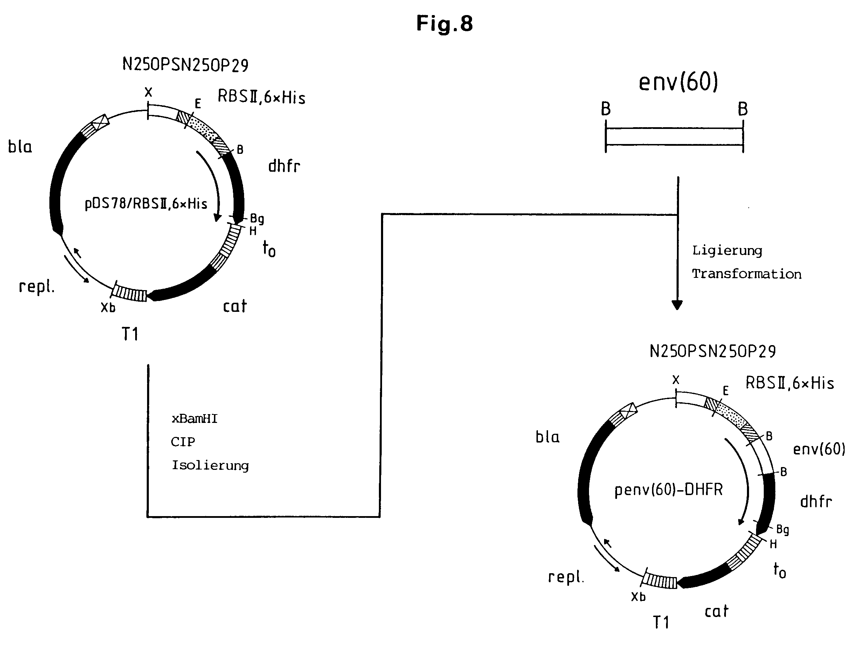

Zur Konstruktion des Plasmids penv(60)-DHFR wurde das mit dem Restriktionsenzym BamHI linearisierte Plasmid pDS78/RBSII,6xHis mit dem synthetisch hergestellten HIV-2 env(60)Gen, verknüpft (Abb. 8).

B) Präparation des mit BamHI linearisierten Plasmids pDS78/RBSII,6xHis (Fragment 1)

4 pmol des Plasmids pDS78/RBSII wurden mit dem Restriktionsenzym BamHI geschnitten. Anschliessend wurde die DNA mit CIP [calf intestinal phosphatase] behandelt. Die Enzyme wurden dann hitzeaktiviert und nach Zugabe von Probenpuffer wurde die DNA in einem 1% low melt Agarose Gel aufgetrennt. Die entsprechende DNA Bande wurde nach Anfärbung mit Ethidiumbromid unter UV-Licht (300 nm) ausgeschnitten und die DNA nach Standardmethode extrahiert [Maniatis et al., supra].

C) Präparation des HIV-2 env(60)-Gens (Fragment 2)

Die Herstellung dieses Gens ist in Beispiel 1 beschrieben.

D) Zusammensetzen des Plasmids penv(60)-DHFR

0,05 pmol des Fragments 1 und 0,1 pmol des Fragments 2 wurden mit 1E T4-Ligase inkubiert (15°C, 2h). Nach Hitzeinaktivierung des Enzyms wurde die DNA in den E. coli-Stamm M15, der das Plasmid pDMI,1 enthielt, transformiert. Die Zellen wurden auf LB-Agarplatten, enthaltend 100 µg/ml Ampicillin und 25 µg/ml Kanamycin, ausplattiert. Die Platten wurden für 15 Stunden bei 37°C inkubiert. Die Ligierung ergab etwa 500 Kolonien. Einzelne Kolonien wurden in je 10 ml LB Medium überführt, über Nacht bei 37°C wachsen gelassen und anschliessend wurde die Plasmid-DNA nach Standard-Methoden [Maniatis et al., supra] isoliert.

E) Sequenzanalyse des in das Plasmid pDS78/RBSII,6xHis integrierten HIV-2 env(60)-Gens

Um nachzuweisen, dass die richtige HIV-2 env(60)-Gensequenz in der richtigen Orientierung in der BamHI Stelle des Plasmids pDS78/RBSII,6xHis enthalten ist, wurde die doppelsträngige zirkuläre Plasmid-DNA sequenziert, wobei ein mit [$\gamma$-$^{32}$P]-ATP markierte Startersequenz (primer) benutzt wurde.

0,3 pmol der isolierten Plasmid-DNA wurden mit Alkohol gefällt, das Sediment einmal mit 80% Aethanol gewaschen, getrocknet und schliesslich in 8 µl 1/4 TE-Puffer gelöst. Nach Zugabe von 2 pmol der radioaktiv markierten Startersequenz wurde die Probe zunächst für 2 Minuten bei 95° C und für 5 Minuten bei 42° inkubiert. Danach wurde die DNA nach der Methode von Sanger et al. [Proc. Natl. Acad. Sci. USA 74, 5463-6567 (1977)] sequenziert. Die Sequenzanalyse ergab, dass die korrekte HIV-2 env(60)-Gensequenz in der BamHI Restriktionsstelle des Plasmids pDS78/RBSII,6xHis integriert worden war.

Beispiel 4

Konstruktion des Plasmids pDHFR-env(60)

A) Prinzipien

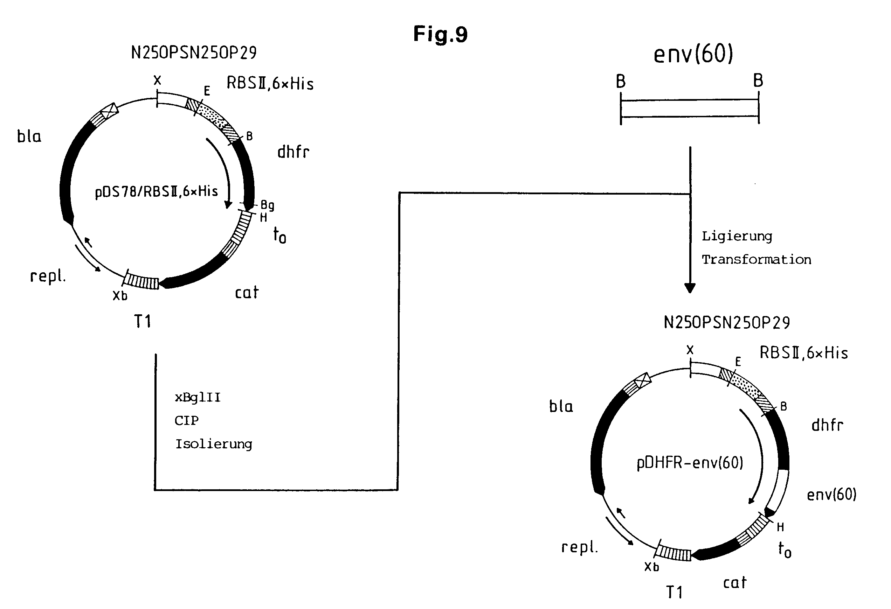

Zur Konstruktion des Plasmids pDHFR-env(60) wurden das mit dem Restriktionsenzym BglII linearisierte Plasmid pDS78/RBSII,6xHis mit dem synthetisch hergestellten HIV-2 env(60)-Gen verknüpft (Abb. 9).

B) Präparation des BglII linearisierten Plasmids pDS78/RBSII,6xHis (Fragment 1)

4 pmol des Plasmids pDS78/RBSII,6xHis wurden mit dem Restriktionsenzym BGIII geschnitten. Anschliessend wurde die DNA mit CIP [calf intestinal phosphatase] behandelt. Die Enzyme wurden hitzeinaktiviert und nach Zugabe von Probenpuffer wurde die DNA in einem 1% low melt Agarose Gel aufgetrennt. Die entsprechende DNA-Bande wurde nach Anfärbung mit Ethidiumbromid unter UV-Licht (300 nm) ausgeschnitten und die DNA nach Standardmethode extrahiert [Maniatis et al., supra].

C- Präparation des HIV-2 env(60)-Gens (Fragment 2)

Die Herstellung dieses Gens ist in Beispiel 1 beschrieben.

D) Zusammensetzen des Plasmids pDHFR-env(60)

0,05 pmol des Fragments 1 und 0,1 pmol des Fragments 2 wurden mit 1E T4-Ligase inkubiert (15°C, 2h). Nach Hitzeinaktivierung des Enzyms wurde die DNA in den E. coli-Stamm M15, der das Plasmid pDMI,1 enthielt, transformiert. Die Zellen wurden auf LB-Agarplatten ausplattiert, die 100 µg/ml Ampicillin und 25 µg/ml Kanamycin enthielten. Die Platten wurden für 15 Stunden bei 37°C inkubiert. Die Ligierung ergab etwa 500 Kolonien. Einzelne Kolonien wurden in je 10 ml LB Medium über bei 37°C wachsen gelassen und anschliessend wurde die Plasmid-DNA nach Standard-Methoden [Maniatis et al., supra] isoliert.

E) Sequenzanalyse des in das Plasmid pDS78/RBSII,6xHis integrierten HIV-2 env(60)-Gens

Um nachzuweisen, dass die richtige HIV-2 env(60)-Gensequenz in der richtigen Orientierung in der BglII Stelle des Plasmids pDS78/RBSII,6xHis enthalten ist, wurde die doppelsträngige zirkuläre Plasmid-DNA sequenziert, wobei ein mit [$\gamma$-$^{32}$P]-ATP markierte Startersequenz (primer) benutzt wurde.

0,3 pmol der isolierten Plasmid-DNA wurden mit Alkohol gefällt, das Sediment einmal mit 80% Aethanol gewaschen, getrocknet und schliesslich in 8 µl 1/4 TE-Puffer gelöst. Nach Zugabe von 2 pmol der radioaktiv markierten Startersequenz wurde die Probe zunächst für 2 Minuten bei 95° C und dann für 5 Minuten bei 42° inkubiert. Dann wurde die DNA nach der Methode von Sanger et al. [Proc. Natl. Acad. Sci. USA 74, 5463-6567 (1977)] sequenziert. Die Sequenzanalyse ergab, dass die korrekte HIV-2 env(60)-Gensequenz in der BglII Restriktionsstelle des Plasmids pDS78/RBSII,6xHis integriert worden war.

Beispiel 5

Reaktivität der ENV(60)-DHFR und DHFR-ENV(60) Polypeptide mit HIV positiven Seren.

A. Prinzipien

Um nachzuweisen, dass das env(60)-Gen eine antigene determinante Gruppe kodiert, die von Antikörpern in Seren von HIV-2 infizierten Personen erkannt wird, wurden die Polypeptide ENV(60)-DHFR und DHFR-ENV(60) in E. coli exprimiert, danach auf Nitrozellulosefilter transferiert und mit geeigneten Seren inkubiert. Das HIV-1 ENV(80)-DHFR Polypeptid [Certa et al., EMBO J. 5, 3051-3056 (1986)] diente als Kontrolle.

B. Expression der ENV(60)-DHFR und DHFR-ENV(60) Polypeptide in E. coli

E. coli M15 Zellen, enthaltend das Plasmid pDMI,1, wurden mit dem Plasmid penv(60)-DHFR bzw. pDHFR-env(60) transformiert und in LB-Medium [Maniatis et al., supra], enthaltend 100 µg/ml Ampicillin und 20 µg/ml Kanamycin, wachsen gelassen. Bei einer optischen Dichte von $OD_{600}$ = 0,7 wurden die Kulturen mit IPTG (2 mM Endkonz.) induziert und für weitere 4 Stunden wachsen gelassen. Danach wurden die Zellen mittels Zentrifugation geerntet.

C. Analyse der in E. coli exprimierten ENV(60)-DHFR und DHFR-ENV(60) Polypeptide

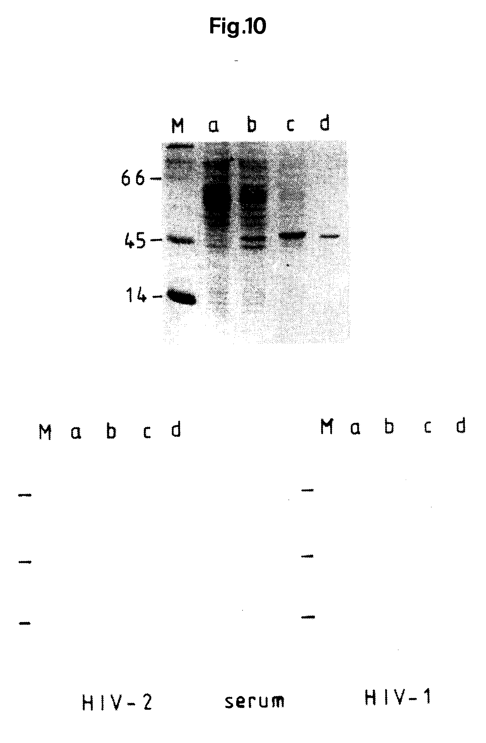

50 µl der Zellkulturen wurden in 125 mM Tris-HCl, pH 6,8, enthaltend 3% SDS, 3% β-Mercaptoethanol und 20% Glycerin resuspendiert. Die Proben wurden während 5 Minuten gekocht und anschliessend mittels 12,5% Polyacrylamidgelelektrophorese aufgetrennt [U.K. Laemmli, Nature 227, 680-685 (1970)]. Die Proteinbanden wurden mittels Coomassie-Blau-Färbung sichtbar gemacht (Fig. 10, oberer Teil).

D. Reaktivität der ENV(60)-DHFR und DHFR-ENV(60) Polypeptide mit HIV Antikörpern

Proben der induzierten Zellkulturen (siehe Abschnitt B), ein E. coli Kontroll-Lysat und gereinigtes HIV-1 ENV(80)-DHFR Polypeptid (1 µg pro Spur) wurden, wie in Abschnitt C beschrieben, elektrophoretisch aufgetrennt (2 Gele). Die ungefärbten Gele wurden jeweils mit einer Nitrocellulosemembran bedeckt. Die belegten Gele wurden dann mit jeweils 2 Blatt Filterpapier bedeckt und in eine Transfer-Kammer überführt. Diese wurde mit Transferpuffer (25 mM Tris, pH 8,3, enthaltend 192 mM Glycin und 20% Methanol) gefüllt und der Transfer der Polypeptide erfolgte während 12 Stunden bei 4°C, und 100 mA Stromstärke. Danach wurden die Nitrocellulosemembranen zuerst während 4 Stunden bei Raumtemperatur in PBS [Maniatis et al., supra], enthaltend 5% getrocknete Magermilch, inkubiert. Dann wurde die eine Nitrocellulosemembran in PBS, enthaltend 5% getrocknete Magermilch und 1:1000 verdünntes HIV-1 positives Serum, und die andere Membran in PBS, enthaltend 5% getrocknete Magermilch und 1:1000 verdünntes HIV-2 positives Serum, während 4 Stunden bei Raumtemperatur inkubiert. Danach wurden die Nitrocellulosemembranen zunächst 3x mit PBS, enthaltend 0,3% Tween-20, gewaschen und danach während 1 Stunde bei Raumtemperatur in PBS, enthaltend 0,3% Tween-20, 5% Ziegenserum und 1:2000 verdünntes Ziegen-Anti-Human-IgG-Peroxidase-Konjugat (Nordic), inkubiert. Nach drei Waschungen mit PBS wurden die Proteinbanden auf den Nitrozellulosemembranen, welche mit Antikörpern reagiert hatten, mittels Inkubation in 25 ml PBS enthaltend 5 µg 4-Chloro-1-naphtol und 5 µl 30% Wasserstoffperoxid, sichtbar gemacht. Die Reaktion wurde durch Zugabe von PBS gestoppt.

Wie in Figur 10 zu sehen ist, werden die ENV(60)-DHFR und DHFR-ENV(60) Polypeptide nur von Antikörpern im Serum von HIV-2 infizierten Patienten erkannt. Andererseits wird das ENV(80)-DHFR Polypeptid nur von Antikörpern im Serum von HIV-1 infizierten Patienten erkannt.

Beispiel 6

Konstruktion des Plasmids pRBSII-6xHis

A. Beschreibung des Plasmids pDS56/RBSII

Zur Konstruktion des Plasmids pRBSII-6xHis wurde das Plasmid pDS56/RBSII verwendet. Mit diesem Plasmid sowie dem Plasmid pDMI,1 transformierte E. coli Zellen wurden bei der Deutschen Sammlung von Mikroorganism in Braunschweig am 23. Dezember 1987 nach dem Budapester Vertrg hinterlegt [E. coli M15 (pDS56/BSII; pDMI,1), DSM-Nr.: 4330].

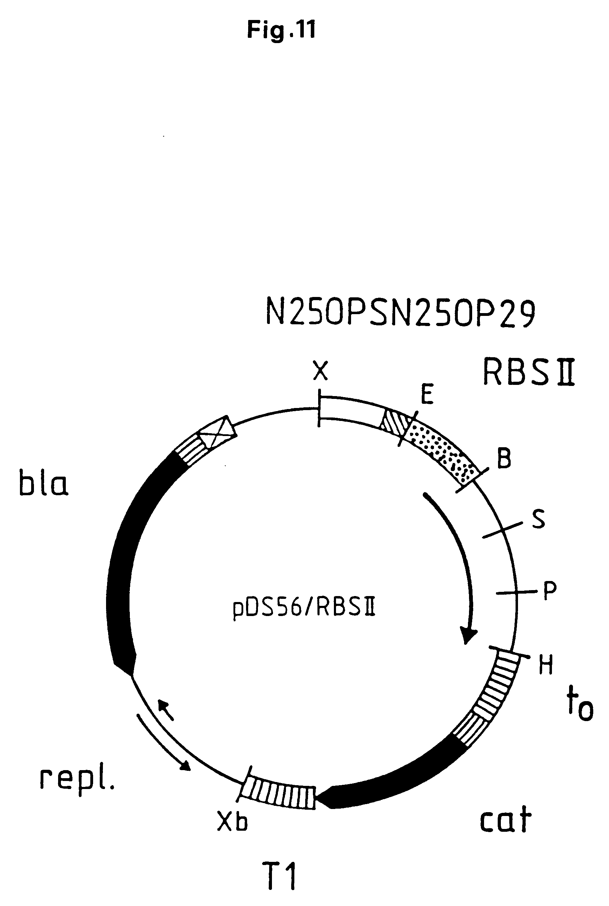

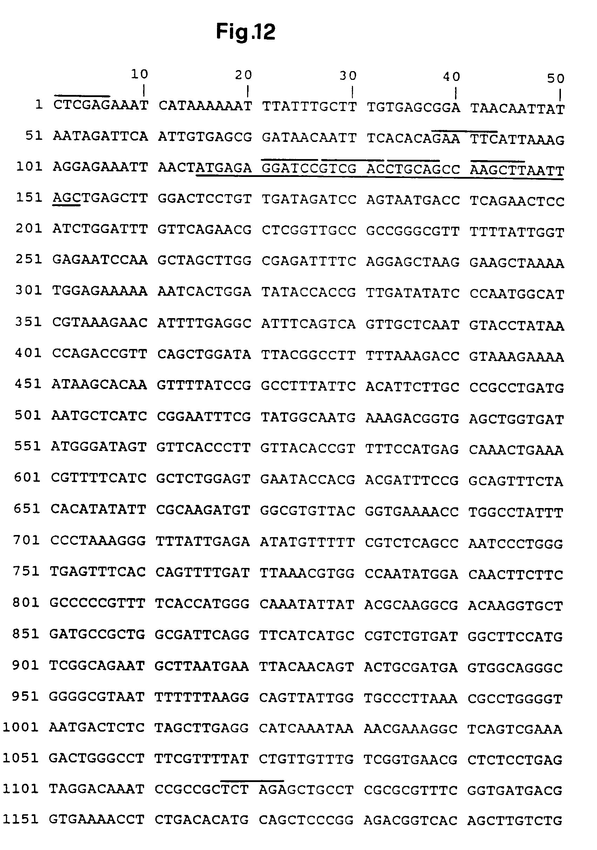

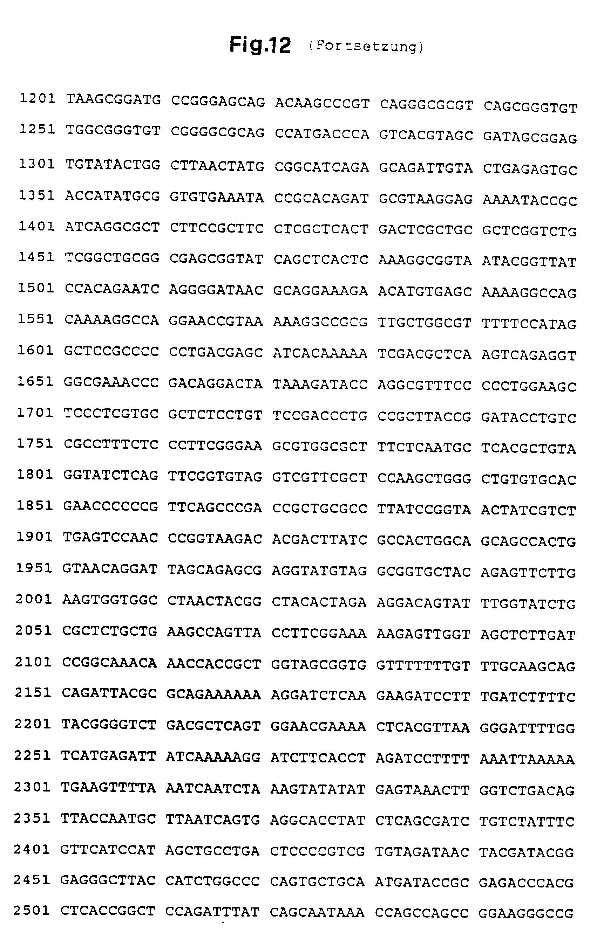

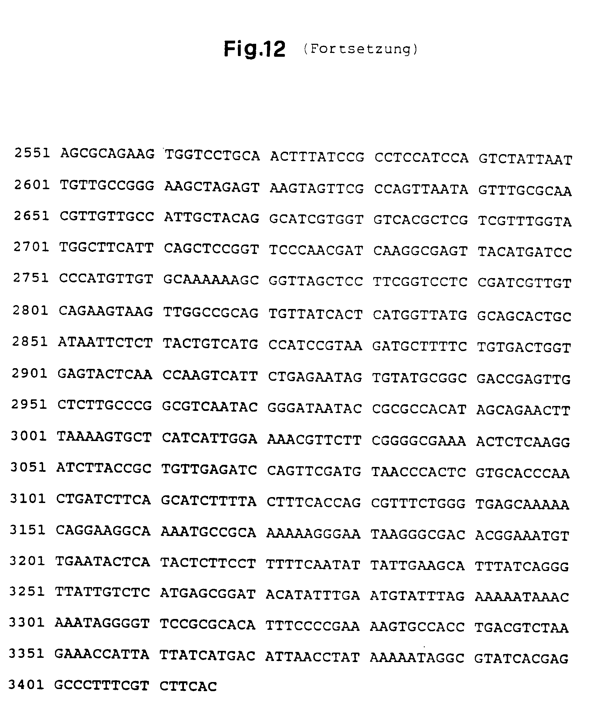

Der Anteil von pDS56/RBSII (Fig. 11 und 12), der zwischen den Schnittstellen für die Restriktionsenzyme XbaI und XhoI liegt und die Replikationsregion sowie das Gen für die $\beta$-Lactamase, das den Zellen Ampicillinresistenz verleiht, enthält, stammt ursprünglich aus dem Plasmid pRB322 (Bolivar et al., supra; Sutcliffe, supra). Jedoch ist das Gen für die $\beta$-Lactamase dahingegen modifiziert, dass die Schnittstellen für die Restriktionsenzyme HincII und PstI eliminiert sind. Diese Veränderung in der DNA-Sequenz wirken sich aber nicht auf die Aminosäuresequenz der $\beta$-Lactamase aus. Der verbleibende Teil des Plasmids trägt das regulierbare Promotor/Operator-Element N250PSN250P29 gefolgt von der Ribosomenbindungsstelle RBSII, die Teil eines EcoRI/BamHI-Fragments ist. Es folgen der Terminator $t_o$ des E. coli-Phyagen Lambda (Schwarz et al., supra), das Promotor-freie Gen der Chloramphenicolacetyltransferase (Marcoli et al., supra) und der Terminator T1 des E. coli rrnB Operons (Brosius et al., supra).

Auf Grund der hohen Effizienz der Expressionssignale N250PSN250P29 und RBSII kann das Plasmid pDS56/RBSII und seine Derivate, wie das Plasmid pRBSII-6xHis, auch nur dann stabil in E. coli-Zellen gehalten werden, wenn das Promotor/Operator Element durch das Binden eines lac-Repressors an den Operator reprimiert ist (siehe hierzu Beispiel 2, Abschnitt A).

B. Konstruktion des Plasmids pRBSII-6xHis

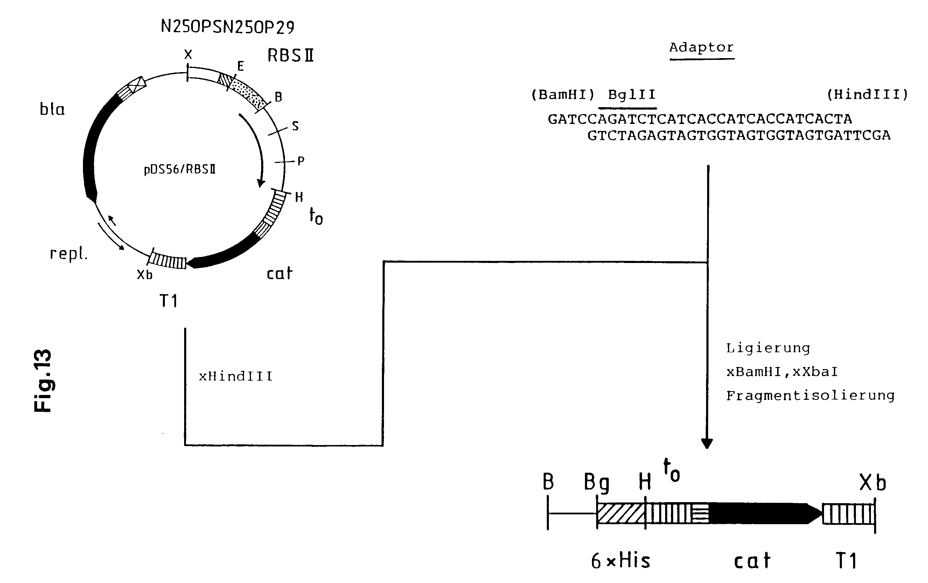

(1) 2 pmol des Plasmids pDS56/RBSII wurden mit dem Restriktionsenzym HindIII geschnitten. Nach Aufarbeitung der Probe wurden dem geschnittenen Plasmid 50 pmol eines phosphorylierten Adaptors (der für 6 Histidine kodiert) zugegeben und die Probe mit T4-DNA-Ligase, wie beschrieben, inkubiert. Nach Aufarbeitung des Ligierungsansatzes wurde die DNA mit den Restriktionsenzymen BamHI und XbaI geschnitten und das BamHI-XbaI-Fragment mit der für 6 Histidine kodierenden Region, dem Terminator $t_o$, dem cat-Gen und dem Terminator T1 mittels Agarose-Gelelektrophorese isoliert (Abb. 13).

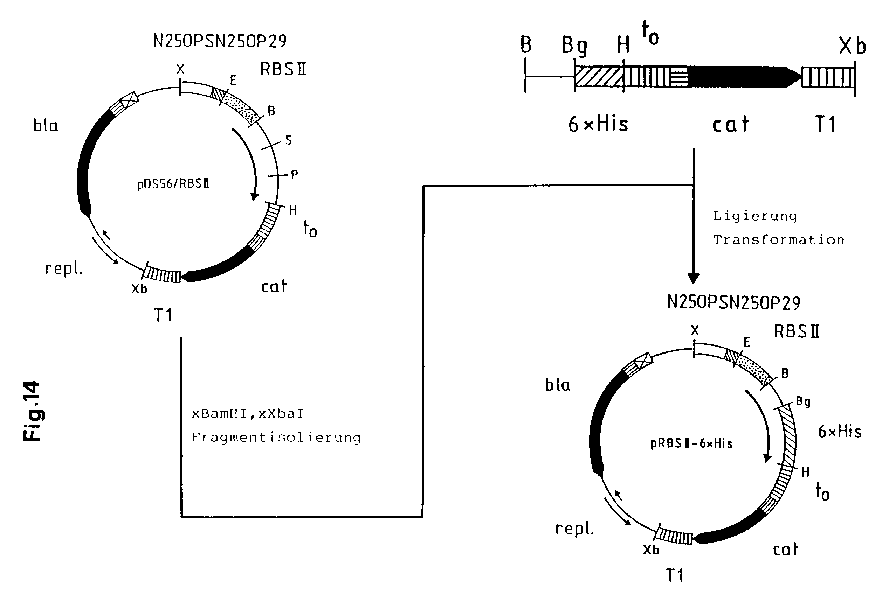

(2) 2 pmol des Plasmids pDS56/RBSII wurden mit den Restriktionsenzymen XbaI und BamHI geschnitten und das XbaI/BamHI-Fragment mit der Replikationsregion, dem bla-Gen dem Promotor N25OPSN25OP29 und der Ribosomenbindungsstelle RBSII mittels Agarose-Gelelektrophorese isoliert (Abb. 14).

(3) Je 0,1 pmol der isolierten Fragmente wurden, wie beschrieben (Beispiel 2), ligiert und anschliessend in den E. coli-Stamm M15 (pDMI,1) transformiert. Nach Plattierung und Inkubation (Beispiel 2.B) wurden einzelnen Kolonien in 10 ml Medium, wie beschrieben, aufgewachsen und die Plasmide nach der Methode von Birnboim und Doly (supra) isoliert. Eine Restriktionsanalyse mit den Enzymen BamHI und XbaI ergab, dass die Plasmide die 2 gewünschten Fragmente enthielten. Diese Plasmide erhielten die Bezeichnung pBSII-6xHis (Abb. 14).

Beispiel 7

Konstruktion des Plasmids pRBSII-env(80)-gag(419)-env(60)-6xHis

A) Prinzipien

Zur Konstruktion des Plasmids pRBSII-env(80)-gag(419)-env(60)-6xHis wurde das HIV-2 Gen env(60) sowie die HIV-1 Gene env(80) und gag(419) mit dem Expressionsvektor pRBSII-6xHis verknüpft.

B) Präparation des mit den Restriktionsenzymen BamHI und BglII geschnittenen Plasmids pRBSII-6xHis

2 pmol des Plasmids pRBSII-6xHis wurden mit je 10 Einheiten der Restriktionsenzyme BamHI und BglII geschnitten. Nach Zugabe von Probenpuffer wurde die DNA auf einem 1%-Agarosegel aufgetrennt. Die der Plasmid-DNA entsprechende Bande wurde nach Anfärbung mit Ethidiumbromid unter UV-Licht (300nm) ausgeschnitten und durch Elektroeluation gereinigt (Maniatis et al., supra). Die Enden dies gereinigten Fragmentes wurden dann mit Phosphatase dephosphoryliert. Anschliessend wurde die DNA mit Phenol: Chloroform (1:1) extrahiert, mit Ethanol gefällt und in 1/4 TE-Puffer gelöst.

C) Präparation des HIV-1 gag(419)-Gens

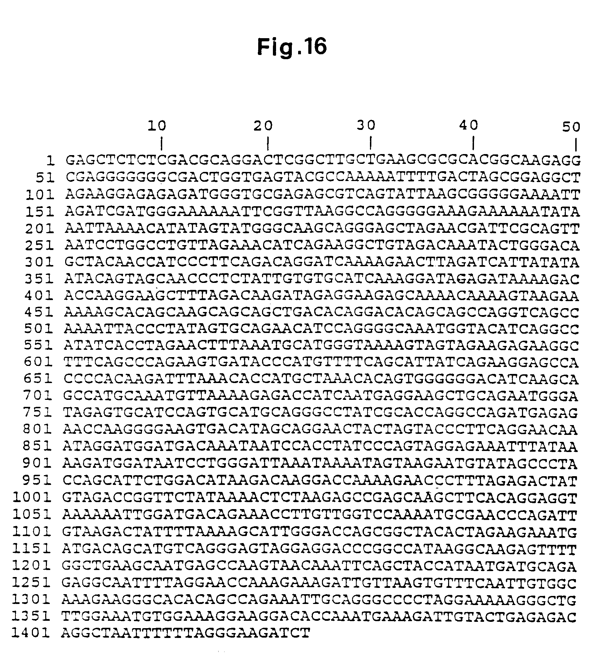

2 pmol des Plasmids pU-GAG, welches ein SstI/BglII Fragment des HIV-1 gag-Gens enthält (dessen Nukleotidsequenz in Figur 16 gezeigt ist), wurden mit 12 Einheiten des Restriktionsenzyms XmnI verdaut. Anschliessend wurde die DNA mit Phenol: Chloroform (1:1) extrahiert und mit 2 Volumen Ethanol bei -20°C ausgefällt. Das NDA-Pellet wurde in 50µl 50mM Tris-HCl, pH 7.6, enthaltend 10mM $MgCl_2$, 10mM DTT, 0,5mM ATP, 100pmol phosphorylierte 10er BamHI-linker (5′-CCGGATCCGG-3′), aufgenommen. Nach Zugabe von einer Einheit T4-DNA Ligase wurde über Nacht bei 14°C inkubiert. Dann wurde die Mischung während 10 Minuten bei 65°C inkubiert und mit Restriktionsenzympuffer auf ein Volumen von 100µl gebracht. 100 Einheiten BamHI und 10 Einheiten HindIII wurden zugegeben und die Mischung danach während 3 Stunden bei 37°C inkubiert. Anschliessend wurde die DNA mit Phenol und Chloroform extrahiert und mit Ethanol gefällt. Das DNA-Pellet wurde in Probenpuffer gelöst und auf einem 6%-Polyacrylamidgel aufgetrennt. Nach dem Anfärben mit Ethidiumbromid wurde eine Bande mit 250 Basenpaaren ausgeschnitten und durch Elektroeluation isoliert.

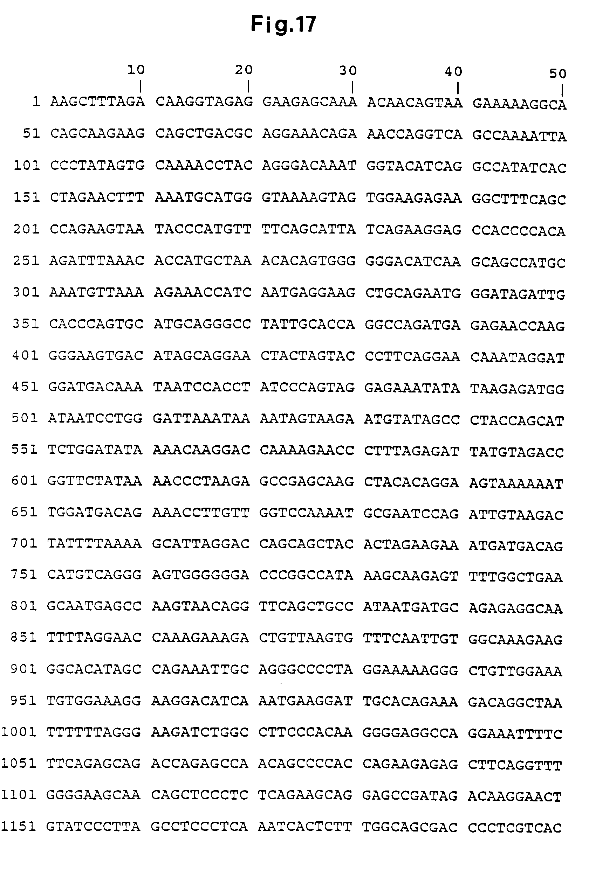

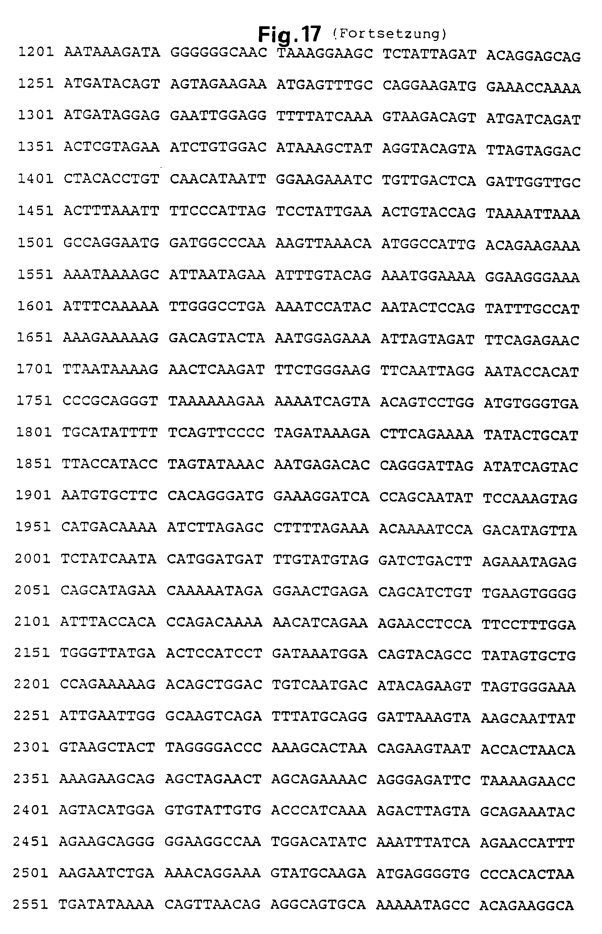

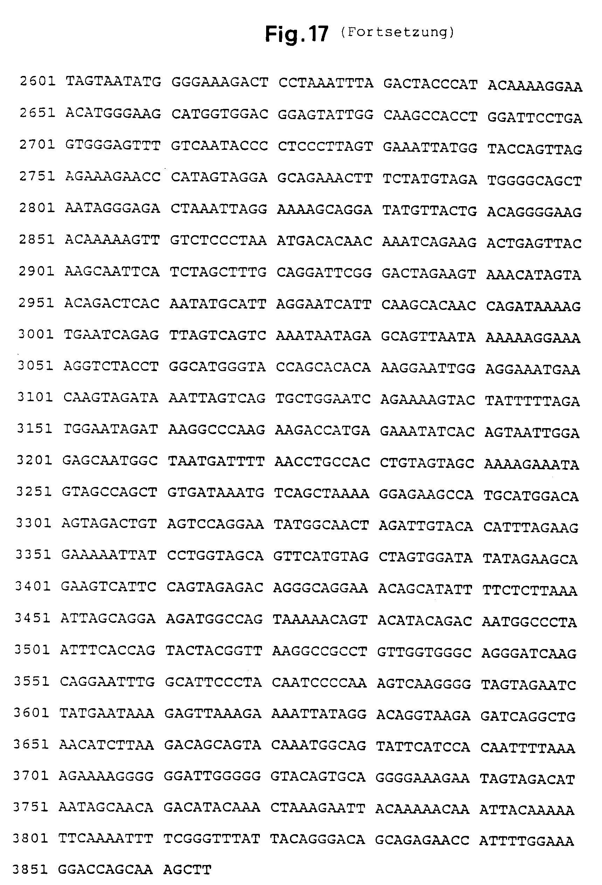

2 pmol des Plasmids p2-3U/HindIII10, welches ein HindIII Fragment des HIV-1 gag-Gens enthält (dessen Sequenz in Fig. 17 gezeigt ist), wurden mit 11 Einheiten BglII während einer Stunde bei 37°C verdaut, 10 Minuten auf 65°C erhitzt, auf Raumtemperatur abgekühlt und 45 Minuten mit dem Klenow Fragment der E.coli DNA polymerase inkubiert. Danach wurde die DNA mit Phenol: Chloroform (1:1) extrahiert, mit Ethanol gefällt, in 50µl 50 mM Tris-HCl, pH7.6, enthalend 10mM $MgCl_2$, 10mM DTT, 0.5mM ATP, 100 pmol phosphorylierter 12er BglII-linker (5′-GGAAGATCTTCC-3′) und 1 Einheit T4 DNA-Ligase, aufgenommen über Nacht bei 14°C inkubiert. Das Reaktionsgemisch wurde dann auf 65°C erhitzt und mit Restriktionsenzympuffer auf ein Volumen von 100µl gebracht. 100 Einheiten BglII wurden zugegeben und danach wurde für 3 Stunden bei 37°C inkubiert. Nach Zugabe von NaCl (50mM Endkonzentration) wurden 10 Einheiten HindIII zugegeben und es wurde 1 Stunde bei 37°C inkubiert. Nach der Extraktion mit Phenol: Chloroform (1:1) wurde die DNA mit Ethanol gefällt und anschliessend auf ein 1% Agarosegel aufgetragen. Ein Fragment mit 1020 Basenpaaren wurde ausgeschnitten und durch Elektroeluation isoliert und gereinigt.

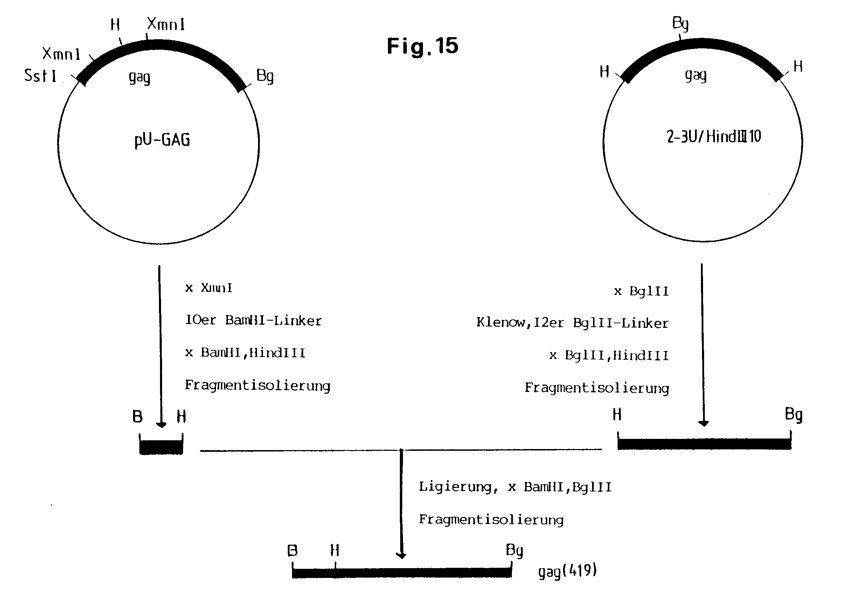

Je 1 pmol des BamHI-HindIII Fragments und des HindIII-BglII Fragments wurden mit 1 Einheit T4-DNA Ligase miteinander verknüpft. Nach Hitzeinaktivierung der Ligase wurden die ligierten Fragmente mit BamHI und BglII nachgeschnitten und auf ein 1% Agarosegel aufgetragen. Das dem HIV-1 gag(419)-Gen entsprechende Fragment wurde durch Elektroeluation isoliert und gereinigt. (Fig. 15)

D) Präparation des HIV-1 env(80)-Gens

Die Präparation des HIV-1 env(80)-Gens wurde, wie von Certa et al., [EMBOJ.5, 3051-3056 (1986)] beschrieben, hergestellt.

E) Präparation des HIV-2 env(60)-Gens

Die Herstellung des HIV-2 env(60)-Gens ist in Beispiel 1 beschrieben.

F) Konstruktion des Plasmids pRBSII-gag(419)-6xHis

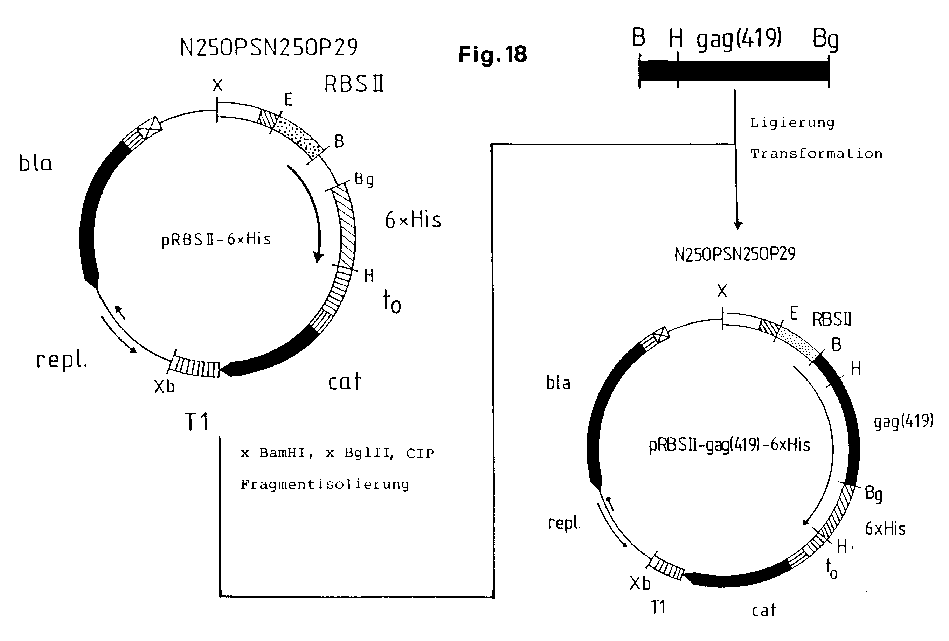

Zur Konstruktion des Plasmids pRBSII-gag(419)-6xHis wurden 0.1 pmol des mit BamHI und BglII linearisierten Vektors pRBSII-6xHis (siehe B) mit 0.3 pmol des gag(419) -Gens durch Inkubation mit 1 Einheit T4-DNA Ligase bei 14°C über Nacht verknüpft. Nach Hitzeinaktivierung des Enzyms wurde die DNA in den E.coli-Stamm W3110, der das Plasmid pDMI,1 enthielt, transformiert. Die Zellen wurden auf LB-Agarplatten ausplattiert, die 100µg/ml Ampicillin und 25µg/ml Kanamycin enthielten. Die Platten wurden über Nacht bei 37°C inkubiert. Einzelne Kolonien wurden über Nacht bei 37°C wachsen gelassen und anschliessend wurde die Plasmid-DNA nach Standardmethoden [Maniatis et al., supra] isoliert (Fig. 18).

G) Konstruktion des Plasmids pRBSII-env(80)-gag(419)-6xHis

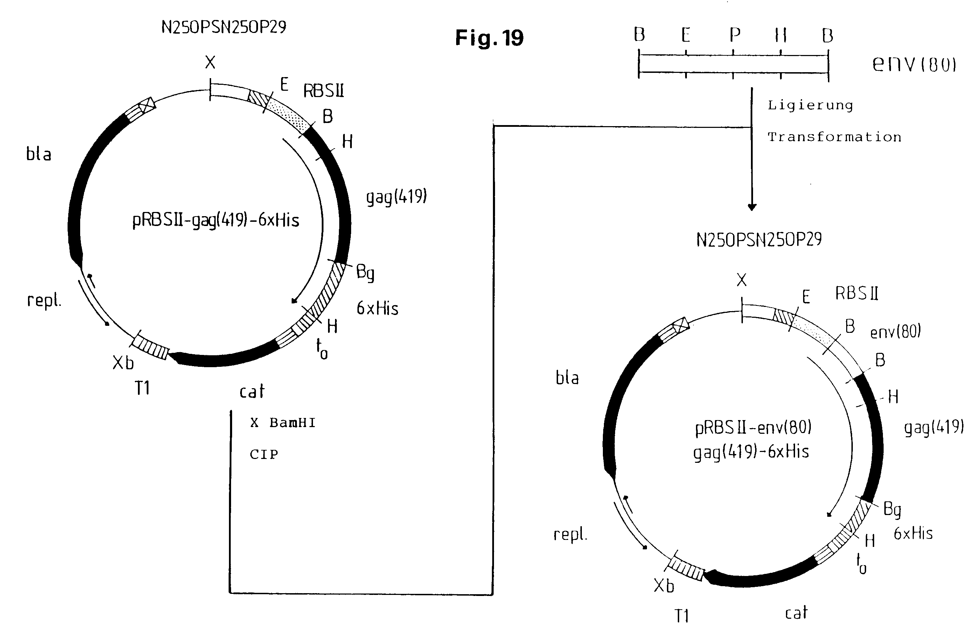

Zur Konstruktion des Plasmids pRBSII-env(80)-gag(419)-6xHis wurden 0.1 pmol des Plasmids pRBSII-gag(419)-6xHis mit BamHI geschnitten unt mit CIP behandelt. Die DNA wurde mit Phenol: Chloroform (1:1) extrahiert und mit 2 Volumen Ethanol gefällt. Die mit BamHI linearisierte pRBSII-gag(419)-6xHis Plasmid-DNA wurde mit 0.3 pmol des HIV-1 env(80)-Gens und 1 Einheit T4-DNA Ligase über Nacht bei 14°C inkubiert. Nach Hitzeinaktivierung des Enzyms wurde die DNA in den E. coli-Stamm W3110, der das Plasmid pDMI,1 enthielt, transformiert. Die Zellen wurden auf LB-Agarplatten ausplattiert, die 100µg/ml Ampicillin und 25µg/ml Kanamycin enthielten. Die Platten wurden über Nacht bei 37°C inkubiert. Einzelne Kolonien wurden über Nacht bei 37°C wachsen gelassen und anschliessend wurde die Plasmid-DNA nach Standardmethoden [Maniatis et al., supra] isoliert (Fig. 19).

H) Konstruktion des Plasmids pRBSII-env(80)-gag(419)-env(60)-6xHis

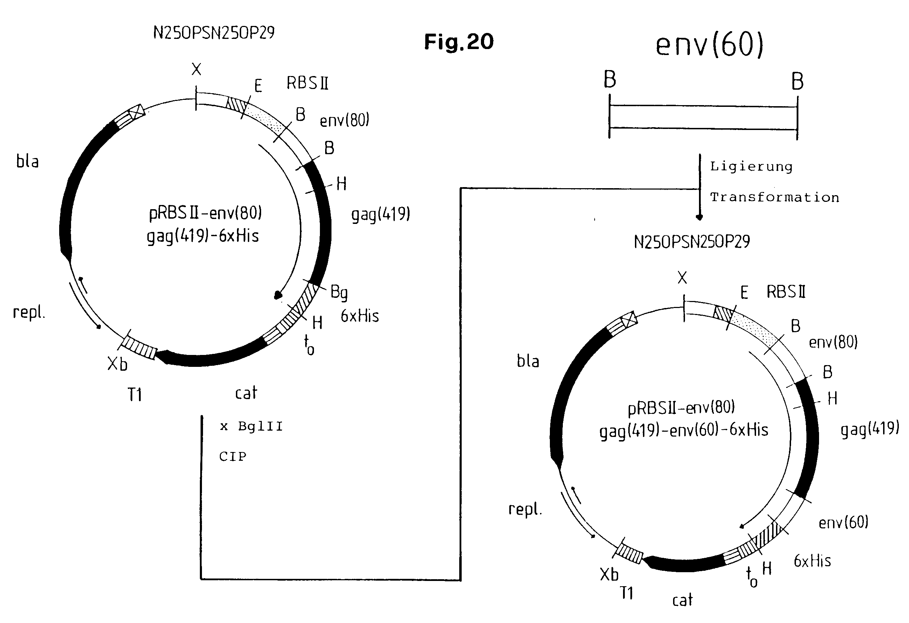

Zur Konstruktion des Plasmids pRBSII-env(80)-gag(419)-env(60)-6xHis wurden 0.1pmol des Plasmids pRBSII-env(80)-gag(419)-6xHis BglII geschnitten und mit CIP behandelt. Anschliessend wurde die DNA mit Phenol: Chloroform (1:1) extrahiert und mit 2 Volumen Ethanol gefällt. Die mit BglII linearisierte pRBSII-env-(80)-gag(419)-6xHis Plasmid-DNA wurde dann mit 0.3 pmol des HIV-2 env(60)-Gens und 1 Einheit T4-DNA Ligase über Nacht bei 14°C inkubiert. Nach Hitzeinaktivierung des Enzyms wurde die DNA in den E. coli-Stamm W3110, der das Plasmid pDMI,1 enthielt, transformiert. Die Zellen wurden auf LB-Agarplatten ausplattiert, die 100µg/ml Ampicillin und 25µg/ml Kanamycin enthielten. Die Platten wurden über Nacht bei 37°C inkubiert. Einzelne Kolonien wurden über Nacht bei 37°C wachsen gelassen und anschliessend wurde die Plasmid-DNA nach Standardmethoden [Maniatis et al., supra] isoliert (Fig. 20).

## Beispiel 8

### Reaktivität des ENV(80)-GAG(419)-ENV(60)-Polypeptids mit HIV positiven Seren

### A. Prinzipien

Um nachzuweisen, dass das ENV(80)-GAG(419)-ENV(60)-Polypeptid mit Seren von HIV-1 und HIV-2 infizierten Personen reagiert, wurde das ENV(80)-GAG(418)-ENV(60)-Polypeptid in E. coli exprimiert, danach gereinigt und mit geeigneten Seren im Enzymimmunoassay (EIA) getestet.

### B. Expression des ENV(80)-GAG(419)-ENV(60)-Polypeptids in E. coli

E. coli W3110 Zellen, enthaltend das Plasmid pDMI,1, wurden mit dem Plasmid pRBSII-env(80)-gag-(419)-env(60)-6xHis transformiert und in LB-Medium [Maniatis et al., supra], enthaltend 100µg/ml Ampicillin und 25µg/ml Kanamycin, wachsen gelassen. Bei einer optischen Dichte von $OD_{600}$ = 1,0 wurde die Kultur mit IPTG (2 mM Endkonz.) induziert und für weitere 2 Stunden wachsen gelassen. Danach wurden die Zellen mittels Zentrifugation geerntet.

### C. Reinigung des in E. coli exprimierten ENV(80)-GAG(419)-ENV(60)-Polypeptids

Die geernteten Zellen wurden in PBS-Puffer (0,2 g/l KCl, 8,0 g/l NaCl, 0,2 g/l $KH_2PO_4$, 1,144 g/l $Na_2HPO_4$, pH 7,0) resuspendiert und mittels Hochdruckhomogenisator aufgebrochen. Das erhaltene Zellhomogenisat wurde zentrifugiert und das Pellet wurde zweimal mit 0,1M Natriumphosphatpuffer, pH 8,0, enthaltend 6M Guanidin•HCl, extrahiert.

Nichtgelöstes Material wurde mittels Zentrifugation entfernt und falls notwendig filtriert. Die geklärte Lösung wurde auf eine NTA-Säule (deren Struktur und Herstellung in der europäischen Patentanmeldung, Publikations-Nr. 253 303 beschrieben ist) aufgetragen. Die Säule wurde anschliessend zuerst mit 0,1M Natriumphosphatpuffer, pH 8,0 enthaltend 6M Guanidin•HCl, und dann mit 0,1M Natriumphosphatpuffer, pH 6,5, enthaltend 8M Harnstoff, gewaschen. Die Eluation des ENV(80)-GAG(419)-ENV(60)-Polypeptids erfolgte mit 0,1M Natriumphosphatpuffer, pH 4,0, enthaltend 8M Harnstoff.

Das mittels NTA-Säule erhaltene ENV(80)-GAG(419)-ENV(60)-Eluat wurde anschliessend zweimal an einer Sephacryl S-200-Säule (Pharmacia, Laufpuffer: 50mM Tris•HCl, pH 7,0, enthaltend 5mM EDTA und 1% bzw. 0,1% SDS) chromatographiert. Mittels SDS-Polyacrylamidgelelektrophorese und Aminosäureanalyse konnte eine Reinheit von 89% des erhaltenen ENV(80)-GAG(419)-ENV(60)-Polypeptids nachgewiesen werden.

D. Reaktivität des gereinigten ENV(80)-GAG(419)-ENV(60)-Polypeptids mit HIV positiven Seren

Das gereinigte ENV(80)-GAG(419)-ENV(60)-Polypeptid wurde, wie in der europäischen Patentanmeldung, Publikations-Nr. 270 114 beschrieben, an Polystyrolkugeln gebunden auf seine Reaktivität mit HIV-1 und HIV-2 positiven Seren im Enzymimmunoassay (EIA) getestet. Die mit gereinigtem ENV(80)-GAG(419)-ENV(60)-Polypeptid und gereinigtem DHFR-ENV(60)-Polypeptid (positive HIV-2 Kontrolle) erhaltenen EIA-Werte ergaben folgendes Bild:

| Seren | DHFR-ENV(60) HIV-2 | ENV(80)-GAG(419)-ENV(60) HIV-1/HIV-2 |
|---|---|---|
| HIV-1 positive Seren[1)] | | |
| Mil 3 | 0,132 | 1,684 |
| Mil 22 | 0,104 | 2,094 |
| Mil 24 | 0,140 | 2,244 |
| Mil 26 | 0,062 | 2,198 |
| Mil 30 | 0,059 | 2,345 |
| HIV-2 positive Seren[1)] | | |
| S | 1,271 | 2,072 |
| K | 0,910 | 2,052 |
| D | 1,559 | 2,252 |
| B | 1,232 | 2,104 |
| Negative Seren | | |
| N1 | 0,096 | 0,092 |
| N2 | 0,047 | 1,110 |
| N3 | 0,063 | 0,076 |
| N4 | 0,056 | 0,087 |
| N5 | 0,067 | 0,088 |

1) durch "Western blots" (WB) bestätigt.

Wie zu sehen ist, werden vom ENV(80-GAG(419)-ENV(60)-Polypeptid alle positiven HIV-1 und HIV-2 Seren erkannt.

## Patentansprüche

1. Ein Polypeptid mit der Aminosäuresequenz

SerAlaArgLeuAsnSerTrpGlyCysAlaPheArgGlnValCysHisThrThr
ValProTrpValAsnAspSerLeuAlaProAspTrpAspAsnMetThrTrpGln
GluTrpGluLysGlnValArgTyrLeuGluAlaAsnIleSerLysSerLeuGlu
GlnAlaGlnGly (ENV(60)) (I)

oder Fragmente oder funktionelle Aequivalente davon, kovalent verknüpft mit einem Affinitätspeptid und einem Polypeptid, dessen Aminosäuresequenz mit mindestens einer antigenen und/oder immunogenen determinanten Gruppe des HIV-1 Hüllproteins (env) und/oder des HIV-1 Strukturproteins (gag) übereinstimmt.

2. Ein Polypeptid gemäss Anspruch 1, mit der folgenden Formel:

MetArgGlySerGluAlaGlnGlnHisLeuLeuGlnLeuThrValTrpGlyIleLysGln
LeuGlnAlaArgIleLeuAlaValGluArgTyrLeuLysAspGlnGlnLeuLeuGlyIle
TrpGlyCysSerGlyLysLeuIleCysThrThrAlaValProTrpAsnAlaSerTrpSer
AsnLysLeuLeuGluGlnIleTrpAsnAsnMetThrTrpMetGluTrpAspArgGluIle
AsnAsnTyrThrGlySerGlyIleArgLeuArgProGlyGlyLysLysLysTyrLysLeu
LysHisIleValTrpAlaSerArgGluLeuGluArgPheAlaValAsnProGlyLeuLeu
GluThrSerGluGlyCysArgGlnIleLeuGlyGlnLeuGlnProSerLeuGlnThrGly
SerLysGluLeuArgSerLeuTyrAsnThrValAlaThrLeuTyrCysValHisGlnArg

IleGluIleLysAspThrLysGluAlaLeuAspLysValGluGluGluGlnAsnAsnSer
LysLysLysAlaGlnGlnGluAlaAlaAspAlaGlyAsnArgAsnGlnValSerGlnAsn
TyrProIleValGlnAsnLeuGlnGlyGlnMetValHisGlnAlaIleSerProArgThr
LeuAsnAlaTrpValLysValValGluGluLysAlaPheSerProGluValIleProMet
PheSerAlaLeuSerGluGlyAlaThrProGlnAspLeuAsnThrMetLeuAsnThrVal
GlyGlyHisGlnAlaAlaMetGlnMetLeuLysGluThrIleAsnGluGluAlaAlaGlu
TrpAspArgLeuHisProValHisAlaGlyProIleAlaProGlyGlnMetArgGluPro
ArgGlySerAspIleAlaGlyThrThrSerThrLeuGlnGluGlnIleGlyTrpMetThr
AsnAsnProProIleProValGlyGluIleTyrLysArgTrpIleIleLeuGlyLeuAsn
LysIleValArgMetTyrSerProThrSerIleLeuAspIleLysGlnGlyProLysGlu
ProPheArgAspTyrValAspArgPheTyrLysThrLeuArgAlaGluGlnAlaThrGln
GluValLysAsnTrpMetThrGluThrLeuLeuValGlnAsnAlaAsnProAspCysLys
ThrIleLeuLysAlaLeuGlyProAlaAlaThrLeuGluGluMetMetThrAlaCysGln
GlyValGlyGlyProGlyHisLysAlaArgValLeuAlaGluAlaMetSerGlnValThr
GlySerAlaAlaIleMetMetGlnArgGlyAsnPheArgAsnGlnArgLysThrValLys
CysPheAsnCysGlyLysGluGlyHisIleAlaArgAsnCysArgAlaProArgLysLys
GlyCysTrpLysCysGlyLysGluGlyHisGlnMetLysAspCysThrGluArgGlnAla
AsnPheLeuGlyLysIleGlyArgSerAlaArgLeuAsnSerTrpGlyCysAlaPheArg
GlnValCysHisThrThrValProTrpValAsnAspSerLeuAlaProAspTrpAspAsn
MetThrTrpGlnGluTrpGluLysGlnValArgTyrLeuGluAlaAsnIleSerLysSer
LeuGluGlnAlaGlnGlySerHisHisHisHisHisHis

(ENV(80)-GAG(419)-ENV(60)) (IV)

3. Ein Polypeptid gemäss Anspruch 1 oder 2 mit einem zusätzlichen Methioninrest am Aminoende.

4. Ein Polypeptid gemäss einem der Ansprüche 1-3, welches in Bakterien hergestellt wurde.

5. Ein Polypeptid gemäss einem der Ansprüche 1-3, welches in E. coli hergestellt wurde.

6. Eine DNA-Sequenz, welche ein Polypeptid gemäss einem der Ansprüche 1-3 kodiert.

7. Eine DNA-Sequenz gemäss Anspruch 6, welche die Nukleotidsequenz

TCCGCTCGTCTGAACTCCTGGGGTTGCGCTTTTCGTCAGGTTTGCCACACTACGGTACCGTG
GGTAAACGACAGCTTAGCTCCGGACTGGGATAACATGACTTGGCAGGAATGGGAAAAACAGG
TGCGCTACCTGGAGGCTAACATTTCTAAATCTCTGGAACAGGCTCAGGGA

oder Teilsequenzen davon oder funktionelle Aequivalente dieser Nukleotidsequenz oder Teilsequenzen einschliesst.

8. Ein Expressionsvektor, worin eine DNA-Sequenz gemäss Anspruch 6 oder 7 operativ an eine Expressionskontrollsequenz gebunden ist.

9. Ein Expressionsvektor gemäss Anspruchs 8, der in E. coli replizieren kann.

10. Ein Expressionsvektor gemäss Anspruch 9, der das Plasmid pRBSII-env(80)-gag(419)-env(60)-6xHis ist.

11. Ein mit einem Expressionsvektor gemäss einem der Ansprüche 8-10 transformiertes Bakterium.

12. Ein mit einem Expressionsvektor gemäss einem dem Ansprüche 8-10 transformierter E. coli Stamm.

13. Ein mit einem Expressionsvektor gemäss einem der Ansprüche 8-13 transformierter E. coli M15 Stamm.

14. Ein Polypeptid gemäss einem der Ansprüche 1-5 als Antigen.

15. Verfahren zur Herstellung eines Polypeptids gemäss einem der Ansprüche 1-5, dadurch gekennzeichnet, dass man ein Bakterium mit einem Expressionsvektor gemäss einem der Ansprüche 8-10 transformiert, dann unter geeigneten Wachstumsbedingungen kultiviert und das besagte Polypeptid isoliert und, falls gewünscht, reinigt.

16. Verfahren zur Herstellung von HIV-Antikörpern, dadurch gekennzeichnet, dass man einem Säuger oder Vogel eine ausreichende Menge eines Polypeptids gemäss einem der Ansprüche 1-5 injiziert und die gebildeten Antikörper aus dem Serum dieser Tiere isoliert.

17. Verfahren zum Nachweis von Antikörpern gegen HIV-Viren in menschlichen Seren, dadurch gekennzeichnet, dass man
(a) ein Polypeptid gemäss einem der Ansprüche 1-5 markiert;
(b) dieses markierte Polypeptid mit einer menschlichen Serumprobe reagieren lässt; und
(c) die in der Reaktionsmischung entstandenen markierten Polypeptid/Antikörper-Komplexe nachweist.

18. Verfahren zum Nachweis von Antikörpern gegen HIV-Viren in menschlichen Seren, dadurch gekennzeichnet, dass man
(a) ein Polypeptid gemäss einem der Ansprüche 1-5 an festem Trägermaterial immobilisiert;
(b) eine menschliche Serumprobe mit diesem immobilisierten Protein in Kontakt bringt;
(c) nicht-gebundene Polypeptide und Antikörper durch Waschung entfernt; und
(d) die gewaschenen immobilisierten Polypeptid/Antikörper-Komplexe durch Zugabe von markiertem Staphylococcus aureus Protein A oder durch Zugabe von markierten menschlichen anti-Ig-Antikörpern nachweist.

**19.** Verfahren zum Nachweis von HIV-Viren oder Fragmenten davon in menschlichen Seren oder anderen biologischen Flüssigkeiten, dadurch gekennzeichnet, dass man
(a) eine menschliche Serumprobe oder eine andere Körperflüssigkeitsprobe mit einer bekannten Menge von Antikörpern gegen ein Polypeptid gemäss einem der Ansprüche 1-5 reagieren lässt; und
(b) die in der Reaktionsmischung entstandenen Antigen/Antikörper-Komplexe nachweist.

**20.** Verfahren gemäss Anspruch 19, bei dem die Antikörper mit einem Enzym markiert sind und die in der Reaktionsmischung entstandenen Antigen/Antikörper-Komplexe mittels Enzymimmunoassay nachgewiesen werden.

**21.** Antikörper gegen ein Polypeptid gemäss einem der Ansprüche 1-5.

**22.** Antikörper gemäss Anspruch 21, die monoklonale Antikörper sind.

**23.** Die Verwendung eines Polypeptids gemäss einem der Ansprüche 1-5 zur Herstellung von HIV-Antikörpern.

**24.** Die Verwendung eines Polypeptids gemäss einem der Ansprüche 1-5 zum Nachweis von HIV-Antikörpern in menschlichen Seren oder anderen biologischen Flüssigkeiten.

**25.** Die Verwendung eines Polypeptids gemäss einem der Ansprüche 1-5 zum Nachweis von HIV-Viren oder Fragmenten davon in menschlichen Seren oder anderen biologischen Flüssigkeiten.

**26.** Test-Kit zur Bestimmung von HIV-Viren oder Fragmenten davon bestehend aus einem Gefäss, welches HIV-Antikörper gegen ein Polypeptid gemäss einem der Ansprüche 1-5 enthält

**27.** Test-Kit zur Bestimmung von HIV-Antikörpern bestehend aus einem Gefäss, welches ein Polypeptid gemäss einem der Ansprüche 1-5 enthält.

**Claims**

**1.** A polypeptide having the amino acid sequence

SerAlaArgLeuAsnSerTrpGlyCysAlaPheArgGlnValCysHisThrThr
ValProTrpValAsnAspSerLeuAlaProAspTrpAspAsnMetThrTrpGln
GluTrpGluLysGlnValArgTyrLeuGluAlaAsnIleSerLysSerLeuGluGlnAla
GlnGly (ENV(60))

or fragments or functional equivalents thereof, covalently linked with an affinity peptide and a polypeptide whose amino acid sequence corresponds with at least one antigenic and/or immunogenic determinant of the HIV-1 envelope protein (env) and/or of the HIV-1 core protein (gag).

**2.** A polypeptide in accordance with claim 1 having the following formula

MetArgGlySerGluAlaGlnGlnHisLeuLeuGlnLeuThrValTrpGlyIleLysGln
LeuGlnAlaArgIleLeuAlaValGluArgTyrLeuLysAspGlnGlnLeuLeuGlyIle
TrpGlyCysSerGlyLysLeuIleCysThrThrAlaValProTrpAsnAlaSerTrpSer
AsnLysLeuLeuGluGlnIleTrpAsnAsnMetThrTrpMetGluTrpAspArgGluIle
AsnAsnTyrThrGlySerGlyIleArgLeuArgProGlyGlyLysLysLysTyrLysLeu
LysHisIleValTrpAlaSerArgGluLeuGluArgPheAlaValAsnProGlyLeuLeu
GluThrSerGluGlyCysArgGlnIleLeuGlyGlnLeuGlnProSerLeuGlnThrGly
SerLysGluLeuArgSerLeuTyrAsnThrValAlaThrLeuTyrCysValHisGlnArg
IleGluIleLysAspThrLysGluAlaLeuAspLysValGluGluGluGlnAsnAsnSer
LysLysLysAlaGlnGlnGluAlaAlaAspAlaGlyAsnArgAsnGlnValSerGlnAsn
TyrProIleValGlnAsnLeuGlnGlyGlnMetValHisGlnAlaIleSerProArgThr
LeuAsnAlaTrpValLysValValGluGluLysAlaPheSerProGluValIleProMet
PheSerAlaLeuSerGluGlyAlaThrProGlnAspLeuAsnThrMetLeuAsnThrVal
GlyGlyHisGlnAlaAlaMetGlnMetLeuLysGluThrIleAsnGluGluAlaAlaGlu
TrpAspArgLeuHisProValHisAlaGlyProIleAlaProGlyGlnMetArgGluPro
ArgGlySerAspIleAlaGlyThrThrSerThrLeuGlnGluGlnIleGlyTrpMetThr
AsnAsnProProIleProValGlyGluIleTyrLysArgTrpIleIleLeuGlyLeuAsn
LysIleValArgMetTyrSerProThrSerIleLeuAspIleLysGlnGlyProLysGlu
ProPheArgAspTyrValAspArgPheTyrLysThrLeuArgAlaGluGlnAlaThrGln

GluValLysAsnTrpMetThrGluThrLeuLeuValGlnAsnAlaAsnProAspCysLys
ThrIleLeuLysAlaLeuGlyProAlaAlaThrLeuGluGluMetMetThrAlaCysGln
GlyValGlyGlyProGlyHisLysAlaArgValLeuAlaGluAlaMetSerGlnValThr
GlySerAlaAlaIleMetMetGlnArgGlyAsnPheArgAsnGlnArgLysThrValLys
CysPheAsnCysGlyLysGluGlyHisIleAlaArgAsnCysArgAlaProArgLysLys
GlyCysTrpLysCysGlyLysGluGlyHisGlnMetLysAspCysThrGluArgGlnAla
AsnPheLeuGlyLysIleGlyArgSerAlaArgLeuAsnSerTrpGlyCysAlaPheArg
GlnValCysHisThrThrValProTrpValAsnAspSerLeuAlaProAspTrpAspAsn
MetThrTrpGlnGluTrpGluLysGlnValArgTyrLeuGluAlaAsnIleSerLysSer
LeuGluGlnAlaGlnGlySerHisHisHisHisHisHis

(ENV(80)-GAG(419)-ENV(60)) (IV).

**3.** A polypeptide in accordance with claim 1 or 2 having an additional methionine residue at the amino end.

**4.** A polypeptide in accordance with any one of claims 1-3, which has been produced in bacteria.

**5.** A polypeptide in accordance with any one of claims 1-3, which has been produced in E. coli.

**6.** A DNA sequence which codes for a polypeptide in accordance with any one of claims 1-3.

**7.** A DNA sequence in accordance with claim 6, which includes the nucleotide sequence

TCCGCTCGTCTGAACTCCTGGGGTTGCGCTTTTCGTCAGGTTTGCCACACTACGGTACCG
TGGGTAAACGACAGCTTAGCTCCGGACTGGGATAACATGACTTGGCAGGAATGGGAAAAA
CAGGTGCGCTACCTGGAGGCTAACATTTCTAAATCTCTGGAACAGGCTCAGGGA

or partial sequences thereof or functional equivalents of this nucleotide sequence or partial sequences.

**8.** An expression vector in which a DNA sequence in accordance with claim 6 or 7 is operatively bonded to an expression control sequence.

**9.** An expression vector in accordance with claim 8, which can replicate in E. coli.

**10.** An expression vector in accordance with claim 9, which is the plasmid pRBSII-env(80)-gag(419)-env-(60)-6xHis.

**11.** A bacterium transformed with an expression vector in accordance with any one of claims 8-10.

**12.** An E. coli strain transformed with an expression vector in accordance with any one of claims 8-10.

**13.** An E. coli M15 strain transformed with an expression vector in acordance with any one of claims 8-13.

**14.** A polypeptide in accordance with any one of claims 1-5 as an antigen.

**15.** A process for the production of a polypeptide in accordance with any one of claims 1-5, characterized by transforming a bacterium with an expression vector in accordance with any one of claims 8-10, then cultivating under suitable growth conditions and isolating and, where desired, purifying the said polypeptide.

**16.** A process for the production of HIV antibodies, characterized by injecting a sufficient amount of a polypeptide in accordance with any one of claims 1-5 into a mammal or bird and isolating the antibodies formed from the serum of this animal.

**17.** A method for the detection of antibodies against HIV viruses in human sera, characterized by
(a) labelling a polypeptide in accordance with any one of claims 1-5;
(b) reacting this labelled polypeptide with a human serum sample; and
(c) detecting the labelled polypeptide/antibody complexes resulting in the reaction mixture.

**18.** A method for the detection of antibodies against HIV viruses in human sera, characterized by
(a) immobilizing a polypeptide in accordance with any one of claims 1-5 on a solid carrier material;
(b) bringing a human serum sample into contact with this immobilized protein;
(c) removing non-bound polypeptides and antibodies by washing; and
(d) detecting the washed immobilized polypeptide/antibody complexes by adding labelled Staphylococcus aureus Protein A or by adding labelled human anti-Ig antibodies.

**19.** A method for the detection of HIV viruses or fragments thereof in human sera or in other biological fluids, characterized by
(a) reacting a human serum sample or another body fluid sample with a known amount of antibodies against a polypeptide in accordance with any one of claims 1-5; and
(b) detecting the antigen/antibody complexes resulting in the reaction mixture.

**20.** A method in accordance with claim 19, in which the antibodies are labelled with an enzyme and the antigen/antibody complexes resulting in the reaction mixture are detected by an enzyme immunoassay.

**21.** Antibodies against a polypeptide in accordance with any one of claims 1-5.

**22.** Antibodies in accordance with claim 21, which are monoclonal antibodies.

**23.** The use of a polypeptide in accordance with any one of claims 1-5 for the production of HIV antibodies.

**24.** The use of a polypeptide in accordance with any one of claims 1-5 for the detection of HIV antibodies in human sera or in other biological fluids.

**25.** The use of a polypeptide in accordance with any one of claims 1-5 for the detection of HIV viruses or fragments thereof in human sera or in other biological fluids.

**26.** A test kit for the determination of HIV viruses or fragments thereof, consisting of a container which contains HIV antibodies against a polypeptide in accordance with any one of claims 1-5.

**27.** A test kit for the determination of HIV antibodies, consisting of a container which contains a polypeptide in accordance with any one of claims 1-5.

**Revendications**

**1.** Polypeptide comportant la séquence d'aminoacides

SerAlaArgLeuAsnSerTrpGlyCysAlaPheArgGlnValCysHisThrThr
ValProTrpValAsnAspSerLeuAlaProAspTrpAspAsnMetThrTrpGln
GluTrpGluLysGlnValArgTyrLeuGluAlaAsnIleSerLysSerLeuGlu
GlnAlaGlnGly (ENV(60)) (I)

ou des fragments ou équivalents fonctionnels de celle-ci, lié par covalence à un peptide d'affinité et à un polypeptide dont la séquence d'aminoacides coïncide avec au moins un groupe déterminant antigénique et/ou immunogène de la protéine d'enveloppe (env) de HIV-1 et/ou de la protéine structurale (gag) de HIV-1.

**2.** Polypeptide selon la revendication 1, possédant la formule suivante:

MetArgGlySerGluAlaGlnGlnHisLeuLeuGlnLeuThrValTrpGlyIleLysGln
LeuGlnAlaArgIleLeuAlaValGluArgTyrLeuLysAspGlnGlnLeuLeuGlyIle
TrpGlyCysSerGlyLysLeuIleCysThrThrAlaValProTrpAsnAlaSerTrpSer
AsnLysLeuLeuGluGlnIleTrpAsnAsnMetThrTrpMetGluTrpAspArgGluIle
AsnAsnTyrThrGlySerGlyIleArgLeuArgProGlyGlyLysLysLysTyrLysLeu
LysHisIleValTrpAlaSerArgGluLeuGluArgPheAlaValAsnProGlyLeuLeu
GluThrSerGluGlyCysArgGlnIleLeuGlyGlnLeuGlnProSerLeuGlnThrGly
SerLysGluLeuArgSerLeuTyrAsnThrValAlaThrLeuTyrCysValHisGlnArg
IleGluIleLysAspThrLysGluAlaLeuAspLysValGluGluGluGlnAsnAsnSer
LysLysLysAlaGlnGlnGluAlaAlaAspAlaGlyAsnArgAsnGlnValSerGlnAsn
TyrProIleValGlnAsnLeuGlnGlyGlnMetValHisGlnAlaIleSerProArgThr
LeuAsnAlaTrpValLysValValGluGluLysAlaPheSerProGluValIleProMet
PheSerAlaLeuSerGluGlyAlaThrProGlnAspLeuAsnThrMetLeuAsnThrVal
GlyGlyHisGlnAlaAlaMetGlnMetLeuLysGluThrIleAsnGluGluAlaAlaGlu
TrpAspArgLeuHisProValHisAlaGlyProIleAlaProGlyGlnMetArgGluPro
ArgGlySerAspIleAlaGlyThrThrSerThrLeuGlnGluGlnIleGlyTrpMetThr
AsnAsnProProIleProValGlyGluIleTyrLysArgTrpIleIleLeuGlyLeuAsn
LysIleValArgMetTyrSerProThrSerIleLeuAspIleLysGlnGlyProLysGlu
ProPheArgAspTyrValAspArgPheTyrLysThrLeuArgAlaGluGlnAlaThrGln
GluValLysAsnTrpMetThrGluThrLeuLeuValGlnAsnAlaAsnProAspCysLys
ThrIleLeuLysAlaLeuGlyProAlaAlaThrLeuGluGluMetMetThrAlaCysGln

GlyValGlyGlyProGlyHisLysAlaArgValLeuAlaGluAlaMetSerGlnValThr
GlySerAlaAlaIleMetMetGlnArgGlyAsnPheArgAsnGlnArgLysThrValLys
CysPheAsnCysGlyLysGluGlyHisIleAlaArgAsnCysArgAlaProArgLysLys
GlyCysTrpLysCysGlyLysGluGlyHisGlnMetLysAspCysThrGluArgGlnAla
AsnPheLeuGlyLysIleGlyArgSerAlaArgLeuAsnSerTrpGlyCysAlaPheArg
GlnValCysHisThrThrValProTrpValAsnAspSerLeuAlaProAspTrpAspAsn
MetThrTrpGlnGluTrpGluLysGlnValArgTyrLeuGluAlaAsnIleSerLysSer
LeuGluGlnAlaGlnGlySerHisHisHisHisHisHis

(ENV(80)-GAG(419)-ENV(60)) (IV)

**3.** Polypeptide selon la revendication 1 ou 2, comportant un résidu méthionine supplémentaire à l'extrémité amino.

**4.** Polypeptide selon l'une des revendications 1 à 3, qui a été produit dans des bactéries.

**5.** Polypeptide selon l'une des revendications 1 à 3, qui a été produit dans *E. coli.*

**6.** Séquence d'ADN, codant pour un polypeptide selon l'une des revendications 1 à 3.

**7.** Séquence d'ADN selon la revendication 6, qui comprend la séquence nucléotidique

TCCGCTCGTCTGAACTCCTGGGGTTGCGCTTTTCGTCAGGTTTGCCACACTACGGTACCGTG
GGTAAACGACAGCTTAGCTCCGGACTGGGATAACATGACTTGGCAGGAATGGAAAAACAGG
TGCGCTACCTGGAGGCTAACATTTCTAAATCTCTGGAACAGGCTCAGGGA

ou des séquences partielles de celle-ci, ou des équivalents fonctionnels de cette séquence nucléotidique ou de ces séquences partielles.

**8.** Vecteur d'expression, dans lequel une séquence d'ADN selon la revendication 6 ou 7 est fonctionnellement liée à une séquence régulatrice d'expression.

**9.** Vecteur d'expression selon la revendication 8, qui peut se répliquer dans *E. coli.*

**10.** Vecteur d'expression selon la revendication 9, qui est le plasmide pRBSII-env(80)-gag(419)-env(60)-6xHIS.

**11.** Bactérie transformée par un vecteur d'expression selon l'une des revendications 8 à 10.

**12.** Souche de *E. coli* transformée par un vecteur d'expression selon l'une des revendications 8 à 10.

**13.** Souche M15 de *E. coli* transformée par un vecteur d'expression selon l'une des revendications 8 à 13.

**14.** Polypeptide selon l'une des revendications 1 à 5, en tant qu'antigène.

**15.** Procédé pour la production d'un polypeptide selon l'une des revendications 1 à 5, caractérisé en ce que l'on transforme une bactérie par un vecteur d'expression selon l'une des revendications 8 à 10, puis on la cultive dans des conditions de croissance appropriées, et on isole et, si on le désire, purifie ledit polypeptide.

**16.** Procédé pour la production d'anticorps contre le HIV, caractérisé en ce que l'on injecte à un mammifère ou un oiseau une quantité suffisante d'un polypeptide selon l'une des revendications 1 à 5, et on isole à partir du sérum de ces animaux les anticorps formés.

**17.** Procédé pour la détection d'anticorps dirigés contre des virus de type HIV dans des sérums humains, caractérisé en ce que

(a) on marque un polypeptide selon l'une des revendications 1 à 5,;
(b) on fait réagir ce polypeptide marqué avec un échantillon de sérum humain; et
(c) on met en évidence les complexes polypeptide/anticorps marqués, formés dans le mélange réactionnel.

**18.** Procédé pour la détection d'anticorps dirigés contre des virus de type HIV dans des sérums humains, caractérisé en ce que

(a) on immobilise sur un matériau de support solide un polypeptide selon l'une des revendications 1 à 5;
(b) on met en contact un échantillon de sérum humain avec cette protéine immobilisée;
(c) on élimine par lavage les anticorps et polypeptides non liés; et
(d) on met en évidence les complexes polypeptide/anticorps immobilisés, lavés, par addition de protéine A marquée de *Staphylococcus aureus* ou par addition d'anticorps anti-Ig humains marqués.

**19.** Procédé pour la détection de virus de type HIV ou de fragments de ceux-ci, dans des sérums humains ou d'autres liquides biologiques, caractérisé en ce que

(a) on fait réagir un échantillon de sérum humain ou un autre liquide corporel avec une quantité connue d'anticorps dirigés contre un polypeptide selon l'une des revendications 1 à 5; et
(b) on met en évidence les complexes antigène/anticorps formés dans le mélange réactionnel.

**20.** Procédé selon la revendication 19 dans lequel les anticorps sont marqués à l'aide d'une enzyme et les complexes antigène/anticorps formés dans le mélange réactionnel sont mis en évidence au moyen d'un essai immuno-enzymatique.

**21.** Anticorps dirigé contre un polypeptide selon l'une des revendications 1 à 5.

**22.** Anticorps selon la revendication 21, qui sont des anticorps monoclonaux.

**23.** Utilisation d'un polypeptide selon l'une des revendications 1 à 5, pour la production d'anticorps contre le HIV.

**24.** Utilisation d'un polypeptide selon l'une des revendications 1 à 5, pour la détection d'anticorps contre le HIV dans des sérums humains ou d'autres liquides biologiques.

**25.** Utilisation d'un polypeptide selon l'une des revendications 1 à 5, pour la détection de virus de type HIV ou de fragments de ceux-ci dans des sérums humains ou d'autres liquides biologiques.

**26.** Nécessaire d'essai pour la détermination de virus de type HIV ou de fragments de ceux-ci, constitué d'un récipient qui contient des anticorps contre le HIV, dirigés contre un polypeptide selon l'une des revendicatioins 1 à 5,

**27.** Nécessaire d'essai pour la détermination d'anticorps contre le HIV, constitué d'un récipient qui contient un polypeptide selon l'une des revendications 1 à 5.

**Fig. 1**

(1) (2) (3) (4)
SerAlaArgLeuAsnSerTrpGlyCysAlaPheArgGlnValCysHisThrThrValProTrpValAsnAspSerLeuAlaProAsp
GATCCGCTCGTCTGAACTCCTGGGGTTGCGCTTTTCGTCAGGTTTGCCACACTACGGTACCGTGGGTAAACGACAGCTTAGCTCCGGAC
GCGAGCAGACTTGAGGACCCCAACGCGAAAAGCAGTCCAAACGGTGTGATGCCATGGCACCCATTTGCTGTCGAATCGAGGCCTG
(8) (9) (10)

(4) (5) (6) (7)
TrpAspAsnMetThrTrpGlnGluTrpGluLysGlnValArgTyrLeuGluAlaAsnIleSerLysSerLeuGluGlnAlaGlnGly
TGGGATAACATGACTTGGCAGGAATGGGAAAAACAGGTGCGCTACCTGGAGGCTAACATTTCTAAATCTCTGGAACAGGCTCAGG
ACCCTATTGTACTGAACCGTCCTTACCCTTTTTGTCCACGCGATGGACCTCCGATTGTAAAGATTTAGAGACCTTGTCCGAGTCCCTAG
(11) (12) (13) (14)

Fig. 2

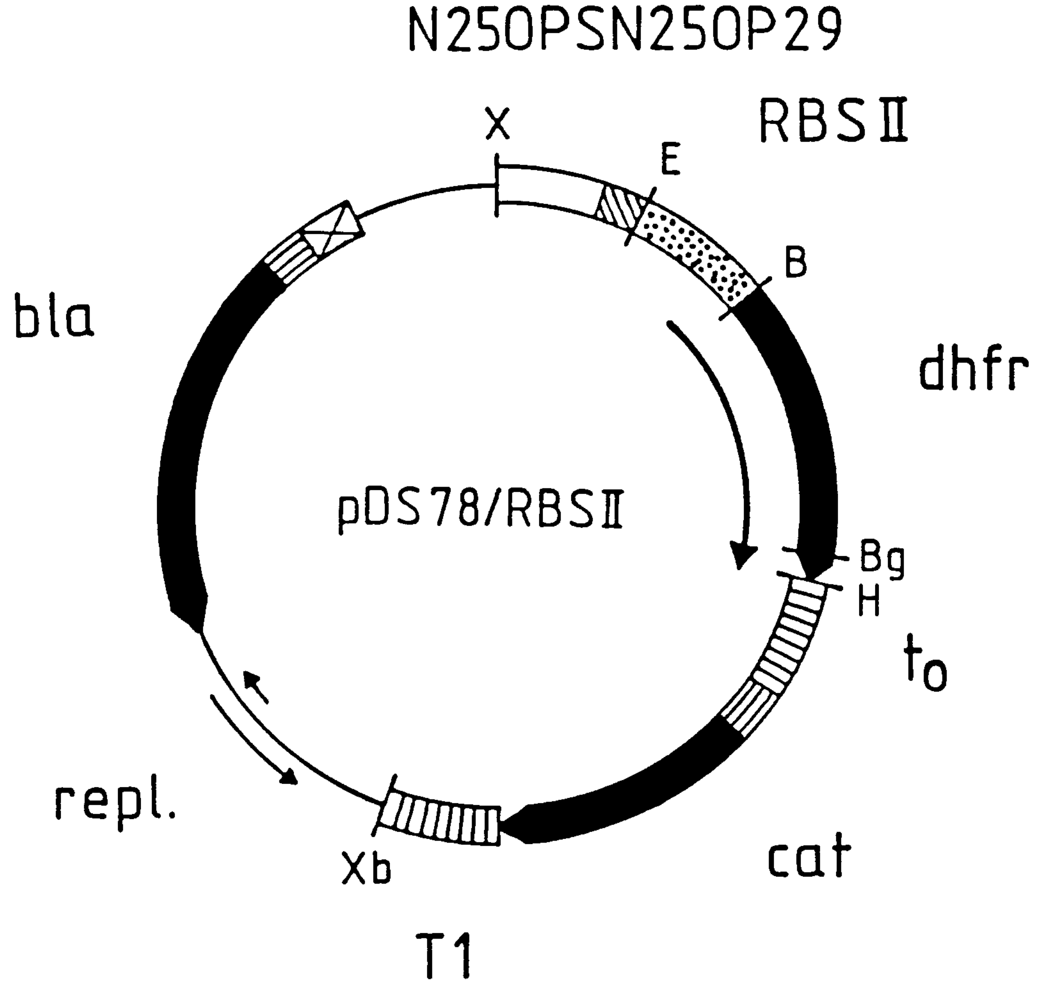
N25OPSN25OP29
X
E
RBSII
B
bla
dhfr
pDS78/RBSII
Bg
H
$t_o$
repl.
Xb
cat
T1

# Fig. 3

```
                10         20         30         40         50
                 |          |          |          |          |
   1 CTCGAGAAAT CATAAAAAAT TTATTTGCTT TGTGAGCGGA TAACAATTAT
  51 AATAGATTCA ATTGTGAGCG GATAACAATT TCACACAGAA TTCATTAAAG
 101 AGGAGAAATT AACTATGAGA GGATCCGGCA TCATGGTTCG ACCATTGAAC
 151 TGCATCGTCG CCGTGTCCCA AAATATGGGG ATTGGCAAGA ACGGAGACCT
 201 ACCCTGGCCT CCGCTCAGGA ACGAGTTCAA GTACTTCCAA AGAATGACCA
 251 CAACCTCTTC AGTGGAAGGT AAACAGAATC TGGTGATTAT GGGTAGGAAA
 301 ACCTGGTTCT CCATTCCTGA GAAGAATCGA CCTTTAAAGG ACAGAATTAA
 351 TATAGTTCTC AGTAGAGAAC TCAAAGAACC ACCACGAGGA GCTCATTTTC
 401 TTGCCAAAAG TTTGGATGAT GCCTTAAGAC TTATTGAACA ACCGGAATTG
 451 GCAAGTAAAG TAGACATGGT TTGGATAGTC GGAGGCAGTT CTGTTTACCA
 501 GGAAGCCATG AATCAACCAG GCCACCTTAG ACTCTTTGTG ACAAGGATCA
 551 TGCAGGAATT TGAAAGTGAC ACGTTTTTCC CAGAAATTGA TTTGGGGAAA
 601 TATAAACTTC TCCCAGAATA CCCAGGCGTC CTCTCTGAGG TCCAGGAGGA
 651 AAAAGGCATC AAGTATAAGT TTGAAGTCTA CGAGAAGAAA GGTTCCAGAT
 701 CTGTTAACCT AGTTTAACAG GAAGATGCTT TCAAGTTCTC TGCTCCCCTC
 751 CTAAAGCTAT GCATTTTTAT AAGACCATGG GACTTTTGCT GGCTTTAGAT
 801 CCGGCCAAGC TTGGACTCCT GTTGATAGAT CCAGTAATGA CCTCAGAACT
 851 CCATCTGGAT TTGTTCAGAA CGCTCGGTTG CCGCCGGGCG TTTTTTATTG
 901 GTGAGAATCC AAGCTAGCTT GGCGAGATTT TCAGGAGCTA AGGAAGCTAA
 951 AATGGAGAAA AAAATCACTG GATATACCAC CGTTGATATA TCCCAATGGC
1001 ATCGTAAAGA ACATTTTGAG GCATTTCAGT CAGTTGCTCA ATGTACCTAT
1051 AACCAGACCG TTCAGCTGGA TATTACGGCC TTTTTAAAGA CCGTAAAGAA
1101 AAATAAGCAC AAGTTTTATC CGGCCTTTAT TCACATTCTT GCCCGCCTGA
1151 TGAATGCTCA TCCGGAATTT CGTATGGCAA TGAAAGACGG TGAGCTGGTG
```

**Fig. 3** (Fortsetzung)

1201 ATATGGGATA GTGTTCACCC TTGTTACACC GTTTTCCATG AGCAAACTGA

1251 AACGTTTTCA TCGCTCTGGA GTGAATACCA CGACGATTTC CGGCAGTTTC

1301 TACACATATA TTCGCAAGAT GTGGCGTGTT ACGGTGAAAA CCTGGCCTAT

1351 TTCCCTAAAG GGTTTATTGA GAATATGTTT TTCGTCTCAG CCAATCCCTG

1401 GGTGAGTTTC ACCAGTTTTG ATTTAAACGT GGCCAATATG GACAACTTCT

1451 TCGCCCCCGT TTTCACCATG GGCAAATATT ATACGCAAGG CGACAAGGTG

1501 CTGATGCCGC TGGCGATTCA GGTTCATCAT GCCGTCTGTG ATGGCTTCCA

1551 TGTCGGCAGA ATGCTTAATG AATTACAACA GTACTGCGAT GAGTGGCAGG

1601 GCGGGGCGTA ATTTTTTTAA GGCAGTTATT GGTGCCCTTA AACGCCTGGG

1651 GTAATGACTC TCTAGCTTGA GGCATCAAAT AAAACGAAAG GCTCAGTCGA

1701 AAGACTGGGC CTTTCGTTTT ATCTGTTGTT TGTCGGTGAA CGCTCTCCTG

1751 AGTAGGACAA ATCCGCCGCT CTAGAGCTGC CTCGCGCGTT TCGGTGATGA

1801 CGGTGAAAAC CTCTGACACA TGCAGCTCCC GGAGACGGTC ACAGCTTGTC

1851 TGTAAGCGGA TGCCGGGAGC AGACAAGCCC GTCAGGGCGC GTCAGCGGGT

1901 GTTGGCGGGT GTCGGGGCGC AGCCATGACC CAGTCACGTA GCGATAGCGG

1951 AGTGTATACT GGCTTAACTA TGCGGCATCA GAGCAGATTG TACTGAGAGT

2001 GCACCATATG CGGTGTGAAA TACCGCACAG ATGCGTAAGG AGAAAATACC

2051 GCATCAGGCG CTCTTCCGCT TCCTCGCTCA CTGACTCGCT GCGCTCGGTC

2101 TGTCGGCTGC GGCGAGCGGT ATCAGCTCAC TCAAAGGCGG TAATACGGTT

2151 ATCCACAGAA TCAGGGGATA ACGCAGGAAA GAACATGTGA GCAAAAGGCC

2201 AGCAAAAGGC CAGGAACCGT AAAAAGGCCG CGTTGCTGGC GTTTTTCCAT

2251 AGGCTCCGCC CCCCTGACGA GCATCACAAA AATCGACGCT CAAGTCAGAG

2301 GTGGCGAAAC CCGACAGGAC TATAAAGATA CCAGGCGTTT CCCCCTGGAA

2351 GCTCCCTCGT GCGCTCTCCT GTTCCGACCC TGCCGCTTAC CGGATACCTG

2401 TCCGCCTTTC TCCCTTCGGG AAGCGTGGCG CTTTCTCAAT GCTCACGCTG

2451 TAGGTATCTC AGTTCGGTGT AGGTCGTTCG CTCCAAGCTG GGCTGTGTGC

2501 ACGAACCCCC CGTTCAGCCC GACCGCTGCG CCTTATCCGG TAACTATCGT

**Fig. 3** (Fortsetzung)

2551 CTTGAGTCCA ACCCGGTAAG ACACGACTTA TCGCCACTGG CAGCAGCCAC
2601 TGGTAACAGG ATTAGCAGAG CGAGGTATGT AGGCGGTGCT ACAGAGTTCT
2651 TGAAGTGGTG GCCTAACTAC GGCTACACTA GAAGGACAGT ATTTGGTATC
2701 TGCGCTCTGC TGAAGCCAGT TACCTTCGGA AAAAGAGTTG GTAGCTCTTG
2751 ATCCGGCAAA CAAACCACCG CTGGTAGCGG TGGTTTTTTT GTTTGCAAGC
2801 AGCAGATTAC GCGCAGAAAA AAAGGATCTC AAGAAGATCC TTTGATCTTT
2851 TCTACGGGGT CTGACGCTCA GTGGAACGAA AACTCACGTT AAGGGATTTT
2901 GGTCATGAGA TTATCAAAAA GGATCTTCAC CTAGATCCTT TTAAATTAAA
2951 AATGAAGTTT TAAATCAATC TAAAGTATAT ATGAGTAAAC TTGGTCTGAC
3001 AGTTACCAAT GCTTAATCAG TGAGGCACCT ATCTCAGCGA TCTGTCTATT
3051 TCGTTCATCC ATAGCTGCCT GACTCCCCGT CGTGTAGATA ACTACGATAC
3101 GGGAGGGCTT ACCATCTGGC CCCAGTGCTG CAATGATACC GCGAGACCCA
3151 CGCTCACCGG CTCCAGATTT ATCAGCAATA AACCAGCCAG CCGGAAGGGC
3201 CGAGCGCAGA AGTGGTCCTG CAACTTTATC CGCCTCCATC CAGTCTATTA
3251 ATTGTTGCCG GGAAGCTAGA GTAAGTAGTT CGCCAGTTAA TAGTTTGCGC
3301 AACGTTGTTG CCATTGCTAC AGGCATCGTG GTGTCACGCT CGTCGTTTGG
3351 TATGGCTTCA TTCAGCTCCG GTTCCCAACG ATCAAGGCGA GTTACATGAT
3401 CCCCCATGTT GTGCAAAAAA GCGGTTAGCT CCTTCGGTCC TCCGATCGTT
3451 GTCAGAAGTA AGTTGGCCGC AGTGTTATCA CTCATGGTTA TGGCAGCACT
3501 GCATAATTCT CTTACTGTCA TGCCATCCGT AAGATGCTTT TCTGTGACTG
3551 GTGAGTACTC AACCAAGTCA TTCTGAGAAT AGTGTATGCG GCGACCGAGT
3601 TGCTCTTGCC CGGCGTCAAT ACGGGATAAT ACCGCGCCAC ATAGCAGAAC
3651 TTTAAAAGTG CTCATCATTG GAAAACGTTC TTCGGGGCGA AAACTCTCAA
3701 GGATCTTACC GCTGTTGAGA TCCAGTTCGA TGTAACCCAC TCGTGCACCC
3751 AACTGATCTT CAGCATCTTT TACTTTCACC AGCGTTTCTG GGTGAGCAAA
3801 AACAGGAAGG CAAAATGCCG CAAAAAAGGG AATAAGGGCG ACACGGAAAT
3851 GTTGAATACT CATACTCTTC CTTTTTCAAT ATTATTGAAG CATTTATCAG
3901 GGTTATTGTC TCATGAGCGG ATACATATTT GAATGTATTT AGAAAAATAA

**Fig. 3** (Fortsetzung)

3951 ACAAATAGGG GTTCCGCGCA CATTTCCCCG AAAAGTGCCA CCTGACGTCT

4001 AAGAAACCAT TATTATCATG ACATTAACCT ATAAAAATAG GCGTATCACG

4051 AGGCCCTTTC GTCTTCAC

Fig.4

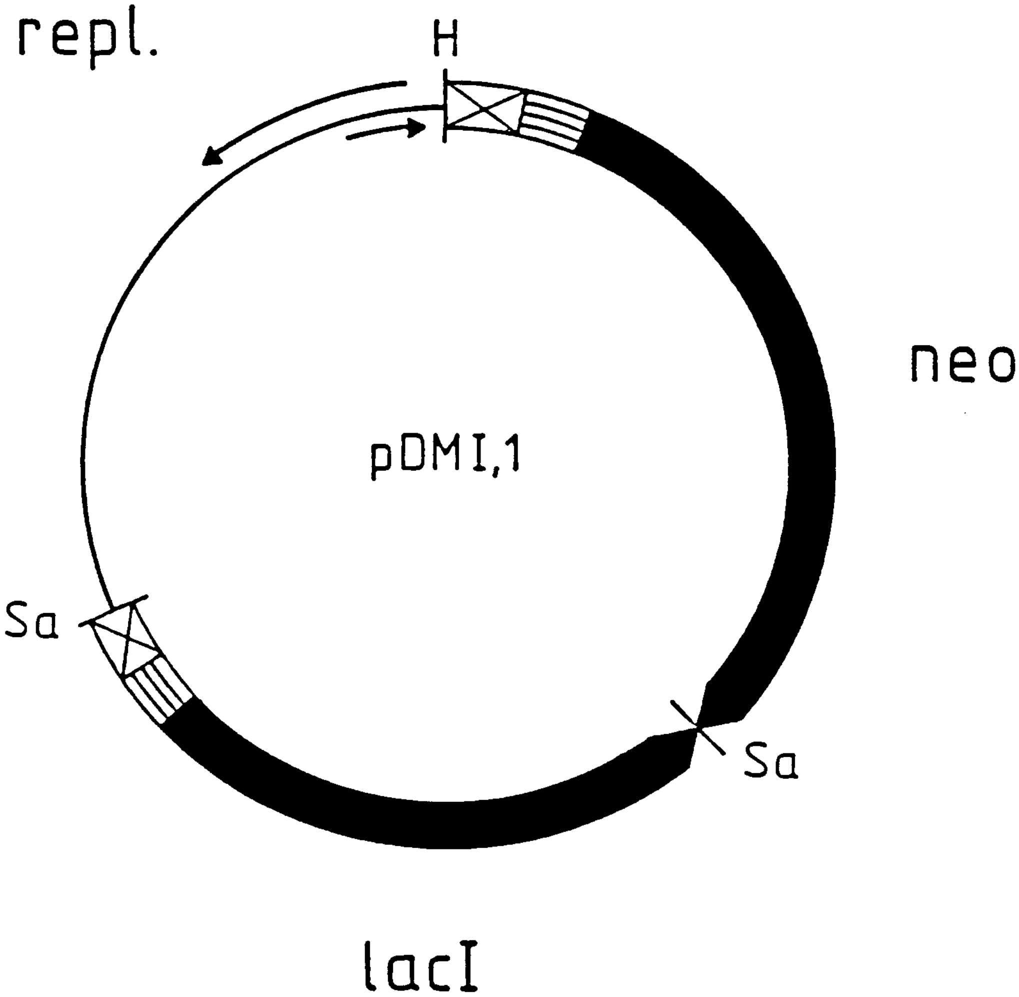

# Fig. 5

```
             10         20         30         40         50
              |          |          |          |          |
   1 AAGCTTCACG CTGCCGCAAG CACTCAGGGC GCAAGGGCTG CTAAAGGAAG
  51 CGGAACACGT AGAAAGCCAG TCCGCAGAAA CGGTGCTGAC CCCGGATGAA
 101 TGTCAGCTAC TGGGCTATCT GGACAAGGGA AAACGCAAGC GCAAAGAGAA
 151 AGCAGGTAGC TTGCAGTGGG CTTACATGGC GATAGCTAGA CTGGGCGGTT
 201 TTATGGACAG CAAGCGAACC GGAATTGCCA GCTGGGGCGC CCTCTGGTAA
 251 GGTTGGGAAG CCCTGCAAAG TAAACTGGAT GGCTTTCTTG CCGCCAAGGA
 301 TCTGATGGCG CAGGGGATCA AGATCTGATC AAGAGACAGG ATGAGGATCG
 351 TTTCGCATGA TTGAACAAGA TGGATTGCAC GCAGGTTCTC CGGCCGCTTG
 401 GGTGGAGAGG CTATTCGGCT ATGACTGGGC ACAACAGACA ATCGGCTGCT
 451 CTGATGCCGC CGTGTTCCGG CTGTCAGCGC AGGGGCGCCC GGTTCTTTTT
 501 GTCAAGACCG ACCTGTCCGG TGCCCTGAAT GAACTGCAGG ACGAGGCAGC
 551 GCGGCTATCG TGGCTGGCCA CGACGGGCGT TCCTTGCGCA GCTGTGCTCG
 601 ACGTTGTCAC TGAAGCGGGA AGGGACTGGC TGCTATTGGG CGAAGTGCCG
 651 GGGCAGGATC TCCTGTCATC TCACCTTGCT CCTGCCGAGA AAGTATCCAT
 701 CATGGCTGAT GCAATGCGGC GGCTGCATAC GCTTGATCCG GCTACCTGCC
 751 CATTCGACCA CCAAGCGAAA CATCGCATCG AGCGAGCACG TACTCGGATG
 801 GAAGCCGGTC TTGTCGATCA GGATGATCTG GACGAAGAGC ATCAGGGGCT
 851 CGCGCCAGCC GAACTGTTCG CCAGGCTCAA GGCGCGCATG CCCGACGGCG
 901 AGGATCTCGT CGTGACCCAT GGCGATGCCT GCTTGCCGAA TATCATGGTG
 951 GAAAATGGCC GCTTTTCTGG ATTCATCGAC TGTGGCCGGC TGGGTGTGGC
1001 GGACCGCTAT CAGGACATAG CGTTGGCTAC CCGTGATATT GCTGAAGAGC
1051 TTGGCGGCGA ATGGGCTGAC CGCTTCCTCG TGCTTTACGG TATCGCCGCT
1101 CCCGATTCGC AGCGCATCGC CTTCTATCGC CTTCTTGACG AGTTCTTCTG
1151 AGCGGGACTC TGGGGTTCGA AATGACCGAC CAAGCGACGC CCAACCTGCC
```

**Fig. 5** (Fortsetzung)

1201 ATCACGAGAT TTCGATTCCA CCGCCGCCTT CTATGAAAGG TTGGGCTTCG
1251 GAATCGTTTT CCGGGACGCC GGCTGGATGA TCCTCCAGCG CGGGGATCTC
1301 ATGCTGGAGT TCTTCGCCCA CCCCGGGCTC GATCCCCTCG CGAGTTGGTT
1351 CAGCTGCTGC CTGAGGCTGG ACGACCTCGC GGAGTTCTAC CGGCAGTGCA
1401 AATCCGTCGG CATCCAGGAA ACCAGCAGCG GCTATCCGCG CATCCATGCC
1451 CCCGAACTGC AGGAGTGGGG AGGCACGATG GCCGCTTTGG TCGACAATTC
1501 GCGCTAACTT ACATTAATTG CGTTGCGCTC ACTGCCCGCT TTCCAGTCGG
1551 GAAACCTGTC GTGCCAGCTG CATTAATGAA TCGGCCAACG CGCGGGGAGA
1601 GGCGGTTTGC GTATTGGGCG CCAGGGTGGT TTTTCTTTTC ACCAGTGAGA
1651 CGGGCAACAG CTGATTGCCC TTCACCGCCT GGCCCTGAGA GAGTTGCAGC
1701 AAGCGGTCCA CGCTGGTTTG CCCCAGCAGG CGAAAATCCT GTTTGATGGT
1751 GGTTAACGGC GGGATATAAC ATGAGCTGTC TTCGGTATCG TCGTATCCCA
1801 CTACCGAGAT ATCCGCACCA ACGCGCAGCC CGGACTCGGT AATGGCGCGC
1851 ATTGCGCCCA GCGCCATCTG ATCGTTGGCA ACCAGCATCG CAGTGGGAAC
1901 GATGCCCTCA TTCAGCATTT GCATGGTTTG TTGAAAACCG GACATGGCAC
1951 TCCAGTCGCC TTCCCGTTCC GCTATCGGCT GAATTTGATT GCGAGTGAGA
2001 TATTTATGCC AGCCAGCCAG ACGCAGACGC GCCGAGACAG AACTTAATGG
2051 GCCCGCTAAC AGCGCGATTT GCTGGTGACC CAATGCGACC AGATGCTCCA
2101 CGCCCAGTCG CGTACCGTCT TCATGGGAGA AAATAATACT GTTGATGGGT
2151 GTCTGGTCAG AGACATCAAG AAATAACGCC GGAACATTAG TGCAGGCAGC
2201 TTCCACAGCA ATGGCATCCT GGTCATCCAG CGGATAGTTA ATGATCAGCC
2251 CACTGACGCG TTGCGCGAGA AGATTGTGCA CCGCCGCTTT ACAGGCTTCG
2301 ACGCCGCTTC GTTCTACCAT CGACACCACC ACGCTGGCAC CCAGTTGATC
2351 GGCGCGAGAT TTAATCGCCG CGACAATTTG CGACGGCGCG TGCAGGGCCA
2401 GACTGGAGGT GGCAACGCCA ATCAGCAACG ACTGTTTGCC CGCCAGTTGT
2451 TGTGCCACGC GGTTGGGAAT GTAATTCAGC TCCGCCATCG CCGCTTCCAC
2501 TTTTTCCCGC GTTTTCGCAG AAACGTGGCT GGCCTGGTTC ACCACGCGGG

**Fig. 5** (Fortsetzung)

2551 AAACGGTCTG ATAAGAGACA CCGGCATACT CTGCGACATC GTATAACGTT
2601 ACTGGTTTCA CATTCACCAC CCTGAATTGA CTCTCTTCCG GGCGCTATCA
2651 TGCCATACCG CGAAAGGTTT TGCACCATTC GATGGTGTCA ACGTAAATGC
2701 ATGCCGCTTC GCCTTCGCGC GCGAATTGTC GACCCTGTCC CTCCTGTTCA
2751 GCTACTGACG GGGTGGTGCG TAACGGCAAA AGCACCGCCG GACATCAGCG
2801 CTAGCGGAGT GTATACTGGC TTACTATGTT GGCACTGATG AGGGTGTCAG
2851 TGAAGTGCTT CATGTGGCAG GAGAAAAAAG GCTGCACCGG TGCGTCAGCA
2901 GAATATGTGA TACAGGATAT ATTCCGCTTC CTCGCTCACT GACTCGCTAC
2951 GCTCGGTCGT TCGACTGCGG CGAGCGGAAA TGGCTTACGA ACGGGGCGGA
3001 GATTTCCTGG AAGATGCCAG GAAGATACTT AACAGGGAAG TGAGAGGGCC
3051 GCGGCAAAGC CGTTTTTCCA TAGGCTCCGC CCCCCTGACA AGCATCACGA
3101 AATCTGACGC TCAAATCAGT GGTGGCGAAA CCCGACAGGA CTATAAAGAT
3151 ACCAGGCGTT TCCCCTGGCG GCTCCCTCGT GCGCTCTCCT GTTCCTGCCT
3201 TTCGGTTTAC CGGTGTCATT CCGCTGTTAT GGCCGCGTTT GTCTCATTCC
3251 ACGCCTGACA CTCAGTTCCG GGTAGGCAGT TCGCTCCAAG CTGGACTGTA
3301 TGCACGAACC CCCCGTTCAG TCCGACCGCT GCGCCTTATC CGGTAACTAT
3351 CGTCTTGAGT CCAACCCGGA AAGACATGCA AAAGCACCAC TGGCAGCAGC
3401 CACTGGTAAT TGATTTAGAG GAGTTAGTCT TGAAGTCATG CGCCGGTTAA
3451 GGCTAAACTG AAAGGACAAG TTTTGGTGAC TGCGCTCCTC CAAGCCAGTT
3501 ACCTCGGTTC AAAGAGTTGG TAGCTCAGAG AACCTTCGAA AAACCGCCCT
3551 GCAAGGCGGT TTTTTCGTTT TCAGAGCAAG AGATTACGCG CAGACCAAAA
3601 CGATCTCAAG AAGATCATCT TATTAATCAG ATAAAATATT TCTAGATTTC
3651 AGTGCAATTT ATCTCTTCAA ATGTAGCACC TGAAGTCAGC CCCATACGAT
3701 ATAAGTTGTT AATTCTCATG TTTGACAGCT TATCATCGAT

Fig. 6

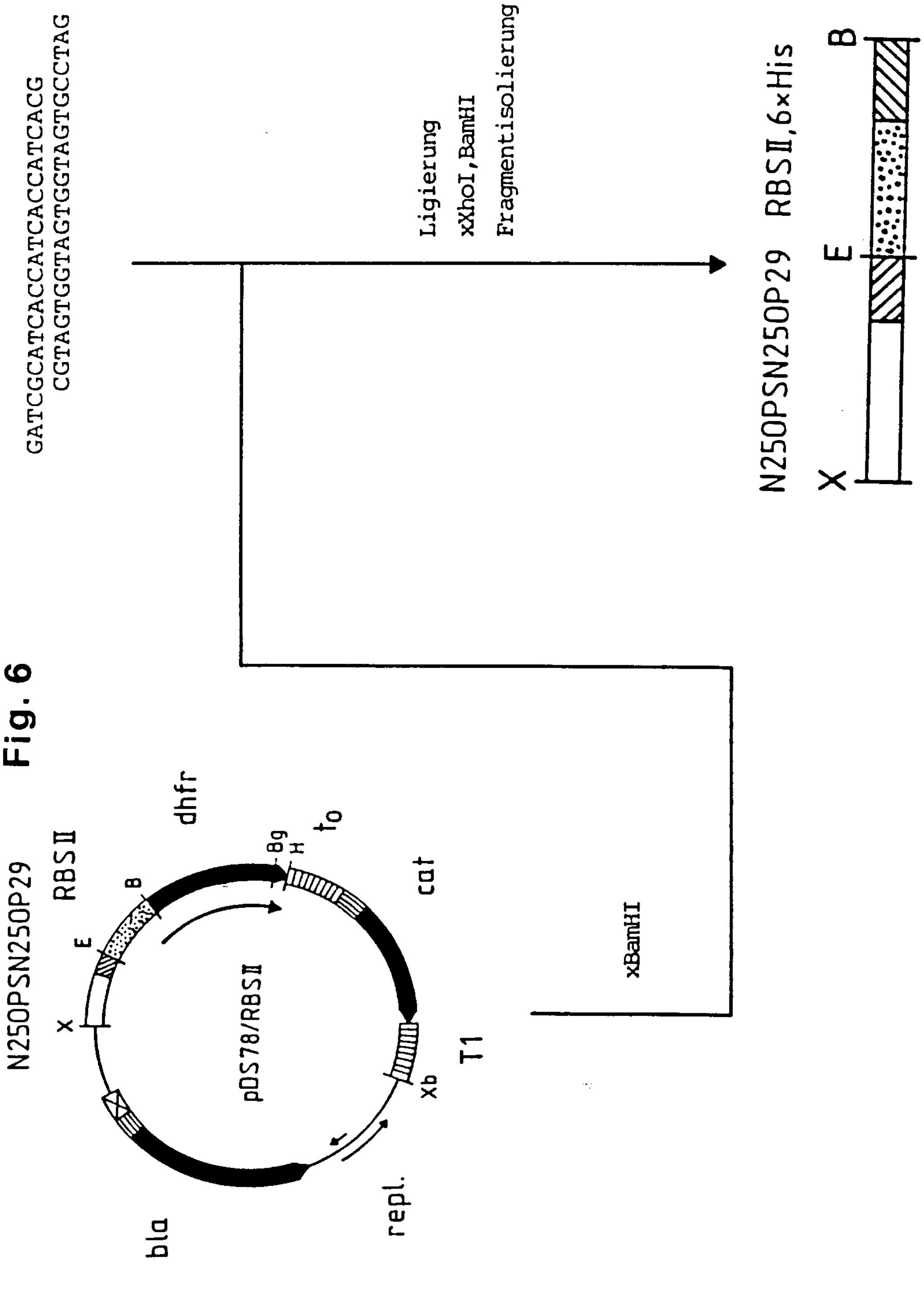
GATCGCATCACCATCACCATCACG
CGTAGTGGTAGTGGTAGTGCCTAG
Ligierung
xXhoI, BamHI
Fragmentisolierung
N250PSN250P29 RBSII,6×His
X
E
B
N250PSN250P29
RBSII
dhfr
Bg
H
to
cat
T1
Xb
repl.
bla
pDS78/RBSII
xBamHI

Fig.7

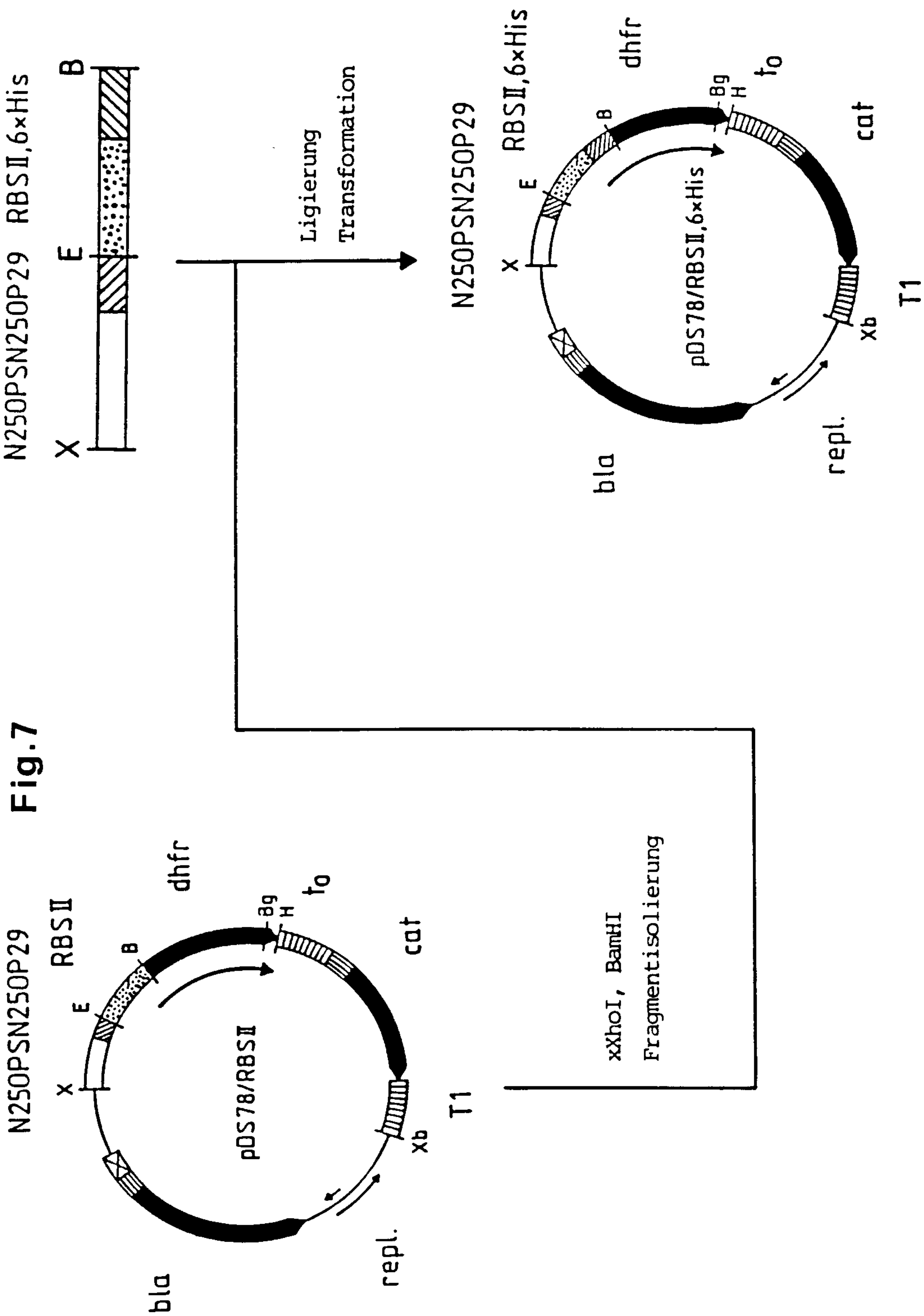
N250PSN250P29
RBSII,6×His
X
E
B
Ligierung
Transformation
N250PSN250P29
RBSII,6×His
dhfr
Bg
H
$t_o$
cat
T1
Xb
repl.
bla
pDS78/RBSII,6×His
N250PSN250P29
RBSII
dhfr
pDS78/RBSII
cat
T1
Xb
repl.
bla
xXhoI, BamHI
Fragmentisolierung

Fig.8

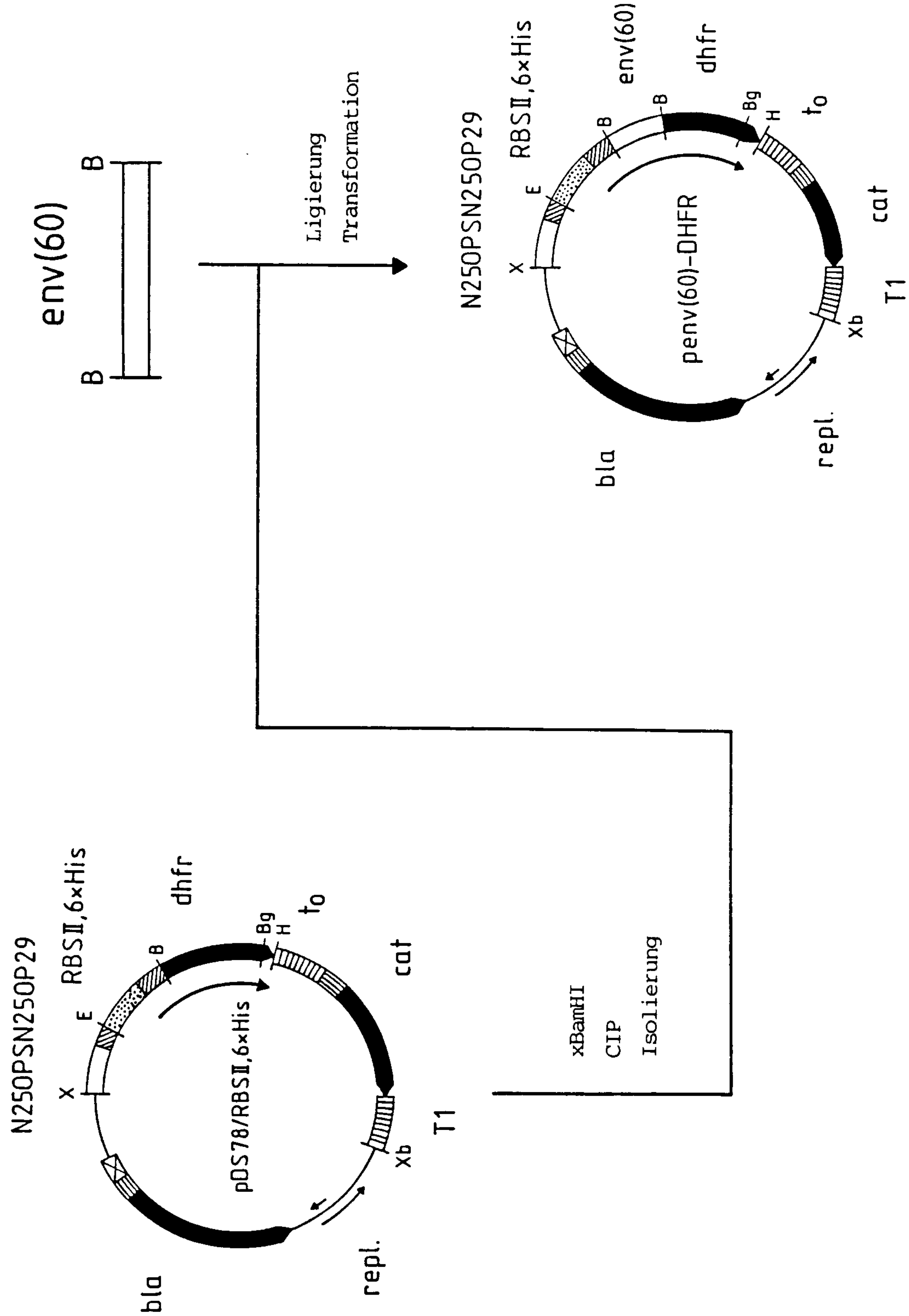

env(60)
B
B
Ligierung
Transformation
N25OPSN25OP29
RBSII,6×His
env(60)
dhfr
X
E
B
B
Bg
H
$t_o$
cat
T1
Xb
repl.
bla
penv(60)-DHFR
N25OPSN25OP29
RBSII,6×His
dhfr
X
E
B
Bg
H
$t_o$
cat
T1
Xb
repl.
bla
pDS78/RBSII,6×His
xBamHI
CIP
Isolierung

Fig.9

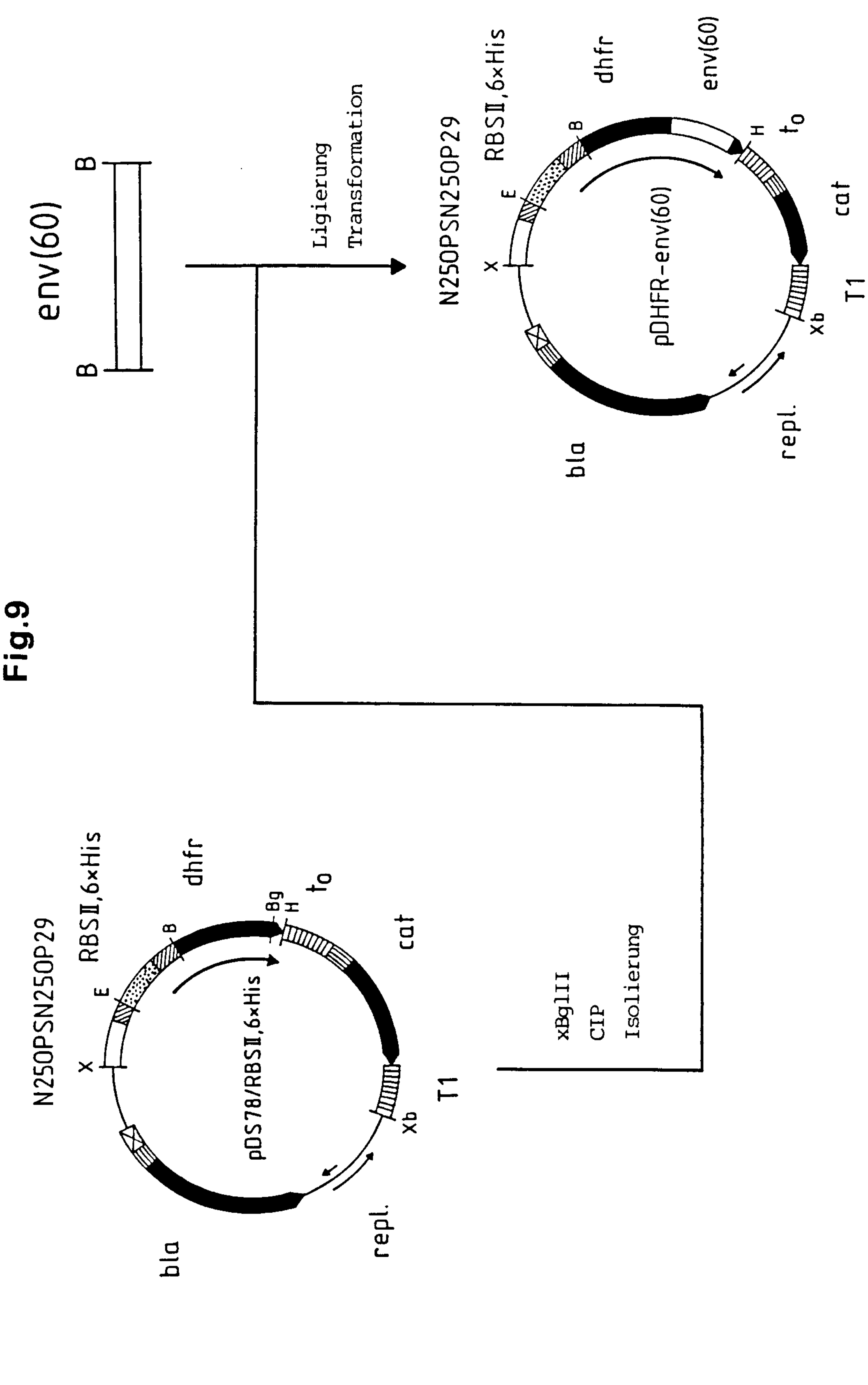
env(60)
B
B
Ligierung
Transformation
N250PSN250P29
RBSII,6×His
dhfr
env(60)
E
B
H
X
$t_o$
cat
pDHFR-env(60)
T1
Xb
bla
repl.
N250PSN250P29
RBSII,6×His
dhfr
Bg
H
$t_o$
E
B
X
cat
pDS78/RBSII,6×His
T1
Xb
xBglII
CIP
Isolierung
bla
repl.

Fig.10

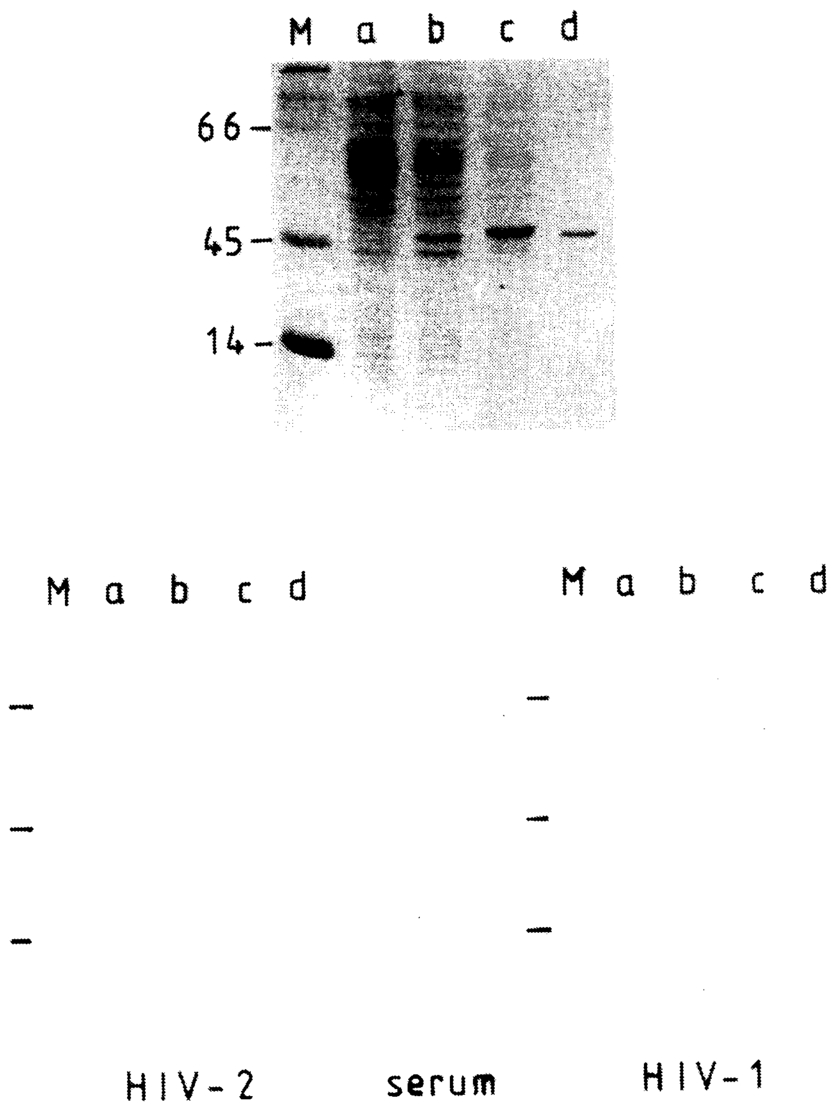

Fig.11

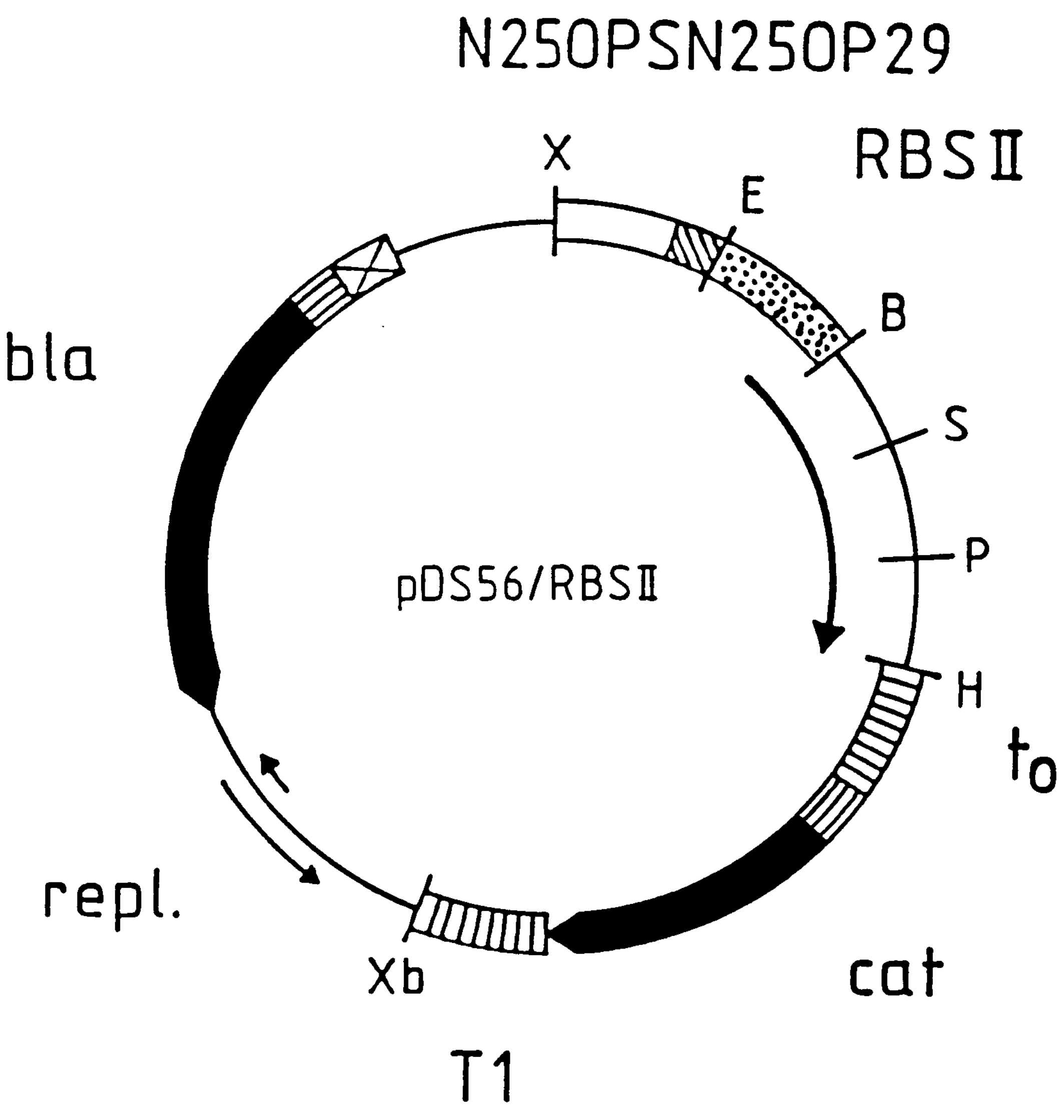
N250PSN250P29
X
E
RBSII
B
S
P
H
to
cat
T1
Xb
repl.
bla
pDS56/RBSII

# Fig.12

```
                10         20         30         40         50
                 |          |          |          |          |
   1 CTCGAGAAAT CATAAAAAAT TTATTTGCTT TGTGAGCGGA TAACAATTAT
  51 AATAGATTCA ATTGTGAGCG GATAACAATT TCACACAGAA TTCATTAAAG
 101 AGGAGAAATT AACTATGAGA GGATCCGTCG ACCTGCAGCC AAGCTTAATT
 151 AGCTGAGCTT GGACTCCTGT TGATAGATCC AGTAATGACC TCAGAACTCC
 201 ATCTGGATTT GTTCAGAACG CTCGGTTGCC GCCGGGCGTT TTTTATTGGT
 251 GAGAATCCAA GCTAGCTTGG CGAGATTTTC AGGAGCTAAG GAAGCTAAAA
 301 TGGAGAAAAA AATCACTGGA TATACCACCG TTGATATATC CCAATGGCAT
 351 CGTAAAGAAC ATTTTGAGGC ATTTCAGTCA GTTGCTCAAT GTACCTATAA
 401 CCAGACCGTT CAGCTGGATA TTACGGCCTT TTTAAAGACC GTAAAGAAAA
 451 ATAAGCACAA GTTTTATCCG GCCTTTATTC ACATTCTTGC CCGCCTGATG
 501 AATGCTCATC CGGAATTTCG TATGGCAATG AAAGACGGTG AGCTGGTGAT
 551 ATGGGATAGT GTTCACCCTT GTTACACCGT TTTCCATGAG CAAACTGAAA
 601 CGTTTTCATC GCTCTGGAGT GAATACCACG ACGATTTCCG GCAGTTTCTA
 651 CACATATATT CGCAAGATGT GGCGTGTTAC GGTGAAAACC TGGCCTATTT
 701 CCCTAAAGGG TTTATTGAGA ATATGTTTTT CGTCTCAGCC AATCCCTGGG
 751 TGAGTTTCAC CAGTTTTGAT TTAAACGTGG CCAATATGGA CAACTTCTTC
 801 GCCCCCGTTT TCACCATGGG CAAATATTAT ACGCAAGGCG ACAAGGTGCT
 851 GATGCCGCTG GCGATTCAGG TTCATCATGC CGTCTGTGAT GGCTTCCATG
 901 TCGGCAGAAT GCTTAATGAA TTACAACAGT ACTGCGATGA GTGGCAGGGC
 951 GGGGCGTAAT TTTTTTAAGG CAGTTATTGG TGCCCTTAAA CGCCTGGGGT
1001 AATGACTCTC TAGCTTGAGG CATCAAATAA AACGAAAGGC TCAGTCGAAA
1051 GACTGGGCCT TTCGTTTTAT CTGTTGTTTG TCGGTGAACG CTCTCCTGAG
1101 TAGGACAAAT CCGCCGCTCT AGAGCTGCCT CGCGCGTTTC GGTGATGACG
1151 GTGAAAACCT CTGACACATG CAGCTCCCGG AGACGGTCAC AGCTTGTCTG
```

**Fig.12** (Fortsetzung)

1201 TAAGCGGATG CCGGGAGCAG ACAAGCCCGT CAGGGCGCGT CAGCGGGTGT
1251 TGGCGGGTGT CGGGGCGCAG CCATGACCCA GTCACGTAGC GATAGCGGAG
1301 TGTATACTGG CTTAACTATG CGGCATCAGA GCAGATTGTA CTGAGAGTGC
1351 ACCATATGCG GTGTGAAATA CCGCACAGAT GCGTAAGGAG AAAATACCGC
1401 ATCAGGCGCT CTTCCGCTTC CTCGCTCACT GACTCGCTGC GCTCGGTCTG
1451 TCGGCTGCGG CGAGCGGTAT CAGCTCACTC AAAGGCGGTA ATACGGTTAT
1501 CCACAGAATC AGGGGATAAC GCAGGAAAGA ACATGTGAGC AAAAGGCCAG
1551 CAAAAGGCCA GGAACCGTAA AAAGGCCGCG TTGCTGGCGT TTTTCCATAG
1601 GCTCCGCCCC CCTGACGAGC ATCACAAAAA TCGACGCTCA AGTCAGAGGT
1651 GGCGAAACCC GACAGGACTA TAAAGATACC AGGCGTTTCC CCCTGGAAGC
1701 TCCCTCGTGC GCTCTCCTGT TCCGACCCTG CCGCTTACCG GATACCTGTC
1751 CGCCTTTCTC CCTTCGGGAA GCGTGGCGCT TTCTCAATGC TCACGCTGTA
1801 GGTATCTCAG TTCGGTGTAG GTCGTTCGCT CCAAGCTGGG CTGTGTGCAC
1851 GAACCCCCCG TTCAGCCCGA CCGCTGCGCC TTATCCGGTA ACTATCGTCT
1901 TGAGTCCAAC CCGGTAAGAC ACGACTTATC GCCACTGGCA GCAGCCACTG
1951 GTAACAGGAT TAGCAGAGCG AGGTATGTAG GCGGTGCTAC AGAGTTCTTG
2001 AAGTGGTGGC CTAACTACGG CTACACTAGA AGGACAGTAT TTGGTATCTG
2051 CGCTCTGCTG AAGCCAGTTA CCTTCGGAAA AAGAGTTGGT AGCTCTTGAT
2101 CCGGCAAACA AACCACCGCT GGTAGCGGTG GTTTTTTTGT TTGCAAGCAG
2151 CAGATTACGC GCAGAAAAAA AGGATCTCAA GAAGATCCTT TGATCTTTTC
2201 TACGGGGTCT GACGCTCAGT GGAACGAAAA CTCACGTTAA GGGATTTTGG
2251 TCATGAGATT ATCAAAAAGG ATCTTCACCT AGATCCTTTT AAATTAAAAA
2301 TGAAGTTTTA AATCAATCTA AAGTATATAT GAGTAAACTT GGTCTGACAG
2351 TTACCAATGC TTAATCAGTG AGGCACCTAT CTCAGCGATC TGTCTATTTC
2401 GTTCATCCAT AGCTGCCTGA CTCCCCGTCG TGTAGATAAC TACGATACGG
2451 GAGGGCTTAC CATCTGGCCC CAGTGCTGCA ATGATACCGC GAGACCCACG
2501 CTCACCGGCT CCAGATTTAT CAGCAATAAA CCAGCCAGCC GGAAGGGCCG

**Fig.12** (Fortsetzung)

2551 AGCGCAGAAG TGGTCCTGCA ACTTTATCCG CCTCCATCCA GTCTATTAAT
2601 TGTTGCCGGG AAGCTAGAGT AAGTAGTTCG CCAGTTAATA GTTTGCGCAA
2651 CGTTGTTGCC ATTGCTACAG GCATCGTGGT GTCACGCTCG TCGTTTGGTA
2701 TGGCTTCATT CAGCTCCGGT TCCCAACGAT CAAGGCGAGT TACATGATCC
2751 CCCATGTTGT GCAAAAAAGC GGTTAGCTCC TTCGGTCCTC CGATCGTTGT
2801 CAGAAGTAAG TTGGCCGCAG TGTTATCACT CATGGTTATG GCAGCACTGC
2851 ATAATTCTCT TACTGTCATG CCATCCGTAA GATGCTTTTC TGTGACTGGT
2901 GAGTACTCAA CCAAGTCATT CTGAGAATAG TGTATGCGGC GACCGAGTTG
2951 CTCTTGCCCG GCGTCAATAC GGGATAATAC CGCGCCACAT AGCAGAACTT
3001 TAAAAGTGCT CATCATTGGA AAACGTTCTT CGGGGCGAAA ACTCTCAAGG
3051 ATCTTACCGC TGTTGAGATC CAGTTCGATG TAACCCACTC GTGCACCCAA
3101 CTGATCTTCA GCATCTTTTA CTTTCACCAG CGTTTCTGGG TGAGCAAAAA
3151 CAGGAAGGCA AAATGCCGCA AAAAAGGGAA TAAGGGCGAC ACGGAAATGT
3201 TGAATACTCA TACTCTTCCT TTTTCAATAT TATTGAAGCA TTTATCAGGG
3251 TTATTGTCTC ATGAGCGGAT ACATATTTGA ATGTATTTAG AAAAATAAAC
3301 AAATAGGGGT TCCGCGCACA TTTCCCCGAA AAGTGCCACC TGACGTCTAA
3351 GAAACCATTA TTATCATGAC ATTAACCTAT AAAAATAGGC GTATCACGAG
3401 GCCCTTTCGT CTTCAC

Fig.13

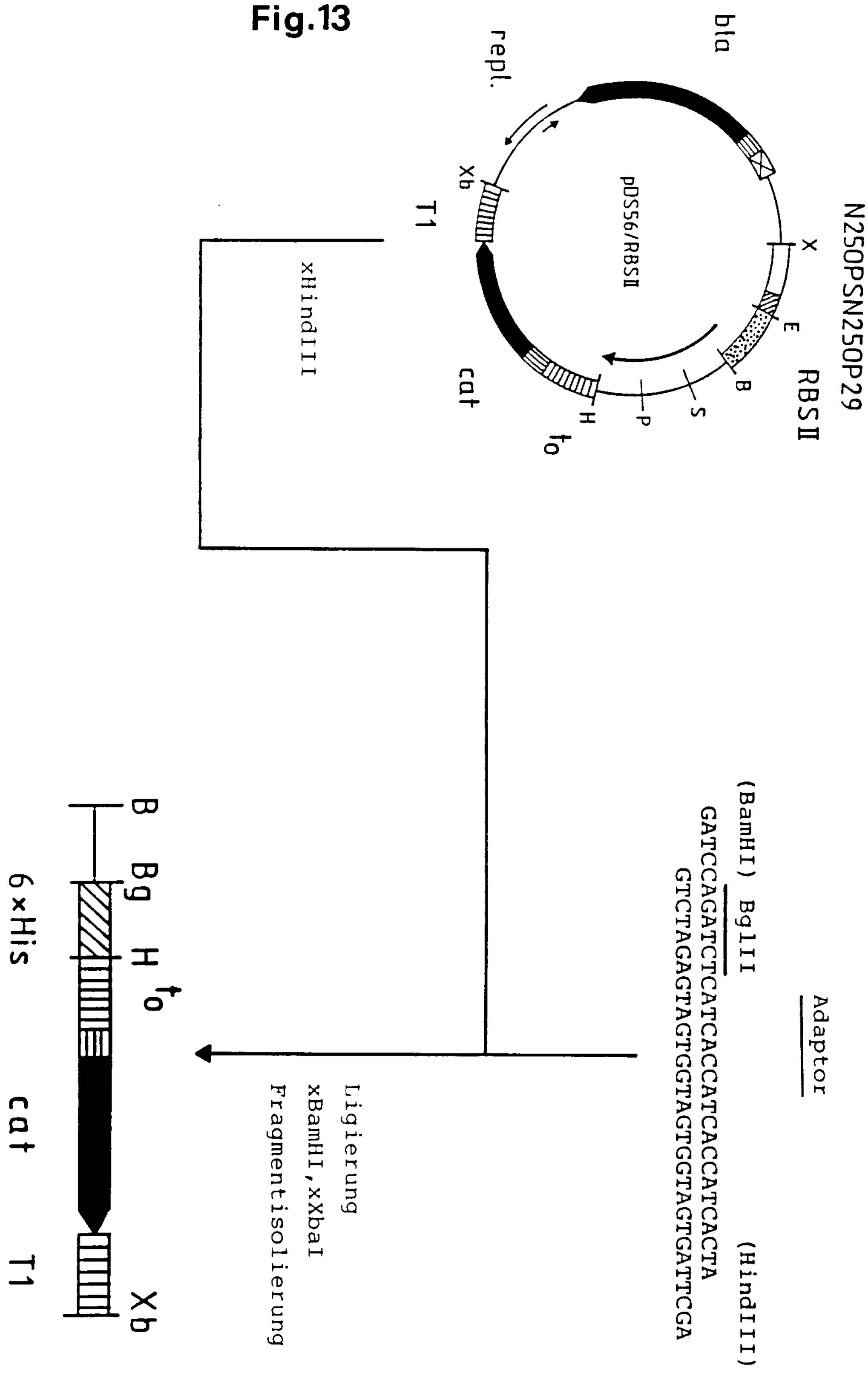
N250PSN250P29
RBSII
pDS56/RBSII
bla
repl.
X
E
B
S
P
H
$t_o$
Xb
T1
cat
xHindIII
Adaptor
(BamHI) BglII (HindIII)
GATCCAGATCTCATCACCATCACCATCACTA
GTCTAGAGTAGTGGTAGTGGTAGTGATTCGA
Ligierung
xBamHI,xXbaI
Fragmentisolierung
B Bg H $t_o$ Xb
6×His
cat
T1

Fig.14

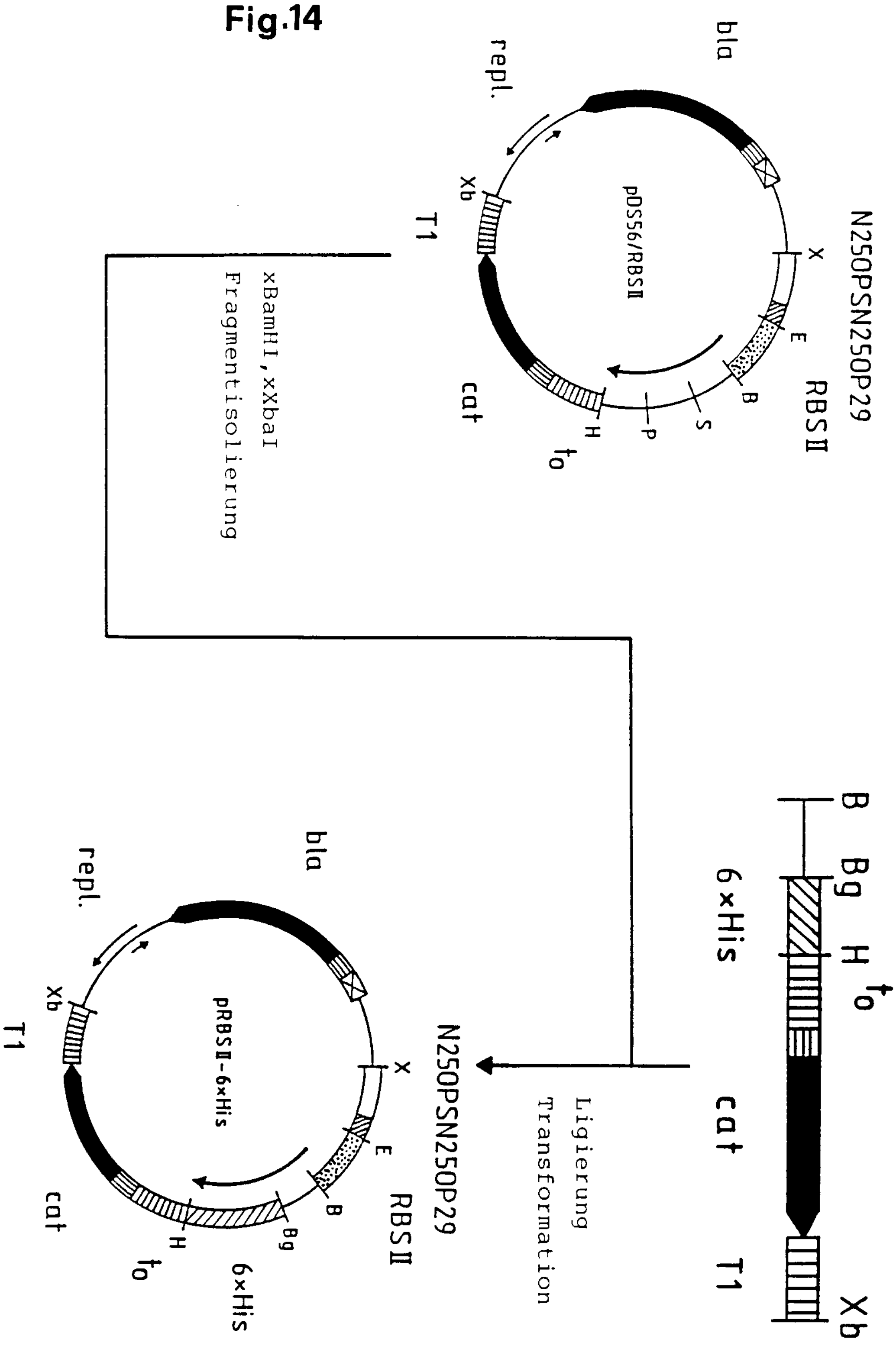
bla
repl.
N25OPSN25OP29
X
E
RBSII
B
S
P
H
$t_o$
cat
T1
Xb
pDS56/RBSII
xBamHI, xXbaI
Fragmentisolierung
B
Bg
H
$t_o$
6×His
cat
T1
Xb
Ligierung
Transformation
bla
repl.
N25OPSN25OP29
X
E
RBSII
B
Bg
6×His
H
$t_o$
cat
T1
Xb
pRBSII-6×His

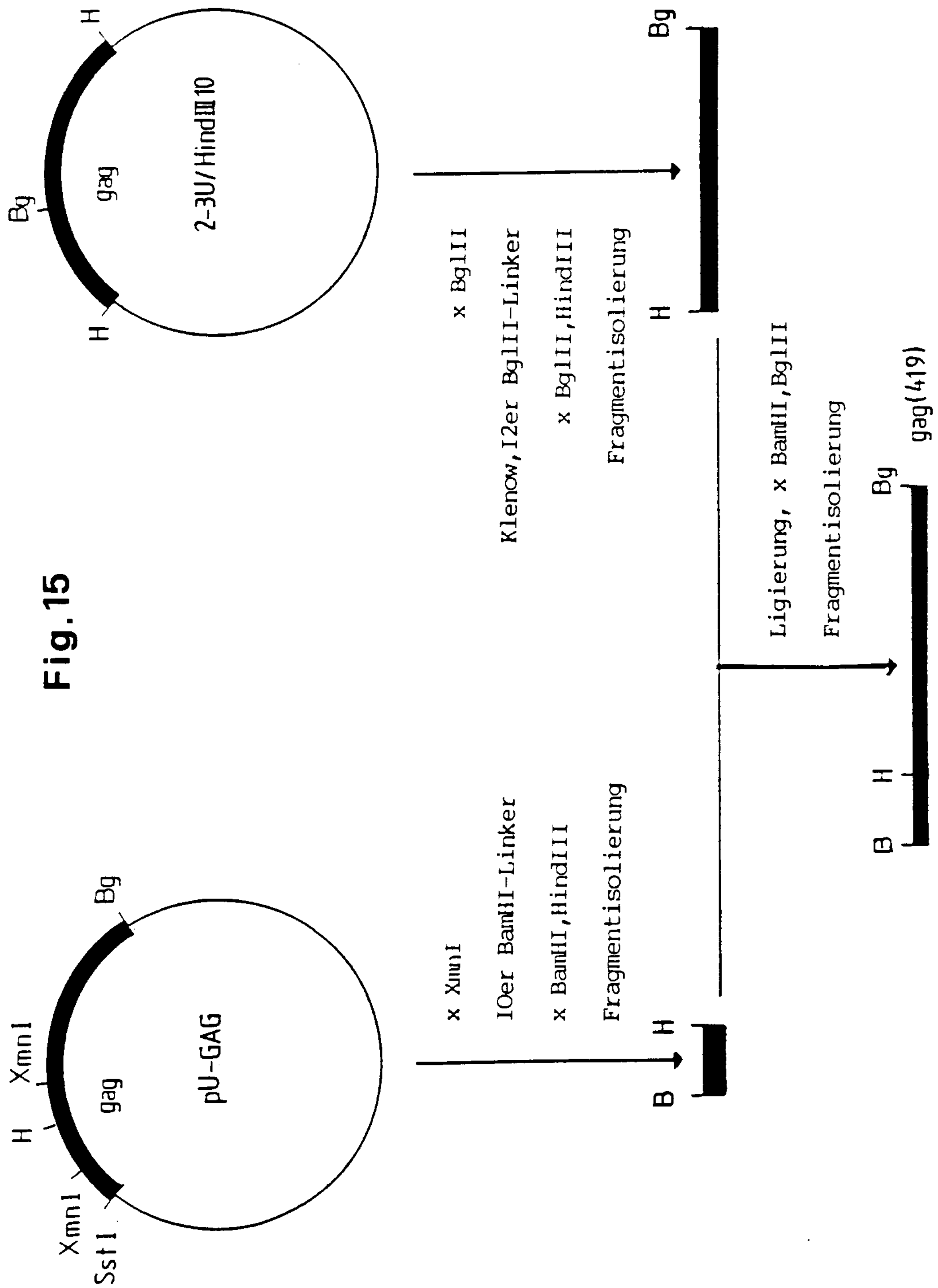
H
Bg
H
gag
2-3U/HindIII10
x BglII
Klenow,12er BglII-Linker
x BglII,HindIII
Fragmentisolierung
H
Bg
Xmn1
H
Xmn1
SstI
Bg
gag
pU-GAG
x XmnI
10er BamHI-Linker
x BamHI,HindIII
Fragmentisolierung
B
H
Ligierung, x BamHI,BglII
Fragmentisolierung
B
H
Bg
gag(419)

Fig.15

# Fig.16

```
           10         20         30         40         50
            |          |          |          |          |
   1 GAGCTCTCTCGACGCAGGACTCGGCTTGCTGAAGCGCGCACGGCAAGAGG
  51 CGAGGGGGGCGACTGGTGAGTACGCCAAAAATTTTGACTAGCGGAGGCT
 101 AGAAGGAGAGAGATGGGTGCGAGAGCGTCAGTATTAAGCGGGGGAAAATT
 151 AGATCGATGGGAAAAAATTCGGTTAAGGCCAGGGGGAAAGAAAAAATATA
 201 AATTAAAACATATAGTATGGGCAAGCAGGGAGCTAGAACGATTCGCAGTT
 251 AATCCTGGCCTGTTAGAAACATCAGAAGGCTGTAGACAAATACTGGGACA
 301 GCTACAACCATCCCTTCAGACAGGATCAAAAGAACTTAGATCATTATATA
 351 ATACAGTAGCAACCCTCTATTGTGTGCATCAAAGGATAGAGATAAAAGAC
 401 ACCAAGGAAGCTTTAGACAAGATAGAGGAAGAGCAAAACAAAAGTAAGAA
 451 AAAAGCACAGCAAGCAGCAGCTGACACAGGACACAGCAGCCAGGTCAGCC
 501 AAAATTACCCTATAGTGCAGAACATCCAGGGGCAAATGGTACATCAGGCC
 551 ATATCACCTAGAACTTTAAATGCATGGGTAAAAGTAGTAGAAGAGAAGGC
 601 TTTCAGCCCAGAAGTGATACCCATGTTTTCAGCATTATCAGAAGGAGCCA
 651 CCCCACAAGATTTAAACACCATGCTAAACACAGTGGGGGGACATCAAGCA
 701 GCCATGCAAATGTTAAAAGAGACCATCAATGAGGAAGCTGCAGAATGGGA
 751 TAGAGTGCATCCAGTGCATGCAGGGCCTATCGCACCAGGCCAGATGAGAG
 801 AACCAAGGGGAAGTGACATAGCAGGAACTACTAGTACCCTTCAGGAACAA
 851 ATAGGATGGATGACAAATAATCCACCTATCCCAGTAGGAGAAATTTATAA
 901 AAGATGGATAATCCTGGGATTAAATAAAATAGTAAGAATGTATAGCCCTA
 951 CCAGCATTCTGGACATAAGACAAGGACCAAAAGAACCCTTTAGAGACTAT
1001 GTAGACCGGTTCTATAAAACTCTAAGAGCCGAGCAAGCTTCACAGGAGGT
1051 AAAAAATTGGATGACAGAAACCTTGTTGGTCCAAAATGCGAACCCAGATT
1101 GTAAGACTATTTTAAAAGCATTGGGACCAGCGGCTACACTAGAAGAAATG
1151 ATGACAGCATGTCAGGGAGTAGGAGGACCCGGCCATAAGGCAAGAGTTTT
1201 GGCTGAAGCAATGAGCCAAGTAACAAATTCAGCTACCATAATGATGCAGA
1251 GAGGCAATTTTAGGAACCAAAGAAAGATTGTTAAGTGTTTCAATTGTGGC
1301 AAAGAAGGGCACACAGCCAGAAATTGCAGGGCCCCTAGGAAAAAGGGCTG
1351 TTGGAAATGTGGAAAGGAAGGACACCAAATGAAAGATTGTACTGAGAGAC
1401 AGGCTAATTTTTTAGGGAAGATCT
```

# Fig.17

| | 10 | 20 | 30 | 40 | 50 |
|---|---|---|---|---|---|
| 1 | AAGCTTTAGA | CAAGGTAGAG | GAAGAGCAAA | ACAACAGTAA | GAAAAAGGCA |
| 51 | CAGCAAGAAG | CAGCTGACGC | AGGAAACAGA | AACCAGGTCA | GCCAAAATTA |
| 101 | CCCTATAGTG | CAAAACCTAC | AGGGACAAAT | GGTACATCAG | GCCATATCAC |
| 151 | CTAGAACTTT | AAATGCATGG | GTAAAAGTAG | TGGAAGAGAA | GGCTTTCAGC |
| 201 | CCAGAAGTAA | TACCCATGTT | TTCAGCATTA | TCAGAAGGAG | CCACCCCACA |
| 251 | AGATTTAAAC | ACCATGCTAA | ACACAGTGGG | GGGACATCAA | GCAGCCATGC |
| 301 | AAATGTTAAA | AGAAACCATC | AATGAGGAAG | CTGCAGAATG | GGATAGATTG |
| 351 | CACCCAGTGC | ATGCAGGGCC | TATTGCACCA | GGCCAGATGA | GAGAACCAAG |
| 401 | GGGAAGTGAC | ATAGCAGGAA | CTACTAGTAC | CCTTCAGGAA | CAAATAGGAT |
| 451 | GGATGACAAA | TAATCCACCT | ATCCCAGTAG | GAGAAATATA | TAAGAGATGG |
| 501 | ATAATCCTGG | GATTAAATAA | AATAGTAAGA | ATGTATAGCC | CTACCAGCAT |
| 551 | TCTGGATATA | AAACAAGGAC | CAAAAGAACC | CTTTAGAGAT | TATGTAGACC |
| 601 | GGTTCTATAA | AACCCTAAGA | GCCGAGCAAG | CTACACAGGA | AGTAAAAAAT |
| 651 | TGGATGACAG | AAACCTTGTT | GGTCCAAAAT | GCGAATCCAG | ATTGTAAGAC |
| 701 | TATTTTAAAA | GCATTAGGAC | CAGCAGCTAC | ACTAGAAGAA | ATGATGACAG |
| 751 | CATGTCAGGG | AGTGGGGGGA | CCCGGCCATA | AAGCAAGAGT | TTTGGCTGAA |
| 801 | GCAATGAGCC | AAGTAACAGG | TTCAGCTGCC | ATAATGATGC | AGAGAGGCAA |
| 851 | TTTTAGGAAC | CAAAGAAAGA | CTGTTAAGTG | TTTCAATTGT | GGCAAAGAAG |
| 901 | GGCACATAGC | CAGAAATTGC | AGGGCCCCTA | GGAAAAAGGG | CTGTTGGAAA |
| 951 | TGTGGAAAGG | AAGGACATCA | AATGAAGGAT | TGCACAGAAA | GACAGGCTAA |
| 1001 | TTTTTTAGGG | AAGATCTGGC | CTTCCCACAA | GGGGAGGCCA | GGAAATTTTC |
| 1051 | TTCAGAGCAG | ACCAGAGCCA | ACAGCCCCAC | CAGAAGAGAG | CTTCAGGTTT |
| 1101 | GGGGAAGCAA | CAGCTCCCTC | TCAGAAGCAG | GAGCCGATAG | ACAAGGAACT |
| 1151 | GTATCCCTTA | GCCTCCCTCA | AATCACTCTT | TGGCAGCGAC | CCCTCGTCAC |

**Fig.17** (Fortsetzung)

1201 AATAAAGATA GGGGGGCAAC TAAAGGAAGC TCTATTAGAT ACAGGAGCAG

1251 ATGATACAGT AGTAGAAGAA ATGAGTTTGC CAGGAAGATG GAAACCAAAA

1301 ATGATAGGAG GAATTGGAGG TTTTATCAAA GTAAGACAGT ATGATCAGAT

1351 ACTCGTAGAA ATCTGTGGAC ATAAAGCTAT AGGTACAGTA TTAGTAGGAC

1401 CTACACCTGT CAACATAATT GGAAGAAATC TGTTGACTCA GATTGGTTGC

1451 ACTTTAAATT TTCCCATTAG TCCTATTGAA ACTGTACCAG TAAAATTAAA

1501 GCCAGGAATG GATGGCCCAA AAGTTAAACA ATGGCCATTG ACAGAAGAAA

1551 AAATAAAAGC ATTAATAGAA ATTTGTACAG AAATGGAAAA GGAAGGGAAA

1601 ATTTCAAAAA TTGGGCCTGA AAATCCATAC AATACTCCAG TATTTGCCAT

1651 AAAGAAAAAG GACAGTACTA AATGGAGAAA ATTAGTAGAT TTCAGAGAAC

1701 TTAATAAAAG AACTCAAGAT TTCTGGGAAG TTCAATTAGG AATACCACAT

1751 CCCGCAGGGT TAAAAAAGAA AAAATCAGTA ACAGTCCTGG ATGTGGGTGA

1801 TGCATATTTT TCAGTTCCCC TAGATAAAGA CTTCAGAAAA TATACTGCAT

1851 TTACCATACC TAGTATAAAC AATGAGACAC CAGGGATTAG ATATCAGTAC

1901 AATGTGCTTC CACAGGGATG GAAAGGATCA CCAGCAATAT TCCAAAGTAG

1951 CATGACAAAA ATCTTAGAGC CTTTTAGAAA ACAAAATCCA GACATAGTTA

2001 TCTATCAATA CATGGATGAT TTGTATGTAG GATCTGACTT AGAAATAGAG

2051 CAGCATAGAA CAAAAATAGA GGAACTGAGA CAGCATCTGT TGAAGTGGGG

2101 ATTTACCACA CCAGACAAAA AACATCAGAA AGAACCTCCA TTCCTTTGGA

2151 TGGGTTATGA ACTCCATCCT GATAAATGGA CAGTACAGCC TATAGTGCTG

2201 CCAGAAAAAG ACAGCTGGAC TGTCAATGAC ATACAGAAGT TAGTGGGAAA

2251 ATTGAATTGG GCAAGTCAGA TTTATGCAGG GATTAAAGTA AAGCAATTAT

2301 GTAAGCTACT TAGGGGACCC AAAGCACTAA CAGAAGTAAT ACCACTAACA

2351 AAAGAAGCAG AGCTAGAACT AGCAGAAAAC AGGGAGATTC TAAAAGAACC

2401 AGTACATGGA GTGTATTGTG ACCCATCAAA AGACTTAGTA GCAGAAATAC

2451 AGAAGCAGGG GGAAGGCCAA TGGACATATC AAATTTATCA AGAACCATTT

2501 AAGAATCTGA AAACAGGAAA GTATGCAAGA ATGAGGGGTG CCCACACTAA

2551 TGATATAAAA CAGTTAACAG AGGCAGTGCA AAAAATAGCC ACAGAAGGCA

**Fig. 17** (Fortsetzung)

2601 TAGTAATATG GGGAAAGACT CCTAAATTTA GACTACCCAT ACAAAAGGAA
2651 ACATGGGAAG CATGGTGGAC GGAGTATTGG CAAGCCACCT GGATTCCTGA
2701 GTGGGAGTTT GTCAATACCC CTCCCTTAGT GAAATTATGG TACCAGTTAG
2751 AGAAAGAACC CATAGTAGGA GCAGAAACTT TCTATGTAGA TGGGGCAGCT
2801 AATAGGGAGA CTAAATTAGG AAAAGCAGGA TATGTTACTG ACAGGGGAAG
2851 ACAAAAAGTT GTCTCCCTAA ATGACACAAC AAATCAGAAG ACTGAGTTAC
2901 AAGCAATTCA TCTAGCTTTG CAGGATTCGG GACTAGAAGT AAACATAGTA
2951 ACAGACTCAC AATATGCATT AGGAATCATT CAAGCACAAC CAGATAAAAG
3001 TGAATCAGAG TTAGTCAGTC AAATAATAGA GCAGTTAATA AAAAAGGAAA
3051 AGGTCTACCT GGCATGGGTA CCAGCACACA AAGGAATTGG AGGAAATGAA
3101 CAAGTAGATA AATTAGTCAG TGCTGGAATC AGAAAAGTAC TATTTTTAGA
3151 TGGAATAGAT AAGGCCCAAG AAGACCATGA GAAATATCAC AGTAATTGGA
3201 GAGCAATGGC TAATGATTTT AACCTGCCAC CTGTAGTAGC AAAAGAAATA
3251 GTAGCCAGCT GTGATAAATG TCAGCTAAAA GGAGAAGCCA TGCATGGACA
3301 AGTAGACTGT AGTCCAGGAA TATGGCAACT AGATTGTACA CATTTAGAAG
3351 GAAAAATTAT CCTGGTAGCA GTTCATGTAG CTAGTGGATA TATAGAAGCA
3401 GAAGTCATTC CAGTAGAGAC AGGGCAGGAA ACAGCATATT TTCTCTTAAA
3451 ATTAGCAGGA AGATGGCCAG TAAAAACAGT ACATACAGAC AATGGCCCTA
3501 ATTTCACCAG TACTACGGTT AAGGCCGCCT GTTGGTGGGC AGGGATCAAG
3551 CAGGAATTTG GCATTCCCTA CAATCCCCAA AGTCAAGGGG TAGTAGAATC
3601 TATGAATAAA GAGTTAAAGA AAATTATAGG ACAGGTAAGA GATCAGGCTG
3651 AACATCTTAA GACAGCAGTA CAAATGGCAG TATTCATCCA CAATTTTAAA
3701 AGAAAAGGGG GGATTGGGGG GTACAGTGCA GGGGAAAGAA TAGTAGACAT
3751 AATAGCAACA GACATACAAA CTAAAGAATT ACAAAAACAA ATTACAAAAA
3801 TTCAAAATTT TCGGGTTTAT TACAGGGACA GCAGAGAACC ATTTTGGAAA
3851 GGACCAGCAA AGCTT

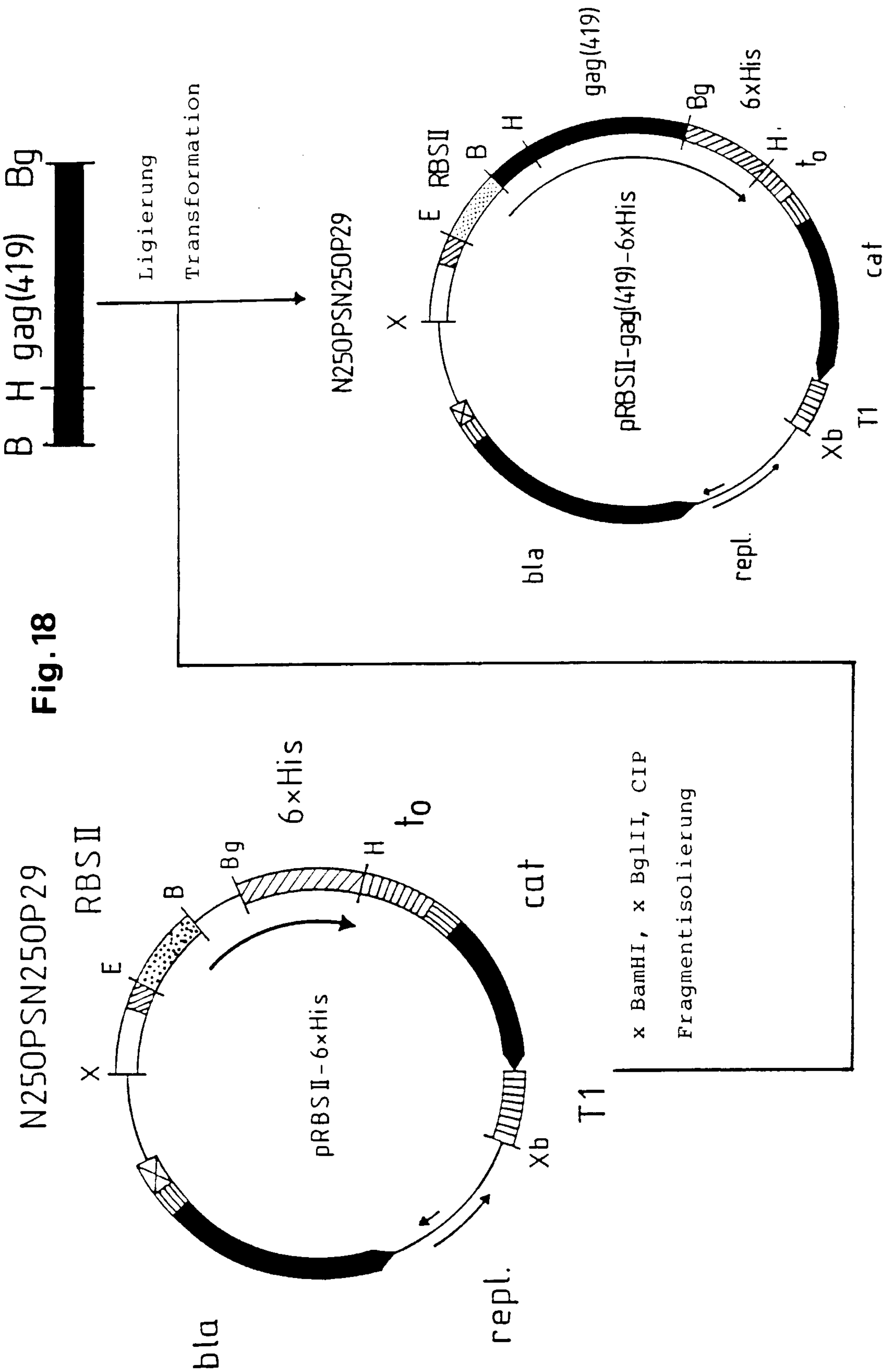
B H gag(419) Bg
Ligierung
Transformation
N250PSN250P29
pRBSII-gag(419)-6xHis
X
E
RBSII
B
H
gag(419)
Bg
6xHis
H
$t_o$
cat
T1
Xb
repl.
bla
N250PSN250P29
RBSII
pRBSII-6xHis
6×His
cat
T1
Xb
repl.
bla
x BamHI, x BglII, CIP
Fragmentisolierung

Fig.18

Fig. 19

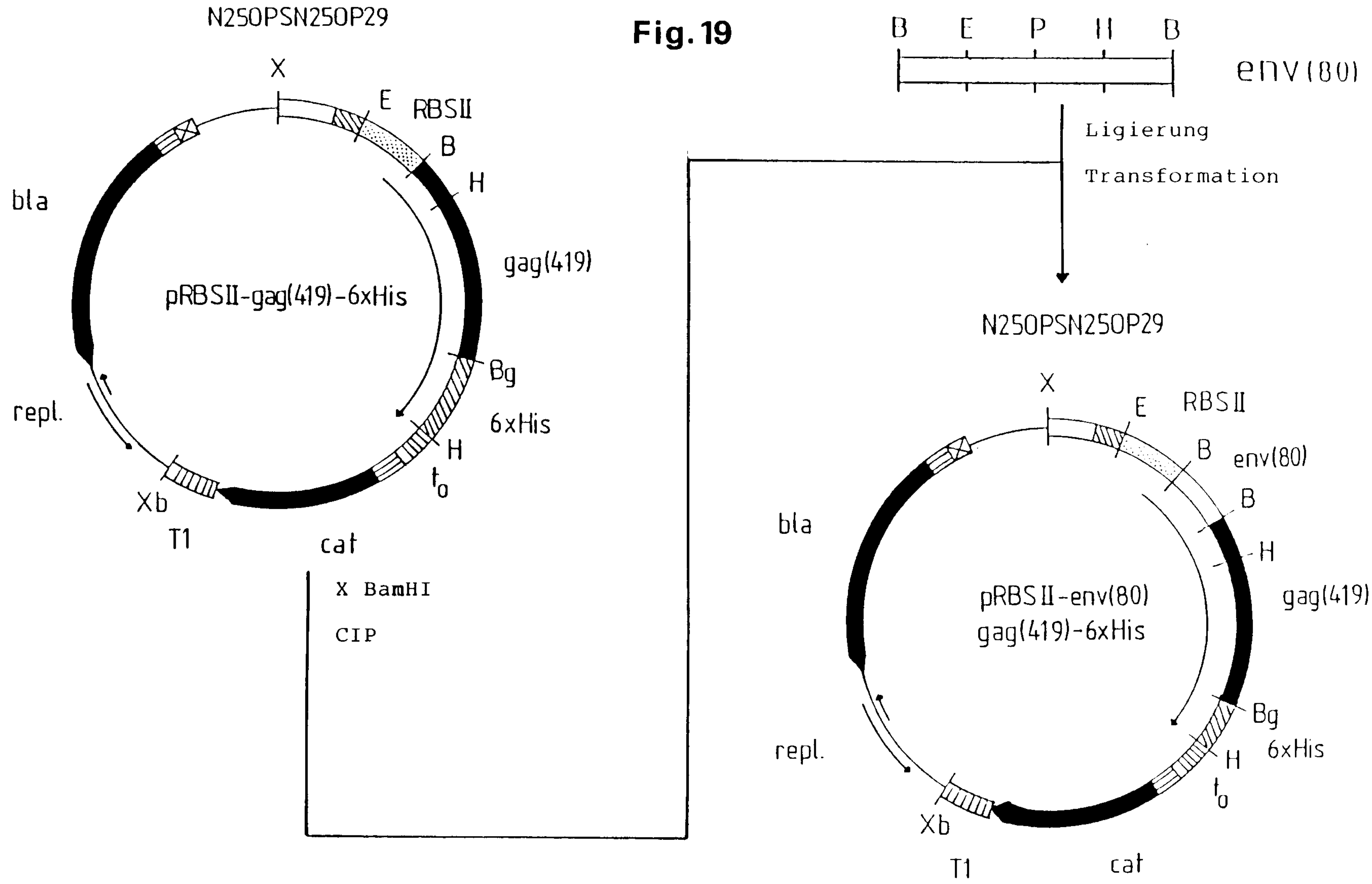
N250PSN250P29
X
E
RBSII
B
H
bla
gag(419)
pRBSII-gag(419)-6xHis
Bg
repl.
6xHis
H
$t_0$
Xb
T1
cat
X BamHI
CIP
B E P H B
env(80)
Ligierung
Transformation
N250PSN250P29
X
E
RBSII
B
env(80)
B
H
bla
gag(419)
pRBSII-env(80)
gag(419)-6xHis
Bg
6xHis
repl.
H
$t_0$
Xb
T1
cat

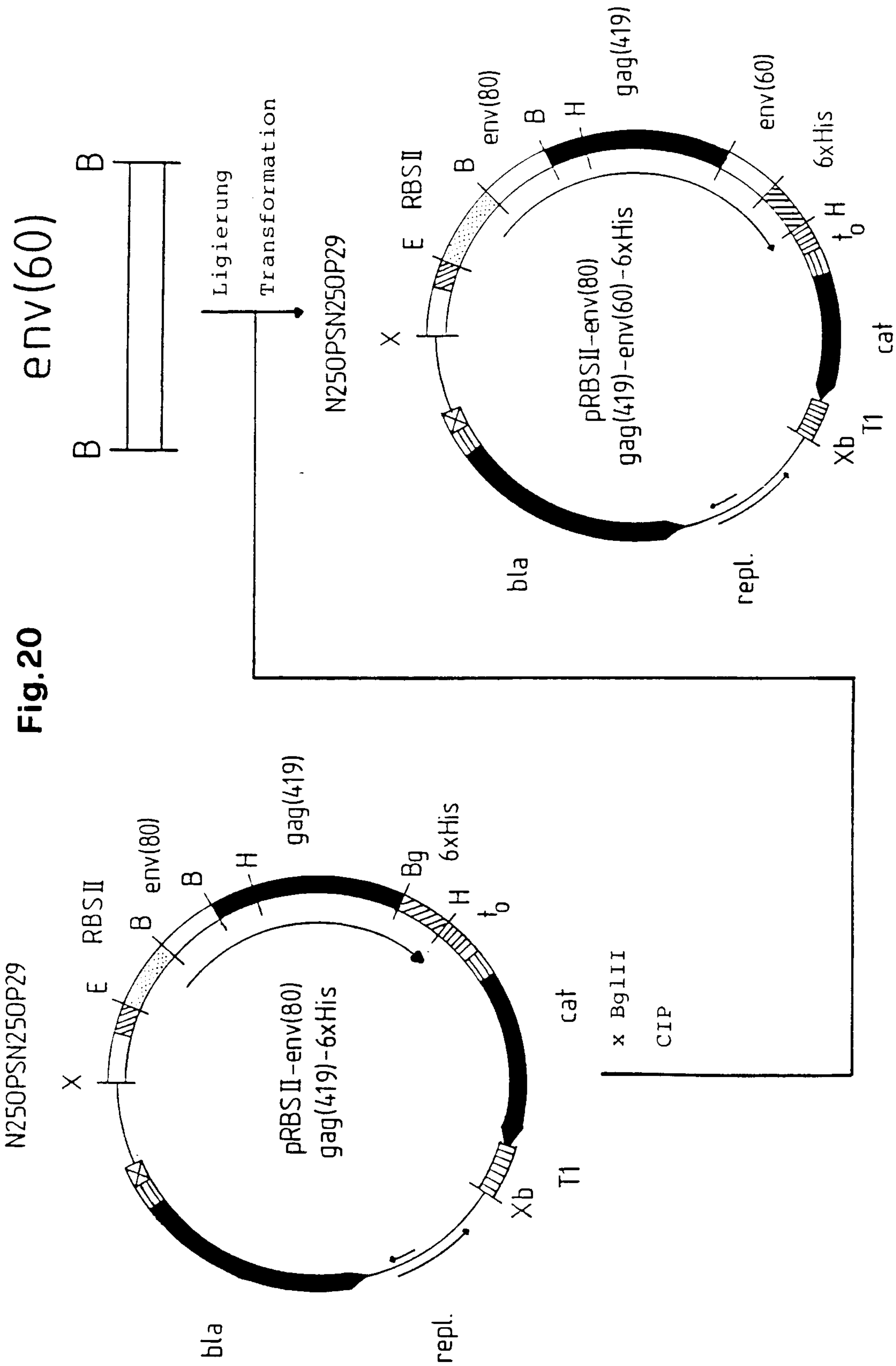
env(60)
B
B
Ligierung
Transformation
N250PSN250P29
X
E
RBSII
B
env(80)
B
H
gag(419)
env(60)
6xHis
H
$t_o$
cat
T1
Xb
repl.
bla
pRBSII-env(80)
gag(419)-env(60)-6xHis
N250PSN250P29
X
E
RBSII
B
env(80)
B
H
gag(419)
Bg
6xHis
H
$t_o$
cat
T1
Xb
repl.
bla
pRBSII-env(80)
gag(419)-6xHis
x BglII
CIP

Fig.20